# EUROPEAN PATENT SPECIFICATION

(11) **EP 4 257 590 B1**
(45) Date of publication and mention of the grant of the patent: **05.11.2025**
(21) Application number: 21900133.6
(22) Date of filing: 06.12.2021
(51) Int. Cl.: C07D 519/00, C07C 57/145, C07C 51/41, A61P 19/02

(54) **SALT OF PYRROLOPYRIMIDINE COMPOUND, CRYSTALLINE FORM THEREOF, AND APPLICATION THEREOF**
SALZ EINER PYRROLOPYRIMIDINVERBINDUNG, KRISTALLINE FORM DAVON UND ANWENDUNG DAVON
SEL DE COMPOSÉ PYRROLOPYRIMIDINE, FORME CRISTALLINE DE CELUI-CI ET UTILISATION ASSOCIÉE

(30) Priority: 04.12.2020 CN 202011413463
(43) Date of publication of application: 11.10.2023
(73) Proprietor: GUANGZHOU JOYO PHARMATECH CO., LTD, Guangzhou, Guangdong, 510663 (CN)
(72) Inventor: LI, Yongguo, Shanghai 201203 (CN); WANG, Chunjuan, Shanghai 201203 (CN); JIA, Guohui, Shanghai 201203 (CN); SHU, Shihui, Shanghai 201203 (CN)
(74) Representative: Secerna LLP
(86) International application number: PCT/CN2021/135784
(87) International publication number: WO 2022/117111

(56) References cited:
- WO-A1-2016/116025
- WO-A1-2016/192563
- WO-A1-2020/244614
- CN-A- 107 805 259
- CN-A- 113 372 343
- CN-A- 113 372 366

## Description

The present application claims the priority of Chinese patent application 202011413463.2 filed on December 04, 2020.

### TECHNICAL FIELD

The present disclosure relates to a salt of a pyrrolopyrimidine compound, a crystalline form thereof, and an application thereof.

### BACKGROUND

Janus kinases (JAKs) are a group of cytoplasmic tyrosine kinases that transmit cytokine signals from membrane receptors to STAT transcription factors. The JAK family includes four members, JAK1, JAK2, JAK3 and TYK2. The JAK-STAT pathway transmits extracellular signals from a variety of cytokines, growth factors and hormones to the nucleus and is responsible for the expression of thousands of protein-coding genes. The JAK-STAT pathway converting extracellular signals into transcriptional responses involves several steps: 1) The conformation of the cytokine receptors on the cell surface changes when they bind to their corresponding cytokine ligands, causing dimerization of the receptor molecules; which leads the receptor-coupled JAK kinases to approach each other and activated through reciprocal tyrosine phosphorylation. 2) The activated JAKs catalyze the phosphorylation of tyrosine residues on the receptors; then the phosphorylated tyrosine sites and surrounding amino acid sequences form a docking site, and STAT proteins containing an SH2 domain are recruited to the docking site. 3) Finally, JAK kinases catalyze the phosphorylation of STAT proteins bound to the receptors; the activated STAT proteins leave the receptors and form dimers, which are then transferred into the nucleus to regulate the transcription of specific genes. JAK-STAT intracellular signal transduction serves interferons, most interleukins, and a variety of cytokines and endocrine factors, such as EPO, TPO, GH, OSM, LIF, CNTF, GM-CSF and PRL (Vainchenker W. et at. (2008)).

JAK-1, JAK-2 and TYK-2 are expressed in various tissue cells in humans, while JAK-3 is mainly expressed in various hematopoietic cells, mainly including bone marrow cells, thymocytes, NK cells, and activated B and T lymphocytes. JAK1 can bind to IL-10, IL-19, IL-20, IL-22, IL-26, IL-28, IFN-α, IFN-γ, IL-6 in the gp130 family, and other receptors containing γc. JAK1 has become a novel target in disease-related fields such as immunity, inflammation and cancer. JAK2 plays an important role in the regulation of a variety of receptor signals including EPO, GH, PRL, IFN-γ, and IL-3, IL-5 and GM-CSF in the βc family. A base mutation JAK2 V617F in the JAK2 gene in humans, is closely related to the development of polycythemia vera (PV), essential thrombocythemia (ET), idiopathic myelofibrosis (IMF), chronic myelogenous leukemia (CML), and the like in myeloproliferative disorders. JAK3 regulates cell signaling by binding to the common γ-chain (γc) in cytokine receptor complexes such as IL-2, IL-4, IL-7, IL-9, IL-15 and IL-21. Either JAK3 or γc mutations can cause severe combined immunodeficiency. Abnormal JAK3 activity manifests as remarkable decreases in T cells and NK cells and loss of B cell function, and seriously affect normal biological functions of the immune system and the like. Based on its functional characteristics and special tissue distribution, JAK3 has become an attractive therapeutic target for immune system-related diseases. TYK2 is the 1st member of the JAK family, which can be activated by a variety of receptors including IFNs, IL-10, IL-6, IL-12, IL-23 and IL-27. In mice, the loss of TYK2 function causes defects in the signaling pathways of various cytokine receptors, leading to viral infection, compromised antibacterial immune functions, and increased risks of pulmonary infection, etc. (John J.O' Shea, 2004, Nature Reviews Drug Discovery 3,555-564). Different JAK family members selectively bind to different cytokine receptors and confer signaling specificity to exert different physiological function. This selective manner allows relatively specific applications of JAK inhibitors in treating diseases. For example, the IL-2 or IL-4 receptor, along with the common γ chain, binds to JAK1 and JAK3, while the type I receptor having the same β chain binds to JAK2. The type I receptor using gp130 (glycoprotein 130) and the type I receptor activated by heterodimeric cytokines preferentially bind to JAK1/2 and TYK2. The type I receptor activated by hormone-like cytokines binds to and activates JAK2 kinase. The type II receptor for interferon binds to JAK1 and TYK2, while the receptor for the IL-10 cytokine family binds to JAK1/2 and TYK2. The specific bindings of the above cytokines and their receptors to JAK family members trigger different physiological effects, providing the possibility for the treatment of different diseases. Heterodimerization of JAK1 with other JAKs transduces cytokine-driven pro-inflammatory signaling. Therefore, inhibition of JAK1 and/or other JAKs is expected to have therapeutic benefit for a range of inflammatory conditions and other diseases driven by JAK-mediated signal transduction (Daniella M. Schwartz, 2017, Nature Reviews Drug Discovery 16,843-862.)

The structural formulas of a compound represented by formula (I-1) and a compound represented by formula (II) are as follows:

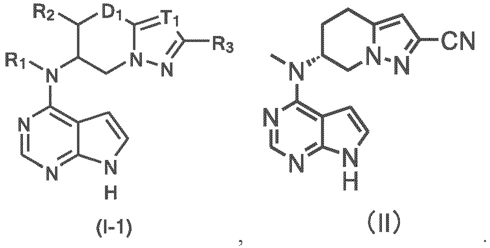

Due to the relatively low solubility of the compound represented by formula (I-1) and the compound represented by formula (II), in order to further improve the solubility of the compounds, we have studied the salt formation of the compound represented by formula (II); at present, there is no report on a salt or crystal form of the compound represented by formula (II). It is well known that the crystal structure of the pharmaceutical active ingredient often affects the chemical and physical stability of the drug, the difference in crystallization conditions and storage conditions may lead to changes in the crystal structure of the compound, sometimes accompanied by the production of other crystalline forms. In general, amorphous drug products do not have a regular crystal structure and often have other defects, such as poor thermodynamic stability. Therefore, it is necessary to improve various properties of the above-mentioned compounds.

WO2016192563A1 discloses a compound represented by the following formula (I) as Janus kinase inhibitors.

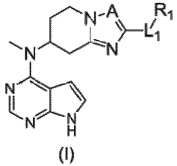

CN107805259A discloses a compound represented by the following formula (I) as Janus kinase inhibitors.

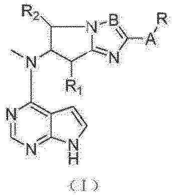

### CONTENT OF THE PRESENT INVENTION

The technical problem to be solved by the present disclosure is the deficiency of the prior arts such as the low solubility of pyrrolopyrimidine compounds, and the present disclosure provides a salt of a pyrrolopyrimidine compound, a crystalline form thereof, and an application thereof. The salt of the pyrrolopyrimidine compound of the present disclosure and the crystalline form thereof have good solubility, stable properties and good hygroscopicity, and have good pharmaceutical prospects.

The present disclosure provides a salt of a compound represented by formula (II) or a hydrate of the salt thereof, 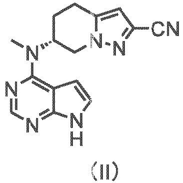 wherein,
acid in the salt is selected from maleic acid, hydrochloric acid and sulfuric acid.

In a certain embodiment, the salt of the compound represented by formula (II) is selected from any one of the following compounds:

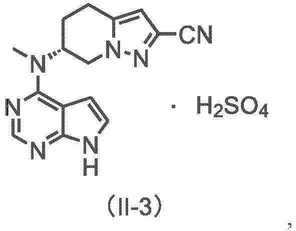

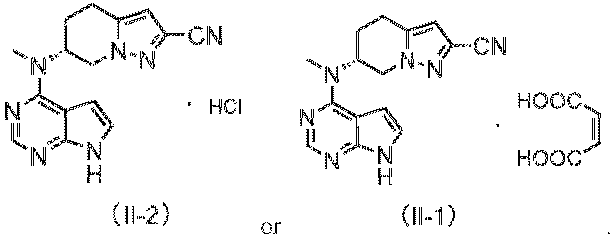

In a certain embodiment, the acid in the salt and the compound represented by formula (II) have a molar ratio of (0.25-1.5):1, preferably (0.5-1):1; more preferably (0.7-1):1; for example, 1:1.

In a certain embodiment, water in the hydrate of the salt and the compound represented by formula (II) have a molar ratio of (0-3):1, for example, 1.5.

In a certain embodiment, the hydrate of the salt of the compound represented by formula (II) is selected from a hydrate of the following compound:

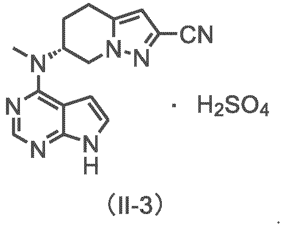

The present disclosure provides a crystal form A of the compound represented by formula (II), which has an X-ray powder diffraction (XRPD) pattern expressed by 2θ angles having diffraction peaks at 12.69°, 13.84°, 15.27°, 15.90°, 16.62°, 19.07°, 27.66° and 25.62°;

In a certain embodiment, in the X-ray powder diffraction pattern of the crystal form A of the compound represented by formula (II) expressed by 2θ angles, 2θ values may also be shown in Table 1:

**Table 1**

| 2θ Angle (°) | Intensity [cts] | Half-peak width [°2θ] | Interplanar spacing d [Å] | Relative intensity (%) |
|---|---|---|---|---|
| 6.33 | 189.22 | 0.1535 | 13.96 | 9.91 |
| 12.69 | 1867.24 | 0.1535 | 6.98 | 97.83 |
| 13.84 | 1317.68 | 0.3070 | 6.40 | 69.04 |
| 14.91 | 533.47 | 0.2047 | 5.94 | 27.95 |
| 15.37 | 1463.93 | 0.1279 | 5.77 | 76.70 |
| 15.90 | 864.22 | 0.1535 | 5.57 | 45.28 |
| 16.62 | 1265.90 | 0.2047 | 5.33 | 66.33 |
| 19.07 | 763.40 | 0.2558 | 4.65 | 40.00 |
| 22.15 | 339.86 | 0.1535 | 4.01 | 17.81 |
| 24.96 | 476.25 | 0.2047 | 3.57 | 24.95 |
| 25.62 | 1908.61 | 0.2558 | 3.48 | 100.00 |
| 26.95 | 584.93 | 0.1535 | 3.31 | 30.65 |
| 27.66 | 569.85 | 0.1791 | 3.23 | 29.86 |
| 30.19 | 110.29 | 0.4093 | 2.96 | 5.78 |
| 37.92 | 59.82 | 0.2047 | 2.37 | 3.13 |
| 38.88 | 144.74 | 0.1791 | 2.32 | 7.58 |

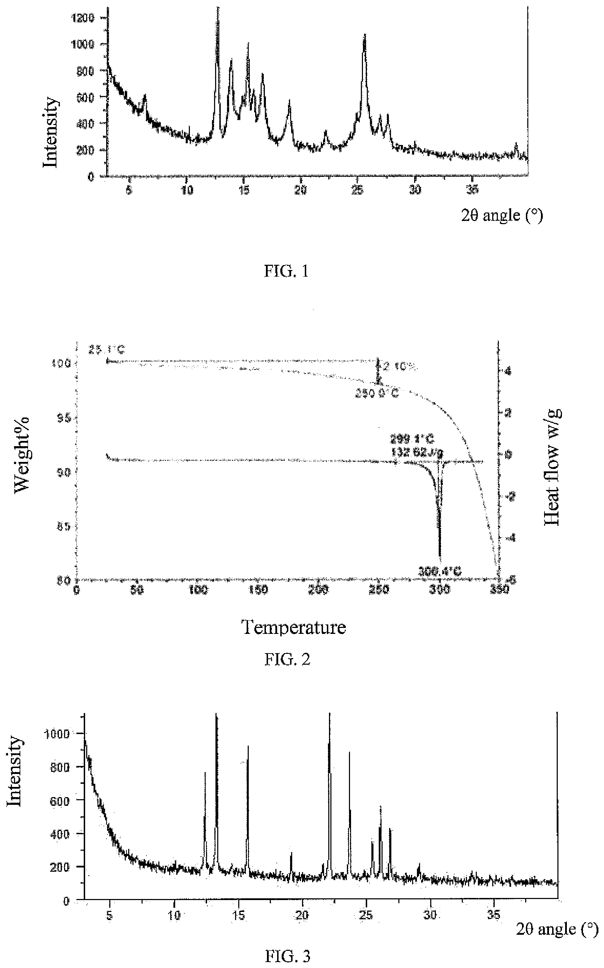

In a certain embodiment, the crystal form A of the compound represented by formula (II) has an X-ray powder diffraction pattern basically as shown in FIG. 1.

In a certain embodiment, the X-ray powder diffraction pattern of the crystal form A of the compound represented by formula (II) is detected under the condition of a Cu-Kα ray source.

In a certain embodiment, the crystal form A of the compound represented by formula (II) has a differential scanning calorimetry curve having an absorption peak with a peak temperature of 300.4°C.

In a certain embodiment, the crystal form A of the compound represented by formula (II) has a thermogravimetric analysis curve (TGA) with a weight loss of 2.1% in a temperature range of 25.1 °C to 250°C.

In a certain embodiment, the crystal form A of the compound represented by formula (II) has a differential scanning calorimetry (DSC) curve and a thermogravimetric analysis curve (TGA) as shown in FIG. 2.

In a certain embodiment, the thermogravimetric analysis curve of the crystal form A of the compound represented by formula (II) is detected under the conditions of a heating interval and a heating rate of 10°C/min.

In a certain embodiment, the differential scanning calorimetry curve of the crystal form A of the compound represented by formula (II) is detected under the conditions of a heating interval and a heating rate of 10°C/min.

The present disclosure provides a crystal form B of the compound represented by formula (II), which has an X-ray powder diffraction pattern expressed by 2θ angles having diffraction peaks at 12.40°, 13.31°, 15.75°, 22. 16°, 23.72°, 25.49°, 26.12° and 26.87°.

In a certain embodiment, in the X-ray powder diffraction pattern of the crystal form B of the compound represented by formula (II) expressed by 2θ angles, 2θ values may also be shown in Table 2:

**Table 2**

| 20 Angle (°) | Intensity [cts] | Half-peak width [°2θ] | Interplanar spacing d [Å] | Relative intensity (%) |
|---|---|---|---|---|
| 12.40 | 425.67 | 0.1535 | 7.14 | 43.82 |
| 13.31 | 771.32 | 0.1535 | 6.65 | 79.40 |
| 15.75 | 743.84 | 0.1023 | 5.63 | 76.57 |
| 19.12 | 146.14 | 0.1023 | 4.64 | 15.04 |
| 22.16 | 971.40 | 0.1023 | 4.01 | 100.00 |
| 23.72 | 610.80 | 0.1535 | 3.75 | 62.88 |
| 25.49 | 202.03 | 0.1023 | 3.49 | 20.80 |
| 26.12 | 410.06 | 0.1023 | 3.41 | 42.21 |
| 26.87 | 255.32 | 0.1023 | 3.32 | 26.28 |
| 33.42 | 23.71 | 0.6140 | 2.68 | 2.44 |

In a certain embodiment, the crystal form B of the compound represented by formula (II) has an X-ray powder diffraction pattern basically as shown in FIG. 3.

In a certain embodiment, the X-ray powder diffraction pattern of the crystal form B of the compound represented by formula (II) is detected under the condition of a Cu-Kα ray source.

The present disclosure provides a crystal form A of the maleate represented by formula (II-1), which has an X-ray powder diffraction pattern expressed by 2θ angles having diffraction peaks at 12.24°, 13.14°, 13.73°, 14.56°, 15.52°, 17.54°, 19.54°, 23.19°, 26.55° and 26.91°.

In a certain embodiment, in the X-ray powder diffraction pattern of the crystal form A of the maleate represented by formula (II-1) expressed by 2θ angles, 2θ values may also be shown in Table 3:

**Table 3**

| 20 Angle (°) | Intensity [cts] | Half-peak width [°2θ] | Interplanar spacing d [Å] | Relative intensity (%) |
|---|---|---|---|---|
| 4.40 | 547.60 | 0.1023 | 20.08 | 14.26 |
| 8.76 | 362.92 | 0.1023 | 10.09 | 9.45 |
| 12.24 | 832.77 | 0.1023 | 7.23 | 21.69 |
| 13.14 | 958.55 | 0.0768 | 6.74 | 24.97 |
| 13.73 | 3839.27 | 0.1023 | 6.45 | 100.00 |
| 14.56 | 742.30 | 0.1279 | 6.09 | 19.33 |
| 15.52 | 616.15 | 0.1279 | 5.71 | 16.05 |
| 16.42 | 97.01 | 0.2047 | 5.40 | 2.53 |
| 17.54 | 1346.73 | 0.1279 | 5.06 | 35.08 |
| 18.89 | 99.32 | 0.1535 | 4.70 | 2.59 |
| 19.54 | 1249.74 | 0.1279 | 4.54 | 32.55 |
| 20.37 | 424.03 | 0.1279 | 4.36 | 11.04 |
| 23.19 | 713.75 | 0.1023 | 3.83 | 18.59 |
| 23.70 | 199.40 | 0.2047 | 3.75 | 5.19 |
| 24.59 | 215.45 | 0.1279 | 3.62 | 5.61 |
| 25.52 | 297.48 | 0.1023 | 3.49 | 7.75 |
| 26.55 | 582.97 | 0.1279 | 3.36 | 15.18 |
| 26.91 | 605.56 | 0.1023 | 3.31 | 15.77 |
| 27.11 | 379.05 | 0.0768 | 3.29 | 9.87 |
| 27.98 | 136.01 | 0.1535 | 3.19 | 3.54 |
| 29.30 | 256.57 | 0.1023 | 3.05 | 6.68 |
| 30.01 | 148.63 | 0.1535 | 2.98 | 3.87 |
| 36.24 | 58.56 | 0.3070 | 2.48 | 1.53 |
| 37.44 | 94.91 | 0.2047 | 2.40 | 2.47 |

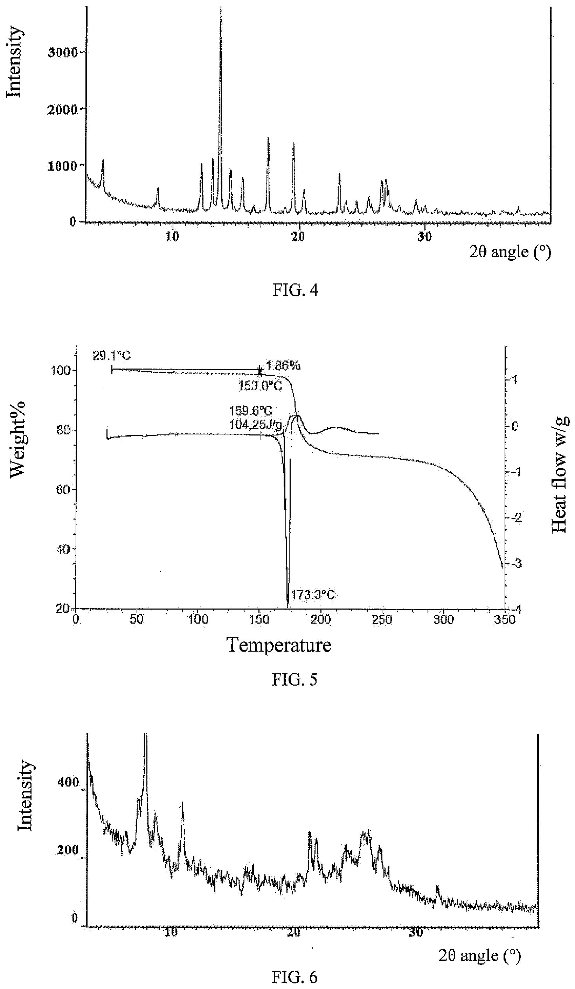

In a certain embodiment, the crystal form A of the maleate represented by formula (II-1) has an X-ray powder diffraction pattern basically as shown in FIG. 4.

In a certain embodiment, the X-ray powder diffraction pattern of the crystal form A of the maleate represented by formula (II-1) is detected under the condition of a Cu-Kα ray source.

In a certain embodiment, the crystal form A of the maleate represented by formula (II-1) has a differential scanning calorimetry (DSC) curve having an absorption peak with a peak temperature of 173.3°C.

In a certain embodiment, the crystal form A of the maleate represented by formula (II-1) has a thermogravimetric analysis curve (TGA) with a weight loss of 1.86% in a temperature range of 29.1°C to 150°C.

In a certain embodiment, the crystal form A of the maleate represented by formula (II-1) has a differential scanning calorimetry (DSC) curve and a thermogravimetric analysis curve (TGA) as shown in FIG. 5.

In a certain embodiment, the thermogravimetric analysis curve of the crystal form A of the maleate represented by formula (II-1) is detected under the conditions of a heating interval and a heating rate of 10°C/min.

In a certain embodiment, the differential scanning calorimetry curve of the crystal form A of the maleate represented by formula (II-1) is detected under the conditions of a heating interval and a heating rate of 10°C/min.

The present disclosure provides a crystal form A of the hydrochloride represented by formula (II-2), which has an X-ray powder diffraction pattern expressed by 2θ angles having diffraction peaks at 7.20°, 7.85°, 8.63°, 10.82°, 21.25°, 21.78°, 24.12°, 25.56°, 26.11° and 27.03°.

In a certain embodiment, in the X-ray powder diffraction pattern of the crystal form A of the hydrochloride represented by formula (II-2) expressed by 2θ angles, 2θ values may also be shown in Table 4:

**Table 4**

| 20 Angle (°) | Intensity [cts] | Half-peak width [°2θ] | Interplanar spacing d [Å] | Relative intensity (%) |
|---|---|---|---|---|
| 6.17 | 32.03 | 0.3070 | 14.32 | 8.01 |
| 7.20 | 152.46 | 0.1535 | 12.28 | 38.14 |
| 7.85 | 399.71 | 0.1535 | 11.26 | 100.00 |
| 8.63 | 120.51 | 0.2558 | 10.25 | 30.15 |
| 10.82 | 204.47 | 0.1279 | 8.18 | 51.15 |
| 12.55 | 27.10 | 0.6140 | 7.06 | 6.78 |
| 13.75 | 27.65 | 0.3070 | 6.44 | 6.92 |
| 15.95 | 38.25 | 0.3070 | 5.56 | 9.57 |
| 19.08 | 21.98 | 0.2047 | 4.65 | 5.50 |
| 20.39 | 29.30 | 0.5117 | 4.36 | 7.33 |
| 21.25 | 140.00 | 0.2047 | 4.18 | 35.03 |
| 21.78 | 134.03 | 0.2047 | 4.08 | 33.53 |
| 24.12 | 106.45 | 0.5117 | 3.69 | 26.63 |
| 25.56 | 153.41 | 0.3070 | 3.49 | 38.38 |
| 26.11 | 150.09 | 0.3070 | 3.41 | 37.55 |
| 27.03 | 112.71 | 0.4093 | 3.30 | 28.20 |
| 31.72 | 44.83 | 0.1535 | 2.82 | 11.22 |

In a certain embodiment, the crystal form A of the hydrochloride represented by formula (II-2) has an X-ray powder diffraction pattern basically as shown in FIG. 6.

In a certain embodiment, the X-ray powder diffraction pattern of the crystal form A of the hydrochloride represented by formula (II-2) is detected under the condition of a Cu-Kα ray source.

In a certain embodiment, the crystal form A of the hydrochloride represented by formula (II-2) has a differential scanning calorimetry curve having three absorption peaks with peak temperatures of 78.1°C, 92.2°C and 274°C, respectively.

In a certain embodiment, the crystal form A of the hydrochloride represented by formula (II-2) has a thermogravimetric analysis curve with a weight loss of 6.71% in a temperature range of 27.2°C to 100°C.

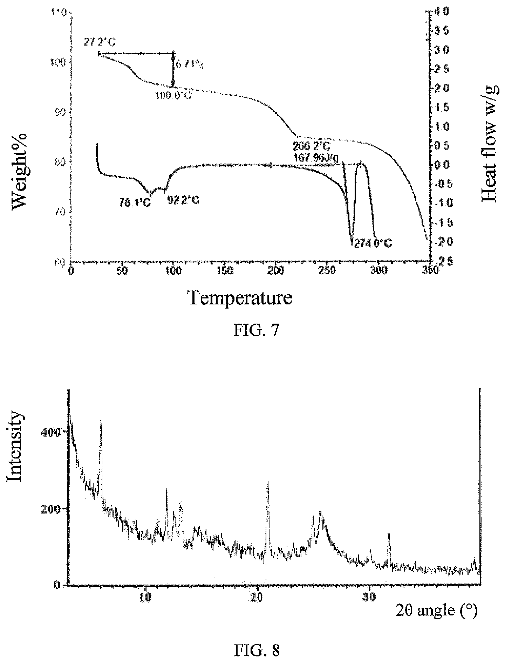

In a certain embodiment, the crystal form A of the hydrochloride represented by formula (II-2) has a differential scanning calorimetry (DSC) curve and a thermogravimetric analysis curve (TGA) as shown in FIG. 7.

The present disclosure provides a crystal form B of the hydrochloride represented by formula (II-2), which has an X-ray powder diffraction pattern expressed by 2θ angles having diffraction peaks at 5.95°, 11.92°, 12.53°, 13.14°, 20.94°, 24.96°, 25.67°, 30.09° and 31.69°.

In a certain embodiment, in the X-ray powder diffraction pattern of the crystal form B of the hydrochloride represented by formula (II-2) expressed by 2θ angles, 2θ values may also be shown in Table 5:

**Table 5**

| 20 Angle (°) | Intensity [cts] | Half-peak width [°2θ] | Interplanar spacing d [Å] | Relative intensity (%) |
|---|---|---|---|---|
| 5.95 | 210.90 | 0.0768 | 14.84 | 100.00 |
| 11.05 | 28.69 | 0.4093 | 8.01 | 13.61 |
| 11.92 | 129.58 | 0.1023 | 7.43 | 61.44 |
| 12.53 | 66.74 | 0.2558 | 7.07 | 31.64 |
| 13.14 | 102.54 | 0.2047 | 6.74 | 48.62 |
| 16.67 | 17.42 | 0.6140 | 5.32 | 8.26 |
| 19.31 | 14.36 | 0.6140 | 4.60 | 6.81 |
| 20.94 | 190.70 | 0.1279 | 4.24 | 90.42 |
| 22.04 | 10.65 | 0.6140 | 4.03 | 5.05 |
| 23.19 | 29.27 | 0.2047 | 3.84 | 13.88 |
| 24.96 | 91.57 | 0.2047 | 3.57 | 43.42 |
| 25.67 | 103.46 | 0.2047 | 3.47 | 49.06 |
| 30.09 | 39.18 | 0.1535 | 2.97 | 18.58 |
| 31.69 | 76.40 | 0.1023 | 2.82 | 36.23 |
| 39.46 | 30.20 | 0.2047 | 2.28 | 14.32 |

In a certain embodiment, the crystal form B of the hydrochloride represented by formula (II-2) has an X-ray powder diffraction pattern basically as shown in FIG. 8.

In a certain embodiment, the X-ray powder diffraction pattern of the crystal form B of the hydrochloride represented by formula (II-2) is detected under the condition of a Cu-Kα ray source.

In a certain embodiment, the crystal form B of the hydrochloride represented by formula (II-2) has a differential scanning calorimetry curve having an absorption peak with a peak temperature of 274.7°C.

In a certain embodiment, the crystal form B of the hydrochloride represented by formula (II-2) has a thermogravimetric analysis curve with a weight loss of 2.34% in a temperature range of 31.4°C to 100°C.

In a certain embodiment, the thermogravimetric analysis curve of the crystal form B of the hydrochloride represented by formula (II-2) is detected under the conditions of a heating interval and a heating rate of 10°C/min.

In a certain embodiment, the differential scanning calorimetry curve of the crystal form B of the hydrochloride represented by formula (II-2) is detected under the conditions of a heating interval and a heating rate of 10°C/min.

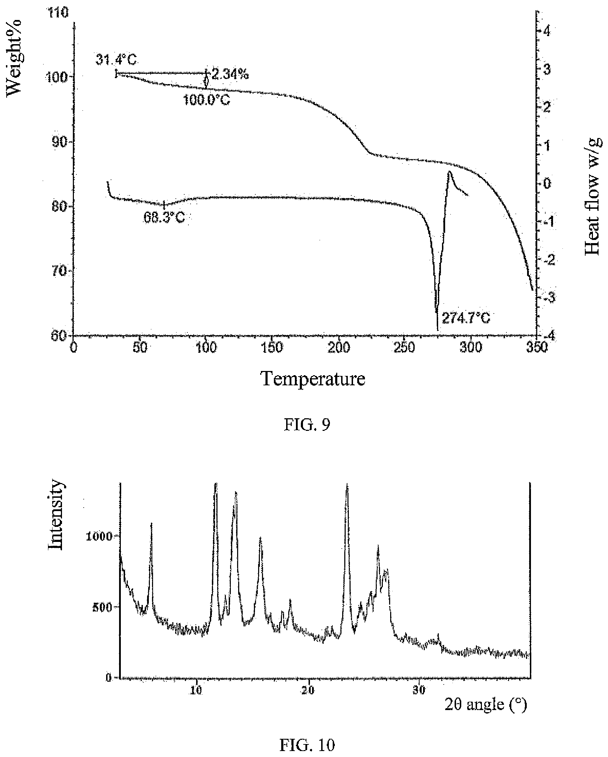

In a certain embodiment, the crystal form B of the hydrochloride represented by formula (II-2) has a differential scanning calorimetry (DSC) curve and a thermogravimetric analysis curve (TGA) as shown in FIG. 9.

The present disclosure provides a crystal form C of the hydrochloride represented by formula (II-2), which has an X-ray powder diffraction pattern expressed by 2θ angles having diffraction peaks at 5.89°, 11.77°, 13.21°, 13.52°, 15.75°, 23.51°, 25.51°, 24.65°, 26.31° and 27.15°.

In a certain embodiment, in the X-ray powder diffraction pattern of the crystal form C of the hydrochloride represented by formula (II-2) expressed by 2θ angles, 2θ values may also be shown in Table 6:

**Table 6**

| 2θ Angle (°) | Intensity [cts] | Half-peak width [°2θ] | Interplanar spacing d [Å] | Relative intensity (%) |
|---|---|---|---|---|
| 5.89 | 661.51 | 0.1023 | 15.00 | 60.87 |
| 11.77 | 1086.75 | 0.1023 | 7.52 | 100.00 |
| 12.58 | 241.70 | 0.2047 | 7.03 | 22.24 |
| 13.21 | 772.40 | 0.2558 | 6.70 | 71.07 |
| 13.52 | 980.86 | 0.1535 | 6.55 | 90.26 |
| 15.75 | 646.70 | 0.2558 | 5.63 | 59.51 |
| 17.71 | 148.92 | 0.2047 | 5.01 | 13.70 |
| 18.41 | 194.85 | 0.3070 | 4.82 | 17.93 |
| 23.51 | 980.88 | 0.3838 | 3.78 | 90.26 |
| 24.65 | 205.46 | 0.4093 | 3.61 | 18.91 |
| 25.51 | 272.94 | 0.3070 | 3.49 | 25.11 |
| 26.31 | 608.00 | 0.2047 | 3.39 | 55.95 |
| 27.15 | 476.10 | 0.2047 | 3.28 | 43.81 |

In a certain embodiment, the crystal form C of the hydrochloride represented by formula (II-2) has an X-ray powder diffraction pattern basically as shown in FIG. 10.

In a certain embodiment, the X-ray powder diffraction pattern of the crystal form C of the hydrochloride represented by formula (II-2) is detected under the condition of a Cu-Kα ray source.

In a certain embodiment, the crystal form C of the hydrochloride represented by formula (II-2) has a differential scanning calorimetry curve having an absorption peak with a peak temperature of 275.1 °C.

In a certain embodiment, the crystal form C of the hydrochloride represented by formula (II-2) has a thermogravimetric analysis curve with a weight loss of 1.32% in a temperature range of 33.7°C to 100°C.

In a certain embodiment, the thermogravimetric analysis curve of the crystal form C of the hydrochloride represented by formula (II-2) is detected under the conditions of a heating interval and a heating rate of 10°C/min.

In a certain embodiment, the differential scanning calorimetry curve of the crystal form C of the hydrochloride represented by formula (II-2) is detected under the conditions of a heating interval and a heating rate of 10°C/min.

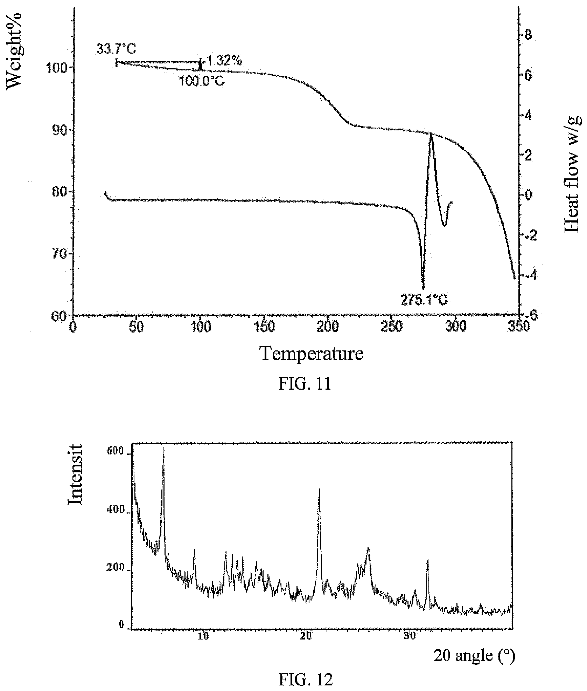

In a certain embodiment, the crystal form C of the hydrochloride represented by formula (II-2) has a differential scanning calorimetry (DSC) curve and a thermogravimetric analysis curve (TGA) as shown in FIG. 11.

The present disclosure provides a crystal form D of the hydrochloride represented by formula (II-2), which has an X-ray powder diffraction pattern expressed by 2θ angles having diffraction peaks at 3.08°, 6.07°, 9.05°, 12.06°, 12.73°, 13.23°, 13.78°, 15.08°, 21.28°, 24.94°, 26.06° and 31.72°.

In a certain embodiment, in the X-ray powder diffraction pattern of the crystal form D of the hydrochloride represented by formula (II-2) expressed by 2θ angles, 2θ values may also be shown in Table 7:

**Table 7**

| 20 Angle (°) | Intensity [cts] | Half-peak width [°2θ] | Interplanar spacing d [Å] | Relative intensity (%) |
|---|---|---|---|---|
| 3.08 | 308.44 | 0.1535 | 28.73 | 79.15 |
| 6.07 | 389.69 | 0.1023 | 14.56 | 100.00 |
| 9.05 | 118.94 | 0.1535 | 9.77 | 30.52 |
| 12.06 | 115.71 | 0.2047 | 7.34 | 29.69 |
| 12.73 | 133.45 | 0.1023 | 6.95 | 34.25 |
| 13.23 | 100.00 | 0.1535 | 6.69 | 25.66 |
| 13.78 | 100.84 | 0.1535 | 6.43 | 25.88 |
| 14.53 | 67.99 | 0.1535 | 6.10 | 17.45 |
| 15.08 | 104.14 | 0.2047 | 5.87 | 26.72 |
| 16.32 | 57.71 | 0.2558 | 5.43 | 14.81 |
| 17.45 | 45.09 | 0.2047 | 5.08 | 11.57 |
| 18.20 | 42.66 | 0.3070 | 4.88 | 10.95 |
| 19.31 | 23.86 | 0.4093 | 4.60 | 6.12 |
| 21.28 | 309.31 | 0.2303 | 4.18 | 79.37 |
| 22.02 | 60.40 | 0.3070 | 4.04 | 15.50 |
| 23.36 | 42.46 | 0.4093 | 3.81 | 10.90 |
| 24.94 | 111.02 | 0.1535 | 3.57 | 28.49 |
| 26.06 | 140.67 | 0.4093 | 3.42 | 36.10 |
| 29.23 | 22.47 | 0.4093 | 3.05 | 5.77 |
| 30.51 | 45.00 | 0.2558 | 2.93 | 11.55 |
| 31.72 | 158.71 | 0.1279 | 2.82 | 40.73 |
| 36.83 | 22.60 | 0.3070 | 2.44 | 5.80 |

In a certain embodiment, the crystal form D of the hydrochloride represented by formula (II-2) has an X-ray powder diffraction pattern basically as shown in FIG. 12.

In a certain embodiment, the X-ray powder diffraction pattern of the crystal form D of the hydrochloride represented by formula (II-2) is detected under the condition of a Cu-Kα ray source.

In a certain embodiment, the crystal form D of the hydrochloride represented by formula (II-2) has a differential scanning calorimetry curve having two absorption peaks with peak temperatures of 273.7°C and 279.3°C, respectively.

In a certain embodiment, the crystal form D of the hydrochloride represented by formula (II-2) has a thermogravimetric analysis curve with a weight loss of 2.92% in a temperature range of 28.3°C to 100°C.

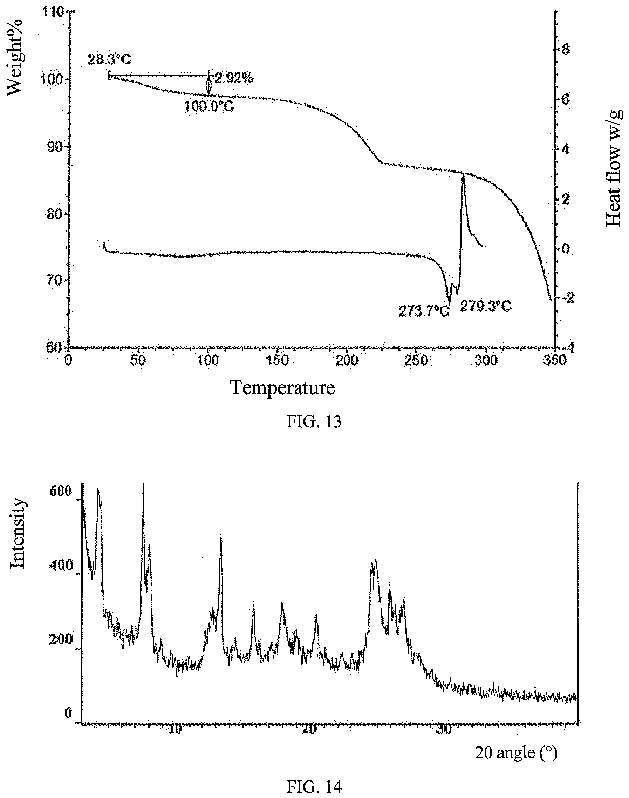

In a certain embodiment, the crystal form D of the hydrochloride represented by formula (II-2) has a differential scanning calorimetry (DSC) curve and a thermogravimetric analysis curve (TGA) as shown in FIG. 13.

In a certain embodiment, the thermogravimetric analysis curve of the crystal form D of the hydrochloride represented by formula (II-2) is detected under the conditions of a heating interval and a heating rate of 10°C/min.

In a certain embodiment, the differential scanning calorimetry curve of the crystal form D of the hydrochloride represented by formula (II-2) is detected under the conditions of a heating interval and a heating rate of 10°C/min.

The present disclosure provides a crystal form A of the sulfate represented by formula (II-3), which has an X-ray powder diffraction pattern expressed by 2θ angles having diffraction peaks at 4.16°, 4.46°, 7.56°, 8.02°, 12.65°, 13.36°, 15.75°, 17.91°, 20.43°, 24.54°, 24.94°, 25.90° and 26.99°.

In a certain embodiment, in the X-ray powder diffraction pattern of the crystal form A of the sulfate represented by formula (II-3) expressed by 2θ angles, 2θ values may also be shown in Table 8:

**Table 8**

| 20 Angle (°) | Intensity [cts] | Half-peak width [°2θ] | Interplanar spacing d [Å] | Relative intensity (%) |
|---|---|---|---|---|
| 4.16 | 360.48 | 0.2047 | 21.23 | 81.60 |
| 4.46 | 318.05 | 0.1023 | 19.81 | 71.99 |
| 7.56 | 441.79 | 0.1023 | 11.69 | 100.00 |
| 8.02 | 279.18 | 0.1535 | 11.03 | 63.19 |
| 9.67 | 16.60 | 0.3070 | 9.15 | 3.76 |
| 12.65 | 135.44 | 0.6140 | 7.00 | 30.66 |
| 13.36 | 341.44 | 0.1279 | 6.63 | 77.29 |
| 14.36 | 62.40 | 0.3070 | 6.17 | 14.12 |
| 15.75 | 176.76 | 0.1279 | 5.63 | 40.01 |
| 17.91 | 171.56 | 0.3070 | 4.95 | 38.83 |
| 19.02 | 94.31 | 0.3070 | 4.66 | 21.35 |
| 20.43 | 152.95 | 0.1535 | 4.35 | 34.62 |
| 22.34 | 48.62 | 0.3070 | 3.98 | 11.00 |
| 24.54 | 271.15 | 0.1535 | 3.63 | 61.38 |
| 24.94 | 291.23 | 0.1535 | 3.57 | 65.92 |
| 25.90 | 219.05 | 0.1279 | 3.44 | 49.58 |
| 26.99 | 190.59 | 0.2047 | 3.30 | 43.14 |
| 27.87 | 52.20 | 0.3582 | 3.20 | 11.82 |

In a certain embodiment, the crystal form A of the sulfate represented by formula (II-3) has an X-ray powder diffraction pattern basically as shown in FIG. 14.

In a certain embodiment, the X-ray powder diffraction pattern of the crystal form A of the sulfate represented by formula (II-3) is detected under the condition of a Cu-Kα ray source.

In a certain embodiment, the crystal form A of the sulfate represented by formula (II-3) has a differential scanning calorimetry curve having two absorption peaks with peak temperatures of 85.1°C and 126.7°C, respectively.

In a certain embodiment, the crystal form A of the sulfate represented by formula (II-3) has a thermogravimetric analysis curve with a weight loss of 8.14% in a temperature range of 28.4°C to 150°C.

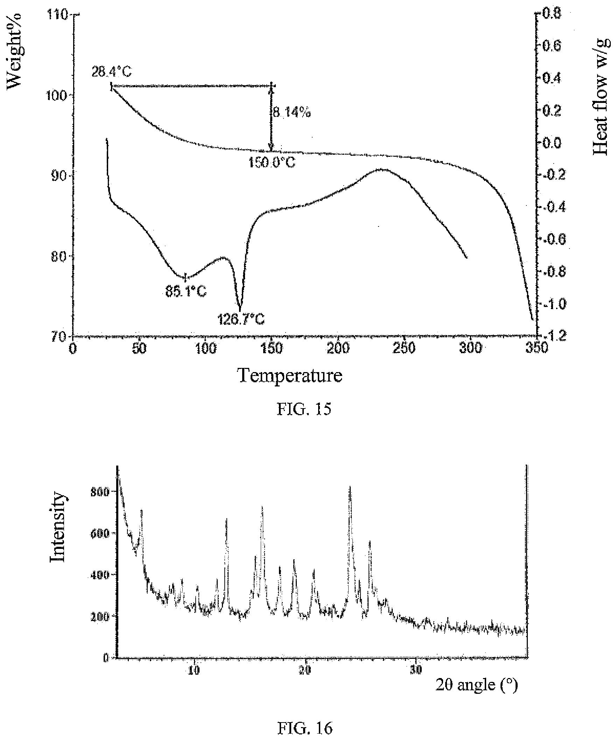

In a certain embodiment, the crystal form A of the sulfate represented by formula (II-3) has a differential scanning calorimetry (DSC) curve and a thermogravimetric analysis curve (TGA) as shown in FIG. 15.

In a certain embodiment, the thermogravimetric analysis curve of the crystal form A of the sulfate represented by formula (II-3) is detected under the conditions of a heating interval and a heating rate of 10°C/min.

In a certain embodiment, the differential scanning calorimetry curve of the crystal form A of the sulfate represented by formula (II-3) is detected under the conditions of a heating interval and a heating rate of 10°C/min.

The present disclosure provides a crystal form C of the sulfate represented by formula (II-3), which has an X-ray powder diffraction pattern expressed by 2θ angles having diffraction peaks at 6.09°, 12.17°, 13.25°, 15.60°, 16.09°, 17.45°, 18.66°, 22.61°, 23.62°, 24.44°, 25.04°, 25.89°, 26.30° and 26.62°.

In a certain embodiment, in the X-ray powder diffraction pattern of the crystal form C of the sulfate represented by formula (II-3) expressed by 2θ angles, 2θ values may also be shown in Table 9:

**Table 9**

| 20 Angle (°) | Intensity [cts] | Half-peak width [°2θ] | Interplanar spacing d [Å] | Relative intensity (%) |
|---|---|---|---|---|
| 6.09 | 492.48 | 0.1023 | 14.51 | 99.99 |
| 9.87 | 41.45 | 0.3070 | 8.97 | 8.42 |
| 10.84 | 40.94 | 0.1535 | 8.16 | 8.31 |
| 12.17 | 450.11 | 0.1535 | 7.28 | 91.39 |
| 13.25 | 140.14 | 0.1023 | 6.68 | 28.45 |
| 15.60 | 138.71 | 0.1535 | 5.68 | 28.16 |
| 16.09 | 214.62 | 0.1791 | 5.51 | 43.58 |
| 17.45 | 154.85 | 0.1791 | 5.08 | 31.44 |
| 18.66 | 492.52 | 0.1279 | 4.75 | 100.00 |
| 19.80 | 126.27 | 0.2558 | 4.48 | 25.64 |
| 20.88 | 112.34 | 0.2558 | 4.26 | 22.81 |
| 22.61 | 158.69 | 0.1535 | 3.93 | 32.22 |
| 23.62 | 174.01 | 0.0768 | 3.77 | 35.33 |
| 24.44 | 150.95 | 0.2047 | 3.64 | 30.65 |
| 25.04 | 156.95 | 0.1535 | 3.56 | 31.87 |
| 25.89 | 312.21 | 0.1023 | 3.44 | 63.39 |
| 26.30 | 214.22 | 0.1535 | 3.39 | 43.49 |
| 26.62 | 305.62 | 0.1279 | 3.35 | 62.05 |
| 27.43 | 106.47 | 0.1535 | 3.25 | 21.62 |
| 27.76 | 74.74 | 0.6140 | 3.21 | 15.17 |

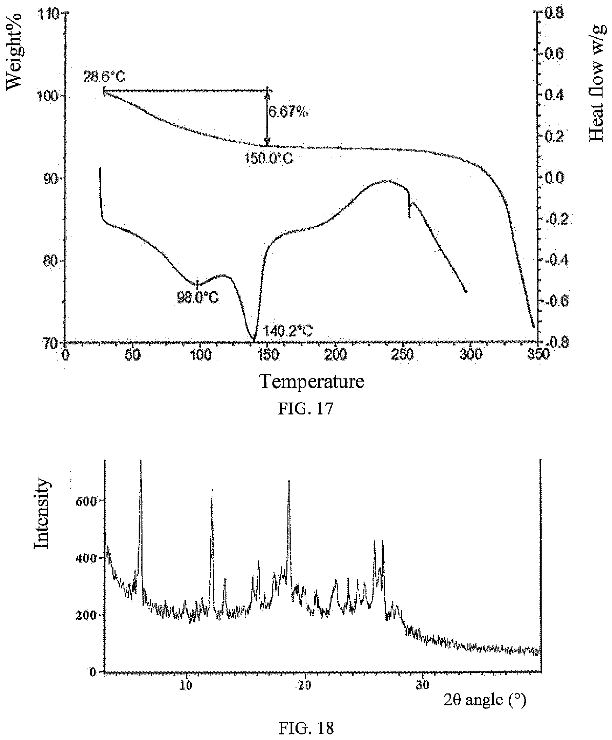

In a certain embodiment, the crystal form C of the sulfate represented by formula (II-3) has an X-ray powder diffraction pattern basically as shown in FIG. 18.

In a certain embodiment, the X-ray powder diffraction pattern of the crystal form C of the sulfate represented by formula (II-3) is detected under the condition of a Cu-Kα ray source.

In a certain embodiment, the crystal form C of the sulfate represented by formula (II-3) has a differential scanning calorimetry curve having two absorption peaks with peak temperatures of 69.3°C and 118.1°C, respectively.

In a certain embodiment, the crystal form C of the sulfate represented by formula (II-3) has a thermogravimetric analysis curve with a weight loss of 6.82% in a temperature range of 28.2°C to 150°C.

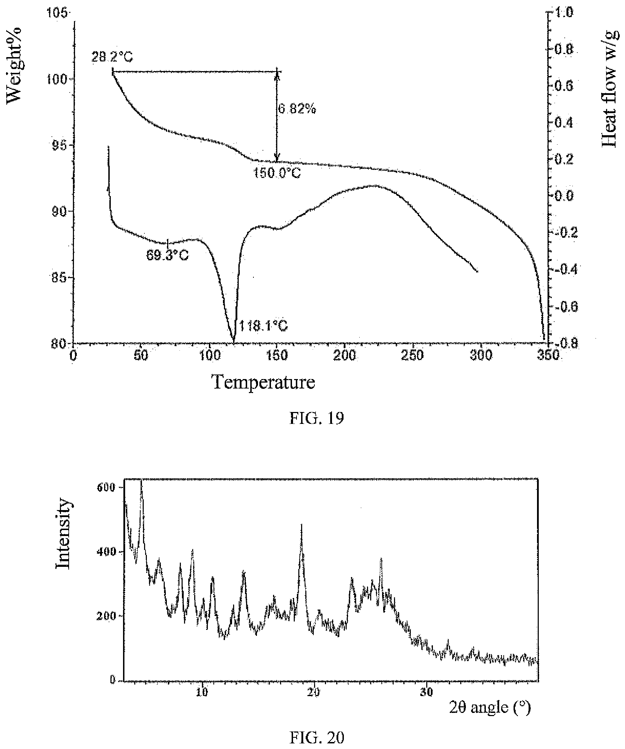

In a certain embodiment, the crystal form C of the sulfate represented by formula (II-3) has a differential scanning calorimetry (DSC) curve and a thermogravimetric analysis curve (TGA) as shown in FIG. 19.

In a certain embodiment, the thermogravimetric analysis curve of the crystal form C of the sulfate represented by formula (II-3) is detected under the conditions of a heating interval and a heating rate of 10°C/min.

In a certain embodiment, the differential scanning calorimetry curve of the crystal form C of the sulfate represented by formula (II-3) is detected under the conditions of a heating interval and a heating rate of 10°C/min.

The present disclosure provides a crystal form D of the sulfate represented by formula (II-3), which has an X-ray powder diffraction pattern expressed by 2θ angles having diffraction peaks at 4.61°, 6.11°, 8.00°, 9.10°, 10.91°, 13.73°, 18.86°, 23.27°, 25.28° and 25.97°.

In a certain embodiment, in the X-ray powder diffraction pattern of the crystal form D of the sulfate represented by formula (II-3) expressed by 2θ angles, 2θ values may also be shown in Table 10:

**Table 10**

| 20 Angle (°) | Intensity [cts] | Half-peak width [°2θ] | Interplanar spacing d [Å] | Relative intensity (%) |
|---|---|---|---|---|
| 4.61 | 223.48 | 0.2047 | 19.19 | 68.84 |
| 6.11 | 95.69 | 0.7164 | 14.46 | 29.48 |
| 8.00 | 147.66 | 0.2558 | 11.05 | 45.48 |
| 9.10 | 209.67 | 0.3582 | 9.71 | 64.58 |
| 10.05 | 61.29 | 0.3070 | 8.81 | 18.88 |
| 10.91 | 152.27 | 0.3582 | 8.11 | 46.90 |
| 12.72 | 80.28 | 0.2047 | 6.96 | 24.73 |
| 13.73 | 175.51 | 0.3582 | 6.45 | 54.06 |
| 16.18 | 77.79 | 0.8187 | 5.48 | 23.96 |
| 18.86 | 324.65 | 0.1279 | 4.71 | 100.00 |
| 20.53 | 48.31 | 0.3582 | 4.33 | 14.88 |
| 23.27 | 127.69 | 0.3582 | 3.82 | 39.33 |
| 25.28 | 99.69 | 0.3070 | 3.52 | 30.71 |
| 25.97 | 180.12 | 0.2047 | 3.43 | 55.48 |
| 31.91 | 44.09 | 0.2047 | 2.80 | 13.58 |
| 34.12 | 18.81 | 0.3070 | 2.63 | 5.79 |
| 38.61 | 14.16 | 0.5117 | 2.33 | 4.36 |

In a certain embodiment, the crystal form D of the sulfate represented by formula (II-3) has an X-ray powder diffraction pattern basically as shown in FIG. 20.

In some embodiments of the present disclosure, the X-ray powder diffraction pattern of the crystal form D of the sulfate represented by formula (II-3) is detected under the condition of a Cu-Kα ray source.

In a certain embodiment, the crystal form D of the sulfate represented by formula (II-3) has a differential scanning calorimetry curve having two absorption peaks with peak temperatures of 78.5°C and 144.3°C, respectively.

In a certain embodiment, the crystal form D of the sulfate represented by formula (II-3) has a thermogravimetric analysis curve with a weight loss of 8.13% in a temperature range of 23.3°C to 150°C.

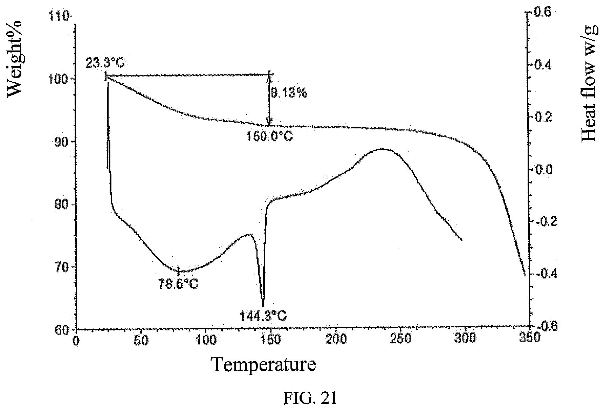

In a certain embodiment, the crystal form D of the sulfate represented by formula (II-3) has a differential scanning calorimetry (DSC) curve and a thermogravimetric analysis curve (TGA) as shown in FIG. 21.

In a certain embodiment, the thermogravimetric analysis curve of the crystal form D of the sulfate represented by formula (II-3) is detected under the conditions of a heating interval and a heating rate of 10°C/min.

In a certain embodiment, the differential scanning calorimetry curve of the crystal form D of the sulfate represented by formula (II-3) is detected under the conditions of a heating interval and a heating rate of 10°C/min.

The present disclosure provides a crystal form B of a hydrate of the sulfate represented by formula (II-3), which has an X-ray powder diffraction pattern expressed by 2θ angles having diffraction peaks at 5.18°, 12.03°, 12.92°, 15.54°, 16.09°, 17.74°, 19.06°, 20.71°, 23.99°, 24.82° and 25.79°.

In a certain embodiment, in the X-ray powder diffraction pattern of the crystal form B of the hydrate of the sulfate represented by formula (II-3) expressed by 2θ angles, 2θ values may also be shown in Table 11:

**Table 11**

| 20 Angle (°) | Intensity [cts] | Half-peak width [°2θ] | Interplanar | Relative |
|---|---|---|---|---|
| 5.18 | 265.23 | 0.1535 | 17.05 | 45.44 |
| 7.95 | 64.18 | 0.3070 | 11.12 | 11.00 |
| 8.82 | 114.15 | 0.1535 | 10.02 | 19.56 |
| 10.23 | 99.11 | 0.2047 | 8.65 | 16.98 |
| 12.03 | 144.85 | 0.1535 | 7.35 | 24.82 |
| 12.92 | 430.46 | 0.1791 | 6.85 | 73.75 |
| 15.54 | 279.62 | 0.2047 | 5.70 | 47.91 |
| 16.09 | 528.12 | 0.1535 | 5.51 | 90.49 |
| 17.74 | 239.95 | 0.1535 | 5.00 | 41.11 |
| 19.06 | 227.53 | 0.3582 | 4.66 | 38.98 |
| 20.71 | 194.57 | 0.2558 | 4.29 | 33.34 |
| 23.99 | 583.64 | 0.2303 | 3.71 | 100.00 |
| 24.82 | 129.68 | 0.2047 | 3.59 | 22.22 |
| 25.79 | 332.19 | 0.1535 | 3.46 | 56.92 |

In a certain embodiment, the crystal form B of the hydrate of the sulfate represented by formula (II-3) has an X-ray powder diffraction pattern basically as shown in FIG. 16.

In a certain embodiment, the X-ray powder diffraction pattern of the crystal form B of the hydrate of the sulfate represented by formula (II-3) is detected under the condition of a Cu-Kα ray source.

In a certain embodiment, the crystal form B of the hydrate of the sulfate represented by formula (II-3) has a differential scanning calorimetry curve having two absorption peaks with peak temperatures of 98°C and 140.2°C, respectively.

In a certain embodiment, the crystal form B of the hydrate of the sulfate represented by formula (II-3) has a thermogravimetric analysis curve with a weight loss of 6.67% in a temperature range of 28.6°C to 150°C.

In a certain embodiment, the crystal form B of the hydrate of the sulfate represented by formula (II-3) has a differential scanning calorimetry (DSC) curve and a thermogravimetric analysis curve (TGA) as shown in FIG. 17.

In a certain embodiment, the thermogravimetric analysis curve of the crystal form B of the hydrate of the sulfate represented by formula (II-3) is detected under the conditions of a heating interval and a heating rate of 10°C/min.

In a certain embodiment, the differential scanning calorimetry curve of the crystal form B of the hydrate of the sulfate represented by formula (II-3) is detected under the conditions of a heating interval and a heating rate of 10°C/min.

In a certain embodiment, water in the crystal form B of the hydrate of the sulfate represented by formula (II-3) and the sulfate represented by formula (II-3) may have a number ratio of 1.5.

The present disclosure also provides a method for preparing the salt of the compound represented by formula (II), which comprises the following step: performing a salt-forming reaction of the compound represented by formula (II) as described above and the acid described above in a solvent.

In a certain embodiment, preferably, the solvent is selected from one or more of tetrahydrofuran, 2-methylfuran, ethyl acetate, cyclohexane, dimethyl sulfoxide, water, n-butanol, isopropanol, ethanol, N-methylpyrrolidone, acetonitrile, acetone, butanone, methyl isobutyl ketone, toluene, dichloromethane, 1,4-dioxane and anisole; preferably, the solvent is selected from one or more of tetrahydrofuran, ethyl acetate, cyclohexane, dimethyl sulfoxide, ethanol, water, n-butanol, N-methylpyrrolidone, acetonitrile and butanone; more preferably, the solvent is selected from tetrahydrofuran, acetone, ethanol, ethyl acetate and acetonitrile aqueous solution.

The present disclosure also provides a method for preparing the crystal form A of the compound represented by formula (II), which comprises the following steps: adding an antisolvent into a solution of the compound represented by formula (II) in tetrahydrofuran at room temperature, and obtaining the crystal form A after crystallization; the antisolvent is an ester solvent or an alkane solvent.

In a certain embodiment, the ester solvent is ethyl acetate or n-butyl acetate, such as ethyl acetate.

In a certain embodiment, the alkane solvent is n-hexane, cyclohexane or n-heptane, such as cyclohexane.

In a certain embodiment, the tetrahydrofuran and the antisolvent have a volume ratio of 1:(0. 1-10); preferably 1:(0.5-3), more preferably 1:1 or 1.25:1.

In a certain embodiment, the compound represented by formula (II) and the tetrahydrofuran have a mass-volume ratio of 2-6 mg/mL; preferably 5:1 mg/mL.

In a certain embodiment, the compound represented by formula (II) and the antisolvent have a mass-volume ratio of 2-6 mg/mL; preferably 5:1 mg/mL or 4:1 mg/mL.

The present disclosure also provides a method for preparing the crystal form B of the compound represented by formula (II), which comprises the following steps: dissolving a crystal form A of a compound represented by formula (II-1) in NMP at room temperature, adding water, and obtaining the crystal form B after crystallization.

In a certain embodiment, the NMP and the water have a volume ratio of 1:(0.1-10); preferably 1:(0.2-2), more preferably 1:0.4.

In a certain embodiment, the crystal form A of the compound represented by formula (II-1) and the NMP have a mass-volume ratio of 1-5 mg/mL; preferably 2:1 mg/mL.

The present disclosure also provides a method for preparing the crystal form A of the compound represented by formula (II-1), which comprises the following steps: dispersing the compound represented by formula (II) and maleic acid in an ester solvent to carry out a reaction shown in the following formula, and collecting a solid to obtain the crystal form A;

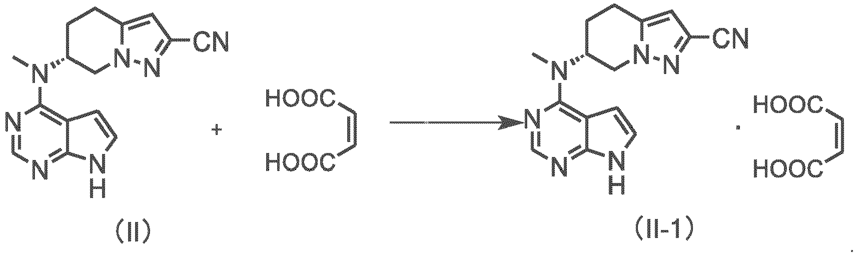

In a certain embodiment, the maleic acid and the compound represented by formula (II) have a molar ratio of 1.04:1.

In a certain embodiment, the compound represented by formula (II) and the ester solvent have a mass-volume ratio of 10 mg/mL.

In a certain embodiment, the ester solvent is a conventional ester solvent in the art, such as ethyl acetate.

The present disclosure also provides a method for preparing the crystal form A of the compound represented by formula (II-2), which comprises the following steps: adding hydrochloric acid to a solution of the compound represented by formula (II) to carry out a reaction shown in the following formula, and collecting a solid to obtain the crystal form A; a solvent of the solution is an acetonitrile aqueous solution;

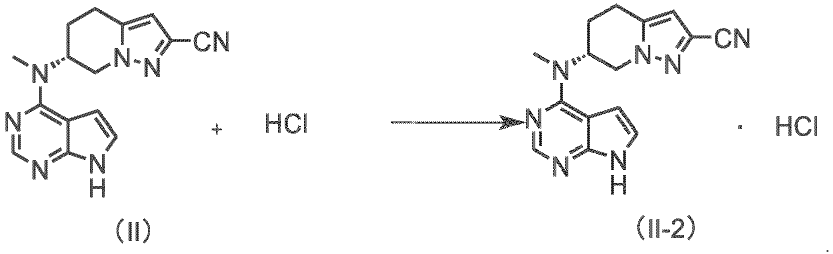

In a certain embodiment, the hydrochloric acid and the compound represented by formula (II) have a molar ratio of 1.05:1.

In a certain embodiment, the compound represented by formula (II) and the acetonitrile aqueous solution have a mass-volume ratio of 40 mg/mL.

In a certain embodiment, the acetonitrile aqueous solution has a volume ratio of acetonitrile to H₂O of 19:1.

The present disclosure also provides a method for preparing the crystal form B of the compound represented by formula (II-2), which comprises the following steps: adding hydrochloric acid to a solution of the compound represented by formula (II) in ethyl acetate to carry out a reaction shown in the following formula, and collecting a solid to obtain the crystal form B;

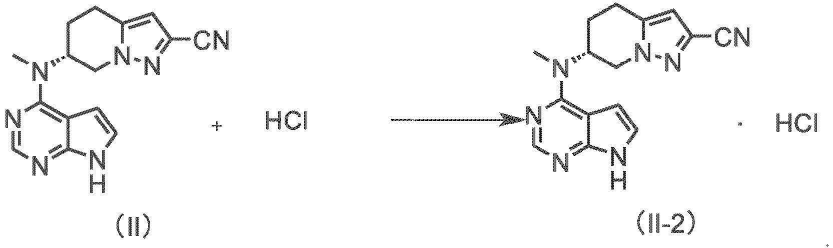

In a certain embodiment, the hydrochloric acid and the compound represented by formula (II) have a molar ratio of 1.05:1.

In a certain embodiment, the compound represented by formula (II) and the ethyl acetate have a mass-volume ratio of 40 mg/mL.

The present disclosure also provides a method for preparing the crystal form C of the compound represented by formula (II-2), which comprises the following steps: adding hydrochloric acid to a solution of the compound represented by formula (II) in ethanol to carry out a reaction shown in the following formula, and collecting a solid to obtain the crystal form C;

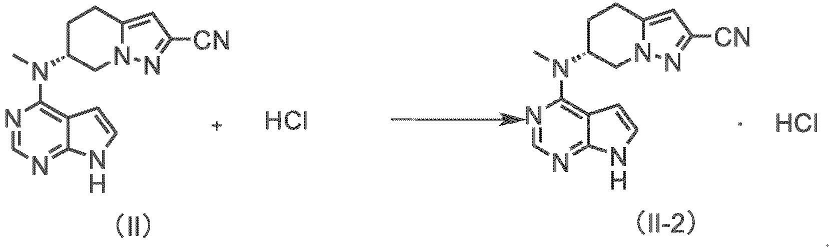

In a certain embodiment, the hydrochloric acid and the compound represented by formula (II) have a molar ratio of 1.05:1.

In a certain embodiment, the compound represented by formula (II) and the ethanol have a mass-volume ratio of 40 mg/mL.

The present disclosure also provides a method for preparing the crystal form D of the compound represented by formula (II-2), which comprises the following steps: adding hydrochloric acid to a solution of the compound represented by formula (II) in tetrahydrofuran to carry out a reaction shown in the following formula, and collecting a solid to obtain the crystal form D;

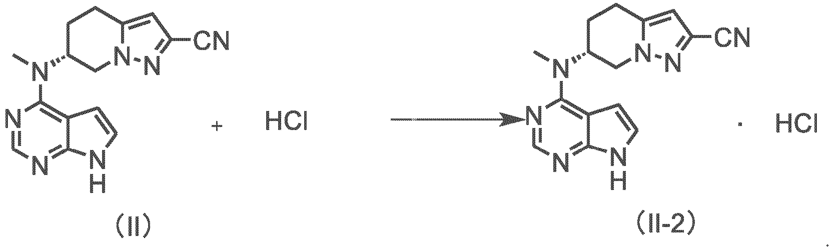

In a certain embodiment, the hydrochloric acid and the compound represented by formula (II) have a molar ratio of 1.05:1.

In a certain embodiment, the compound represented by formula (II) and the tetrahydrofuran have a mass-volume ratio of 40 mg/mL.

The present disclosure also provides a method for preparing the crystal form A of the compound represented by formula (II-3), which comprises the following steps: adding sulfuric acid to a solution of the compound represented by formula (II) to carry out a reaction shown in the following formula, and collecting a solid to obtain the crystal form A; a solvent of the solution is an acetonitrile aqueous solution;

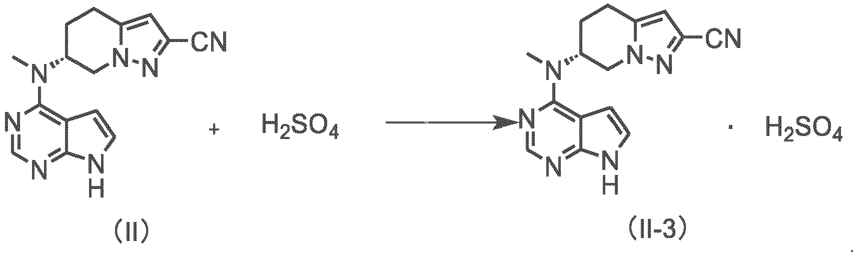

In a certain embodiment, the sulfuric acid and the compound represented by formula (II) have a molar ratio of 1.03:1.

In a certain embodiment, the compound represented by formula (II) and the acetonitrile aqueous solution have a mass-volume ratio of 30 mg/mL.

In a certain embodiment, the acetonitrile and the H₂O in the acetonitrile aqueous solution have a volume ratio of 19:1.

In a certain embodiment, the sulfuric acid has a molar concentration of 4 mol/L.

The present disclosure also provides a method for preparing the crystal form C of the compound represented by formula (II-3), which comprises the following steps: adding sulfuric acid to a solution of the compound represented by formula (II) in ethyl acetate to carry out a reaction shown in the following formula, and collecting a solid to obtain the crystal form C;

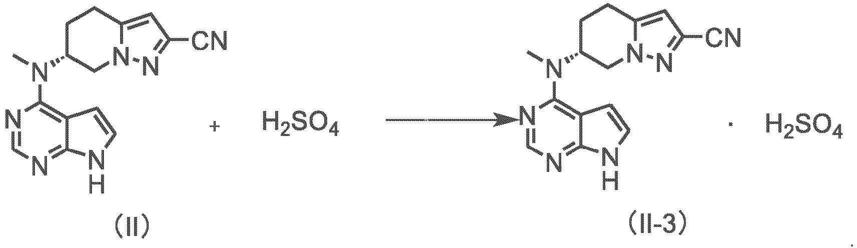

In a certain embodiment, the sulfuric acid and the compound represented by formula (II) have a molar ratio of 1.03:1.

In a certain embodiment, the compound represented by formula (II) and the ethyl acetate have a mass-volume ratio of 30 mg/mL.

In a certain embodiment, the sulfuric acid has a molar concentration of 4 mol/L.

The present disclosure also provides a method for preparing the crystal form D of the compound represented by formula (II-3), which comprises the following steps: adding sulfuric acid to a solution of the compound represented by formula (II) in ethanol to carry out a reaction shown in the following formula, and collecting a solid to obtain the crystal form D;

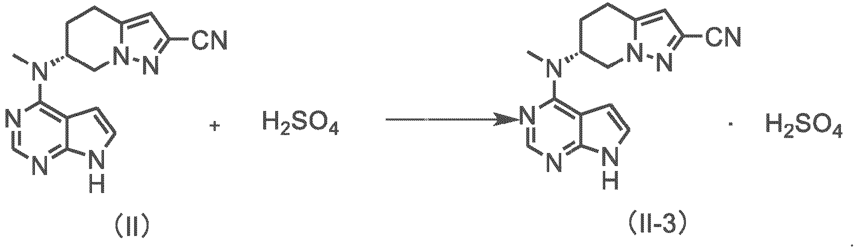

In a certain embodiment, the sulfuric acid and the compound represented by formula (II) have a molar ratio of 1.03:1.

In a certain embodiment, the compound represented by formula (II) and the ethanol have a mass-volume ratio of 30 mg/mL.

In a certain embodiment, the sulfuric acid has a molar concentration of 4 mol/L.

The present disclosure also provides a method for preparing the crystal form B of the hydrate of the compound represented by formula (II-3), which comprises the following steps: adding sulfuric acid into a solution of the compound represented by formula (II) in acetone to carry out a reaction shown in the following formula, and collecting a solid to obtain the crystal form B;

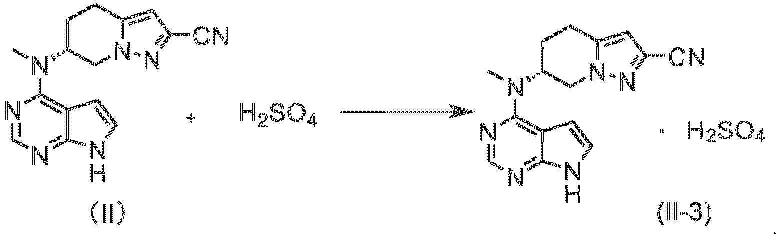

In a certain embodiment, the sulfuric acid and the compound represented by formula (II) have a molar ratio of 1.03:1.

In a certain embodiment, the compound represented by formula (II) and the acetone have a mass-volume ratio of 30 mg/mL.

In a certain embodiment, the sulfuric acid has a molar concentration of 4 mol/L.

In a certain embodiment, in preparing the crystal form A of the compound (II-1), the crystal forms A, B, C and D of compound (II-2), the crystal forms A, C and D of compound (II-3) and the crystal form B of the hydrate of compound (II-3), the compound represented by formula (II) is the crystal form A of the compound represented by formula (II).

The present disclosure also provides the crystal form of the compound represented by formula I, the salt thereof, the hydrate of the salt thereof and related crystal forms for use in the treatment of rheumatoid arthritis.

In a certain embodiment, the crystal forms in the application are the crystal forms A and B of the compound represented by (II), the crystal form A of the compound represented by (II-1), the crystal forms A, B, C and D of the compound represented by (II-2), the crystal forms A, C and D of the compound represented by (II-3), or the crystal form B of the hydrate of the compound represented by (II-3).

### Definition and description:

Unless otherwise indicated, the following terms or phrases used in this document are intended to have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The intermediate compounds of the present disclosure can be prepared by various synthetic methods known to those skilled in the art, including the embodiments described below, the embodiments formed by combining the embodiments described below with other chemical synthesis methods, and equivalent alternatives well-known to those skilled in the art. Preferred embodiments include, but are not limited to, the embodiments of the present disclosure. If the reaction temperature is not specified in the present disclosure, the reaction temperature is room temperature, and the room temperature is generally 20-35°C.

The chemical reactions of the embodiments of the present disclosure are carried out in a suitable solvent, and the solvent should be suitable for the chemical change of the present disclosure, and the reagents and materials required. In order to obtain the compounds of the present disclosure, it is sometimes necessary for those skilled in the art to modify or select the synthetic steps or reaction schemes based on the existing embodiments.

The above preferred conditions can be combined arbitrarily to obtain preferred embodiments of the present disclosure without violating common knowledge in the art.

The reagents and raw materials used in the present disclosure are commercially available.

The present disclosure has the positive progressive effects that:

the crystal forms A and B of the compound represented by (II), the crystal form A of the compound represented by (II-1), the crystal forms A, B, C and D of the compound represented by (II-2), the crystal forms A, C and D of the compound represented by (II-3), and the crystal form B of the hydrate of the compound represented by (II-3) provided in the present disclosure have stable properties, good hygroscopicity, and good pharmaceutical prospects.

### BRIEF DESCRIPTION OF THE DRAWINGS

FIG. 1 is an XRPD pattern of the crystal form A of the compound represented by formula (II) measured by Cu-Kα radiation.
FIG. 2 is a TGA/DSC pattern of the crystal form A of the compound represented by formula (II).
FIG. 3 is an XRPD pattern of the crystal form B of the compound represented by formula (II) measured by Cu-Kα radiation.
FIG. 4 is an XRPD pattern of the crystal form A of the compound represented by formula (II-1) measured by Cu-Kα radiation.
FIG. 5 is a TGA/DSC pattern of the crystal form A of the compound represented by formula (II-1).
FIG. 6 is an XRPD pattern of the crystal form A of the compound represented by formula (II-2) measured by Cu-Kα radiation.
FIG. 7 is a TGA/DSC pattern of the crystal form A of the compound represented by formula (II-2).
FIG. 8 is an XRPD pattern of the crystal form B of the compound represented by formula (II-2) measured by Cu-Kα radiation.
FIG. 9 is a TGA/DSC pattern of the crystal form B of the compound represented by formula (II-2).
FIG. 10 is an XRPD pattern of the crystal form C of the compound represented by formula (II-2) measured by Cu-Kα radiation.
FIG. 11 is a TGA/DSC pattern of the crystal form C of the compound represented by formula (II-2).
FIG. 12 is an XRPD pattern of the crystal form D of the compound represented by formula (II-2) measured by Cu-Kα radiation.
FIG. 13 is a TGA/DSC pattern of the crystal form D of the compound represented by formula (II-2).
FIG. 14 is an XRPD pattern of the crystal form A of a compound represented by formula (II-3) measured by Cu-Kα radiation.
FIG. 15 is a TGA/DSC pattern of the crystal form A of the compound represented by formula (II-3).
FIG. 16 is an XRPD pattern of the crystal form B of the hydrate of the compound represented by formula (II-3) measured by Cu-Kα radiation.
FIG. 17 is a TGA/DSC pattern of the crystal form B of the hydrate of the compound represented by formula (II-3).
FIG. 18 is an XRPD pattern of the crystal form C of the compound represented by formula (II-3) measured by Cu-Kα radiation.
FIG. 19 is a TGA/DSC pattern of the crystal form C of the compound represented by formula (II-3).
FIG. 20 is an XRPD pattern of the crystal form D of the compound represented by formula (II-3) measured by Cu-Kα radiation.
FIG. 21 is a TGA/DSC pattern of the crystal form D of the compound represented by formula (II-3).

### DETAILED DESCRIPTION OF THE PREFERRED EMBODIMENT

The present disclosure will be further described below with reference to embodiments. Experimental methods without specific conditions in the following embodiments are selected according to conventional methods and conditions, or according to the commercial specification.

In the present disclosure, Boc: *tert*-butoxycarbonyl; Cbz: benzyloxycarbonyl; Fmoc: fluorenylmethoxycarbonyl; Alloc: allyloxycarbonyl; Teoc: trimethylsilylethoxycarbonyl; Pht: phthaloyl; Tos: *p*-toluenesulfonyl; Tfa: trifluoroacetyl; Trt: trityl; Dmb: 2,4-dimethoxybenzyl; PMB: *p*-methoxybenzyl; MOM: methoxymethylene, Bn: benzyl, THP: tetrahydropyranyl, Tr: trityl, Ac; acetyl, Bz: benzoyl, Piv: pivaloyl, TMS: trimethylsilyl, TES: triethylsilyl, TBS: *tert*butyldimethylsilyl, TBDPS: *tert*-butyldiphenylsilyl, Ms: methylsulfonyl, Ts: *p*-toluenesulfonyl.

All solvents used in the present disclosure are commercially available and can be directly used without further purification. The reactions are generally carried out under inert nitrogen and in anhydrous solvents. Proton NMR data were recorded on a Bruker Avance III 400 (400MHz) spectrometer, and chemical shifts were expressed in (ppm) at the low field of tetramethylsilane. Mass spectrometry was measured on Agilent 1200 Series Plus 6110 (&1956A). LC/MS or Shimadzu MS includes a DAD: SPD-M20A (LC) and Shimadzu Micromass 2020 detector. The mass spectrometer is equipped with an electrospray ion source (ESI) operating in either positive or negative mode.

**Table-12 Comparison table of Chinese and English names of solvents selected in the present disclosure**

| **English** | **Chinese** | **English** | **Chinese** |
|---|---|---|---|
| MeOH | Methanol | 1,4-dioxane | 1,4-dioxane |
| EtOH | Ethanol | ACN | Acetonitrile |
| IPA | Isopropanol | DCM | Dichloromethane |
| Acetone | Propanone | CHCl₃ | Chloroform |
| MIBK | Methyl isobutyl ketone | toluene | Methylbenzene |
| EtOAc | Ethyl acetate | n-heptane | Heptane |
| IPAc | Isopropyl acetate | DMSO | Dimethyl sulfoxide |
| MTBE | Methyl *tert*-butyl ether | DMAc | *N,N*-Dimethylacetamide |
| THF | Tetrahydrofuran | NMP | *N*-Methylpyrrolidone |
| 2-MeTHF | 2-Methyltetrahydrofuran | H₂O | Water |

Compounds are named manually or by software, and the commercially available compounds use their vendor directory names.

### X-ray powder diffractometer (XRPD)

**Table-13 XRPD test parameters**

| **Parameters** | **Instrument 1** | **Instrument 2** |
|---|---|---|
| Model | Empyrean | X' Pert3 |
| X-Ray | Cu, Kα, Kα1 (Å):1.540598, Kα2 (Å):1.544426 | Cu, Kα, Kα1 (Å):1.540598, Kα2 (Å):1.544426 |
| | Kα2/Kα1 intensity ratio:0.50 | Kα2/Kα1 intensity ratio:0.50 |
| X-ray light tube settings | 45 kV, 40 mA | 45 kV, 40 mA |
| Divergence slit | 1/8° | 1/8° |
| Scanning mode | Continuous | Continuous |
| Scanning range (°2θ) | 3-40 | 3-40 |
| Scanning time per step (seconds) | 17.8 | 46.7 |
| Scanning step size | 0.0167 | 0.0263 |
| (°2θ) | | |
| Test time | About 5 minutes 30 seconds | About 5 minutes |

Thermogravimetric Analysis (TGA) and Differential Scanning Calorimetry (DSC)

TGA and DSC patterns were collected on TA Q5000/5500 thermogravimetric analyzer and TA Q2000/2500 differential scanning calorimeter respectively, and Table-14 lists the test parameters.

**Table-15 DSC and TGA test parameters**

| **Parameters** | **TGA** | **DSC** |
|---|---|---|
| Method | Linear heating | Linear heating |
| Sample tray | Aluminum tray, open | Aluminum tray, capped/uncapped |
| Temperature range | Room temperature - setted end temperature | 25°C - setted end temperature |
| Scanning rate (°C/min) | 10 | 10 |
| Protective gas | Nitrogen | Nitrogen |

**Table-16 High performance liquid chromatography test conditions**

| **Liquid chromatograph** | **Agilent 1290 detector** | |
|---|---|---|
| Chromatographic column | HSS T3, 50×2.1 mm, 1.8 µm | |
| | A: 0.0375% TFA aqueous solution | |
| | B: Can solution of 0.01875% TFA | |
| | **Time (min)** | **%B** |
| Mobile phase | 0.00 | 5 |
| | 2.00 | 5 |
| | 6.00 | 60 |
| | 6.50 | 60 |
| | 6.51 | 5 |
| | 8.00 | 5 |

| **Liquid chromatograph** | **Agilent 1290 detector** | |
|---|---|---|
| Running time | 8.0 min | |
| Mobile phase flow rate | 0.8 mL/min | |
| Injection volume | 5 µL | |
| Detection wavelength | UV at 220 nm/254 nm | |
| Column temperature | 50°C | |
| Injector temperature | RT | |
| Diluent | ACN/H₂O=1:1 (v:v) | |

### Embodiment 1

A preparation method for a compound of formula 10 was as follows:

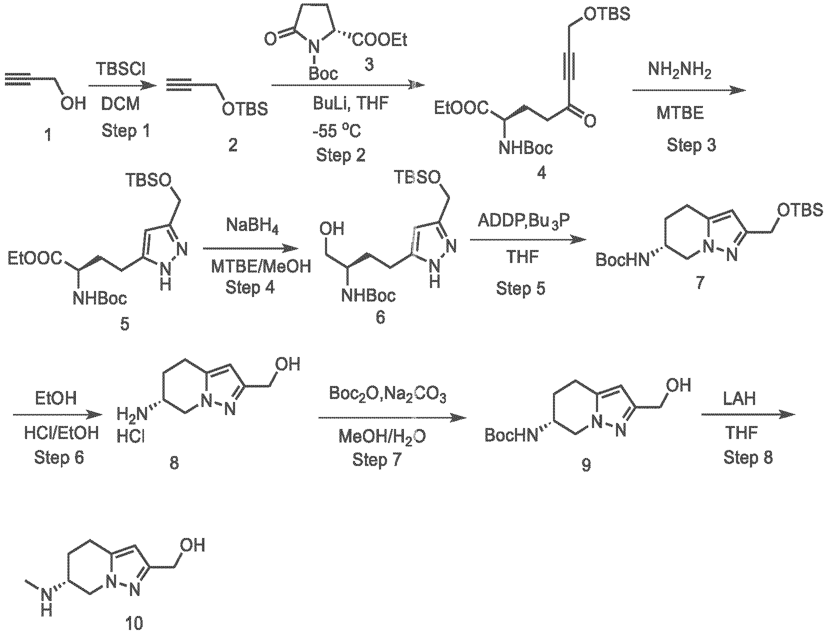

### Step 1:

To a reactor was added DCM (220 g), stirring was started, and then TBSCl (61.5 g) was added thereto. The mixture was stirred and dissolved to clarification, added with a compound represented by formula 1 (21.8 g) and imidazole (39.7 g), introduced with nitrogen, and heated to -2 to 5°C. The temperature was kept and the reaction was carried out for 3 h. After the reaction, 110.0 g of water was added thereto, the mixture was stirred for 0.5 h, left to stand, and layered. The organic phase was concentrated to obtain a product 2;

### Step 2:

To a reactor was added the product 2 (72.3 g) obtained in step 1, THF (430 g) and a compound (63.8 g) of formula (3). The mixture was stirred, nitrogen was introduced thereto, and the temperature was heated to 40 to 50°C, the temperature was kept and the reaction was carried out for 1 h. Then butyllithium (88 g) was added thereto, the temperature was continuously kept and the reaction was carried out for 4 h. After the reaction was completed, water (480 g) and ammonium chloride (30 g) were added thereto and stirred for 0.5 h, and MTBE (200 g) was added thereto and stirred for 0.5 h. The mixture was left to stand, and layered, and the organic phase was collected to obtain a product 4 solution; ¹H NMR (400 MHz, CDCl₃) δ =4.971 (s, 1H), 4.33(s, 2H), 4.177 - 4.144 (m, H), 4.100-4.039(m, 2H), 2.621-2.525 (m, 2H), 2.054-1.912 (m,2H), 1.312 (s,9H), 1.172 -1.127 (m, 3H), 0.781 (s, 9H), 0.001 (s, 6H).

### Step 3:

To the reactor in step 2 was dropwise added 80% hydrazine hydrate (19.8 g), and the temperature was kept at 0 to 5°C. After the dropwise addition, the mixture was heated to 20 to 30°C, the temperature was kept and the reaction was carried out for 4 h. After the reaction was completed, water (400.0 g) was added thereto, the mixture was stirred for 0.5 h, left to stand, and layered. The organic phase was concentrated to obtain a product 5;

### Step 4:

To a reactor was added the product 5 (250 g) and MTBE (400 g), nitrogen was introduced thereto, and the temperature was cooled to 0 to 5°C. Sodium borohydride (30 g) was slowly added thereto, then methanol (120 g) was added thereto. The mixture was heated to room temperature, the temperature was kept and the reaction was carried out for 20 h. After the reaction was completed, water (480 g) and ammonium chloride (40 g) were added thereto and stirred for 0.5 h. The mixture was left to stand, and layered, and the organic phase was concentrated to obtain a product 6;

### Step 5:

To a reactor was added the product 6 (145 g) and THF (630 g), nitrogen was introduced thereto, and the temperature was cooled to 0 to 5°C. Tributylphosphine (40g) was added thereto, stirred, and ADDP was added thereto in batches. The mixture was heated to room temperature, the temperature was kept and the reaction was carried out for 20 h. After the reaction was completed, the mixture was filtered and concentrated to obtain a product 7;

### Step 6:

To a reactor was added 30% (HCl/EtOH, 180 g), cooled to 0 to 5°C, then the product 7 (160 g) was added thereto. The mixture was heated to room temperature, the temperature was kept and the reaction was carried out for 4 h. After the reaction was completed, the mixture was cooled to room temperature, filtered and dried to obtain a product 8;

### Step 7:

To a reactor was added the product 8 (32 g), water (320 g) and sodium carbonate solution (mass fraction of 10%, 180 g), and the mixture was stirred and cooled 0 to 5°C. Then Boc anhydride/methanol solution (50 g of Boc anhydride, 150 g of methanol) was added dropwise thereto. After the dropwise addition, the mixture was heated to room temperature, the temperature was kept and the reaction was carried out for 10 h. After the reaction was completed, the mixture was left to stand, layered, and the organic phase was concentrated to obtain a product 9;

### Step 8:

To a reactor was added THF (90 mL), nitrogen was introduced thereto, and the temperature was lowered to 0 to 5°C. Then lithium aluminum hydride (3.4 g) was added thereto, and the temperature was raised to 50°C, and then the product 9/THF solution (6 g/30 mL) was added dropwise thereto. After the dropwise addition, the temperature was raised to 70°C, and the temperature was kept and the reaction was carried out for 3 h. After the reaction was completed, the temperature was lowered to 5 to 10°C, water (30 g) was added thereto, and the mixture was stirred, filtered, and dried to obtain a product 10.

### Embodiment 2

A synthesis method of a compound represented by formula (II) was as follows:

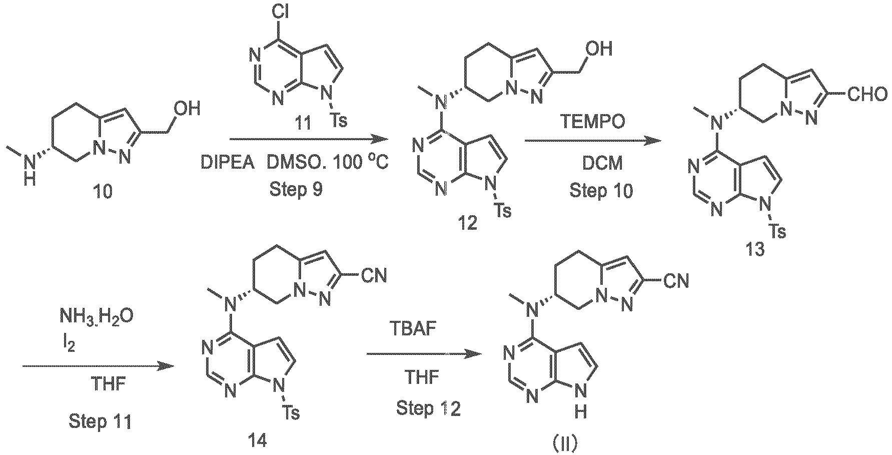

### Step 9:

To a reactor was added the product 10 (0.01 eq) in Embodiment 1, the compound represented by formula (II) (0.00103 eq), DMSO (100 mL) and DIPEA (0.02 eq), and the temperature was raised to 100 to 110°C. After the temperature was kept and the mixture was stirred for 4 h, the temperature was lowered to 20 to 30°C. Water (150 mL) was added thereto, the mixture was stirred for 1 h, filtered, and dried to obtain a product 12;

### Step 10:

To a reactor was added dichloromethane (200 g), the product 12 (15 g), 2,2,6,6-tetramethylpiperidine-1-oxide (TEMPO, 0.52 g), iodobenzene diacetate (11.7 g), reacted at room temperature for 10 h. After the reaction was completed, triethylamine (12 g) was added thereto, the mixture was stirred for 1 h, filtered, and dried to obtain a product 13;

### Step 11:

To a reactor was added the product 13 (73 g), ammonia water (25 mL) and iodine (12.4 g), and the mixture was stirred at room temperature for 12 h. After the reaction was completed, ethyl acetate (100 mL) was added thereto, stirred for 0.5 h, the mixture was left to stand, layered, and the organic phase was concentrated to obtain a product 14;

### Step 12:

To a reactor was added the product 14 (11 g), THF (100 mL) and TBAF (0.1 mol), the temperature was raised to 70°C, the temperature was kept and the reaction was carried out for 12 h. After the reaction was completed, the temperature was lowered to room temperature. 10% NaHCO₃ aqueous solution (100 mL) was added thereto, and the mixture was stirred at 20°C for 2 h, filtered, and dried to obtain a product 15, that is, the compound represented by formula (II);

MS (ESI) calculated for C₁₅H₁₅N₇ 293, found 294 [M + H]⁺; ¹H NMR (400 MHz,DMSO-*d₆*) δ = 12.80 - 12.56 (m, 1H), 8.43 - 8.32 (m, 1H), 7.49 - 7.38 (m, 1H), 7.01 - 6.88 (m, 1H), 6.88 - 6.74 (m, 1H), 5.31 - 5.17 (m, 1H), 4.52 - 4.36 (m, 2H), 3.45 - 3.41 (m, 3H), 3.13 - 3.04 (m, 1H), 3.01 - 2.91 (m, 1H), 2.38 - 2.29 (m, 1H), 2.17 - 2.09(m, 1H).

### Embodiment 3

A preparation method of a crystal form A of the compound represented by formula (II):

To a reactor was added 20 mg of the compound represented by formula (II) and 4 mL of tetrahydrofuran. After the mixture was stirred at room temperature to dissolve to clarification, 4 mL of ethyl acetate was slowly added dropwise thereto, and the mixture was stirred at room temperature for 24 h, filtered, and dried to obtain the crystal form A of the compound represented by formula (II).

### Embodiment 4

A preparation method of a crystal form A of the compound represented by formula (II):

To a reactor was added 20 mg of the compound represented by formula (II) and 4 mL of tetrahydrofuran. After the mixture was stirred at room temperature to dissolve to clarification, 5 mL of cyclohexane was slowly added dropwise thereto, and the mixture was stirred at room temperature for 24 h, filtered, and dried to obtain the crystal form A of the compound represented by formula (II).

The crystal form A has an XRPD pattern as shown in FIG. 1, and the TGA and DSC results are shown in FIG. 2. The TGA results showed that the sample had a weight loss of 2.1% when the temperature rose from 25.1°C to 250°C; the DSC curve had an endothermic peak at 330.46±5°C.

### Embodiment 5

A preparation method of a crystal form B of the compound represented by formula (II):
20 mg of the crystal form A of the maleate was weighed and placed into a reactor, NMP (10 mL) was added thereto to dissolve it. Antisolvent H₂O (4 mL) was added dropwise to the flask, stirred magnetically while dropwise addition, and stirred for 0.5 h after solid precipitation, and filtered to obtain the crystal form B of the compound represented by formula (II).

The crystal form B has an XRPD pattern as shown in FIG. 3.

### Embodiment 6

### A preparation method of a crystal form A of the compound represented by formula (II-1):

399.1 mg of the starting material, i.e., a sample of the crystal form A of the compound represented by formula (II) and 164.7 mg of maleic acid were weighed and placed in a 20 mL glass flask (the maleic acid and the sample of the crystal form A of the compound represented by formula (II) had a molar ratio of 1.04:1), 10 mL EtOAc was added thereto under magnetic stirring (about 750 rpm) and the mixture was dispersed into a uniform suspension. After the suspension was suspended and stirred at room temperature for 2 days, the solid was collected by vacuum filtration and dried under vacuum at room temperature for 20 h, then 490 mg of the solid was obtained (yield: 88.0%).

The crystal form A has an XRPD pattern as shown in FIG. 4, and the TGA and DSC results are shown in FIG. 5. The TGA results showed that the sample had a weight loss of 2.0% when the temperature was raised to 150°C; five endothermic signals were observed in the DSC curve at 50.7°C, 54.8°C, 59.0°C, 75.9°C and 167.7°C (peak temperature). The crystal form A had an acid-base molar ratio of 1:1.

### Embodiment 7

### A preparation method of a crystal form A of a compound represented by formula (II-2):

399.0 mg of the starting material, i.e., the sample of the crystal form A of the compound represented by formula (II) was weighed and placed in a 20 mL glass flask, 10 mL of ACN/H₂O (v:v = 19:1) was added thereto under magnetic stirring (about 750 rpm) and the mixture was dispersed into a uniform suspension, and 120 µL of concentrated hydrochloric acid (the concentrated hydrochloric acid had a specific concentration of37%) (the concentrated hydrochloric acid and the sample of the crystal form A of the compound represented by formula (II) had a molar ratio of 1.05:1) was slowly added thereto. After the mixture was suspended and stirred at room temperature for 6 days, the solid was collected by vacuum filtration and dried under vacuum at room temperature for 20 h, then 270 mg of the solid was obtained.

The crystal form A has an XRPD pattern as shown in FIG. 6, and the TGA/DSC results of a crystal form A of hydrochloride are shown in FIG. 7. The TGA results showed that the sample had a weight loss of 6.7% when the temperature was raised to 100°C; endothermic peaks were observed at 78.1°C, 92.2°C and 274.0°C (peak temperature) in the DSC curve. IC/HPLC test results showed that the crystal form A had an acid-base molar ratio of 1:1.

### Embodiment 8

### A preparation method of a crystal form B of the compound represented by formula (II-2):

399.0 mg of the starting material, i.e., the sample of the crystal form A of the compound represented by formula (II) was weighed and placed in a 20 mL glass flask, 10 mL of ethyl acetate was added thereto under magnetic stirring (about 750 rpm) and the mixture was dispersed into a uniform suspension, and 120 µL of concentrated hydrochloric acid (the concentrated hydrochloric acid and the sample of the crystal form A of the compound represented by formula (II) had a molar ratio of 1.05:1) was slowly added thereto. After the mixture was suspended and stirred at room temperature for 6 days, the solid was collected by vacuum filtration and dried under vacuum at room temperature for 20 h, then 281 mg of the solid was obtained.

The crystal form B has an XRPD pattern as shown in FIG. 8, and the crystal form B of the hydrochloride has a TGA/DSC pattern as shown in FIG. 9. The TGA results showed that the sample had a weight loss of 2.3% when the temperature was raised to 100°C; two endothermic peaks were observed at 68.3°C and 274.7°C (peak temperature) in the DSC curve. IC/HPLC test results showed that the crystal form B had an acid-base molar ratio of 1:1.

### Embodiment 9

### A preparation method of a crystal form C of the compound represented by formula (II-2):

399.0 mg of the starting material, i.e., the sample of the crystal form A of the compound represented by formula (II) was weighed and placed in a 20 mL glass flask, 10 mL of EtOH was added thereto under magnetic stirring (about 750 rpm) and the mixture was dispersed into a uniform suspension, and 120 µL of concentrated hydrochloric acid (the concentrated hydrochloric acid and the sample of the crystal form A of the compound represented by formula (II) had a molar ratio of 1.05:1) was slowly added thereto. After the mixture was suspended and stirred at room temperature for 6 days, the solid was collected by vacuum filtration and dried under vacuum at room temperature for 20 h, then 280 mg of the solid was obtained.

The crystal form C has an XRPD pattern as shown in FIG. 10, and the TGA/DSC results of the crystal form C of the hydrochloride are shown in FIG. 11. The TGA results showed that the sample had a weight loss of 1.3% when the temperature was raised to 100°C; an endothermic peak was observed at 275.1°C (peak temperature) in the DSC curve. IC/HPLC test results showed that the crystal form C had an acid-base molar ratio of 1:1.

### Embodiment 10

### A preparation method of a crystal form D of the compound represented by formula (II-2):

399.0 mg of the starting material, i.e., the sample of the crystal form A of the compound represented by formula (II) was weighed and placed in a 20 mL glass flask, 10 mL of tetrahydrofuran was added thereto under magnetic stirring (about 750 rpm) and the mixture was dispersed into a uniform suspension, and 120 µL of concentrated hydrochloric acid (the concentrated hydrochloric acid and the sample of the crystal form A of the compound represented by formula (II) had a molar ratio of 1.05:1) was slowly added thereto. After the mixture was suspended and stirred at room temperature for 6 days, the solid was collected by vacuum filtration and dried under vacuum at room temperature for 20 h, then 273 mg of the solid was obtained.

The crystal form D has an XRPD pattern as shown in FIG. 12, and the TGA/DSC results of the crystal form D of the hydrochloride are shown in FIG. 13. The TGA results showed that the sample had a weight loss of 2.9% when the temperature was raised to 100°C; overlapping endothermic peaks were observed at 273.7°C and 279.3°C (peak temperature) in the DSC curve. IC/HPLC test results showed that the crystal form D had an acid-base molar ratio of 1:1.

### Embodiment 11

### A preparation method of a crystal form A of the compound represented by formula (II-3):

399.3 mg of the sample of the crystal form A of the compound represented by formula (II) was weighed and placed in a 20 mL glass flask, 13 mL of ACN/H₂O (v:v = 19:1) was added thereto under magnetic stirring (about 750 rpm) and the mixture was dispersed into a uniform suspension, and 350 µL of 4M sulfuric acid (the sulfuric acid and the sample of the crystal form A of the compound represented by formula (II) had a molar ratio of 1.03:1) was slowly added dropwise thereto. After the mixture was suspended and stirred at room temperature for 2 days, the solid was collected by vacuum filtration and dried under vacuum at room temperature for 20 h, then 350 mg of the solid was obtained.

The crystal form A has an XRPD pattern as shown in FIG. 14, and the TGA/DSC results of the crystal form A of the sulfate are shown in FIG. 15. The TGA results showed that the sample had a weight loss of 8.1% when the temperature was raised to 150°C; two endothermic peaks were observed at 85.1°C and 126.7°C (peak temperature) in the DSC curve. IC/HPLC test results showed that the crystal form A had an acid-base molar ratio of 1:1.

### Embodiment 12

### A preparation method of a crystal form B of a hydrate of the compound represented by formula (II-3):

399.3 mg of the sample of the crystal form A of the compound represented by formula (II) was weighed and placed in a 20 mL glass flask, 13 mL of acetone was added thereto under magnetic stirring (about 750 rpm) and the mixture was dispersed into a uniform suspension, and 350 µL of 4M sulfuric acid (the sulfuric acid and the sample of the crystal form A of the compound represented by formula (II) had a molar ratio of 1.03:1) was added dropwise thereto. After the mixture was suspended and stirred at room temperature for 2 days, the solid was collected by vacuum filtration and dried under vacuum at room temperature for 20 h, then 350 mg of the solid (yield of 65.7%) was obtained.

The crystal form B has an XRPD pattern as shown in FIG. 16, and the TGA/DSC results of the crystal form B of the sulfate are shown in FIG. 17. The TGA results showed that the sample had a weight loss of 6.67% when the temperature was raised to 150°C; two endothermic peaks were observed at 98.0°C and 140.2°C (peak temperature) in the DSC curve. According to the weight loss of 6.67% of TGA, it is preliminarily determined that the crystal form B contained about 1.5 water of crystallization. IC/HPLC test results showed that the crystal form B had an acid-base molar ratio of 1:1.

### Embodiment 13

### A preparation method of a crystal form C of the compound represented by formula (II-3):

399.3 mg of the sample of the crystal form A of the compound represented by formula (II) was weighed and placed in a 20 mL glass flask, 13 mL of ethyl acetate was added thereto under magnetic stirring (about 750 rpm) and the mixture was dispersed into a uniform suspension, and 350 µL of 4M sulfuric acid (the sulfuric acid and the sample of the crystal form A of the compound represented by formula (II) had a molar ratio of 1.03:1) was added dropwise thereto. After the mixture was suspended and stirred at room temperature for 2 days, the solid was collected by vacuum filtration and dried under vacuum at room temperature for 20 h, then 351 mg of the solid was obtained.

The crystal form C has an XRPD pattern as shown in FIG. 18, and the TGA/DSC results of the crystal form C of the sulfate are shown in FIG. 19. The TGA results showed that the sample had a weight loss of 6.8% when the temperature was raised to 150°C; two endothermic peaks were observed at 69.3°C and 118.1°C (peak temperature) in the DSC curve. IC/HPLC test results showed that the crystal form C had an acid-base molar ratio of 1:1.

### Embodiment 14

### A preparation method of a crystal form D of the compound represented by formula (II-3):

399.3 mg of the sample of the crystal form A of the compound represented by formula (II) was weighed and placed in a 20 mL glass flask, 13 mL of ethanol was added thereto under magnetic stirring (about 750 rpm) and the mixture was dispersed into a uniform suspension, and 350 µL of 4M sulfuric acid (the sulfuric acid and the sample of the crystal form A of the compound represented by formula (II) had a molar ratio of 1.03:1) was added dropwise thereto. After the mixture was suspended and stirred at room temperature for 2 days, the solid was collected by vacuum filtration and dried under vacuum at room temperature for 20 h, then 350 mg of the solid was obtained.

The crystal form D has an XRPD pattern as shown in FIG. 20, and the TGA/DSC results of the crystal form D of the sulfate are shown in FIG. 21. The TGA results showed that the sample had a weight loss of 8.1% when the temperature was raised to 150°C; two endothermic peaks were observed at 78.5°C and 144.3°C (peak temperature) in the DSC curve. IC/HPLC test results showed that the crystal form C had an acid-base molar ratio of 1:1.

### Embodiment 15 Dynamic solubility evaluation

The dynamic solubility of the crystal form B of the sulfate, the crystal form A of the maleate and the crystal form C of the hydrochloride in water and three biological menstruums was evaluated.

The solubility of each sample rotated and mixed at 37°C with a solid feeding concentration of 10 mg/mL (calculated as a free state, 40 mg of solids were put into 4 mL of solvent) in four systems of water, SGF, FaSSIF, and FeSSIF was determined at different time points (1, 2, 4 and 24 h).

**Table-17 Summary of dynamic solubility test results**

| **Salt form** | **Menstruum** | **1 hour** | | **2 hours** | | **4 hours** | | **24 hours** | |
|---|---|---|---|---|---|---|---|---|---|
| | | S | pH | S | pH | S | pH | S | pH |
| Crystal form B of sulfate | H₂O | 3.7 | 2.0 | 3.8 | 2.2 | 3.7 | 2.1 | 4.0 | 1.9 |
| | SGF | 5.3 | 1.7 | 5.0 | 1.8 | 4.8 | 1.8 | 5.3 | 1.6 |
| | FaSSIF | 3.4 | 2.7 | 3.4 | 2.6 | 3.3 | 2.7 | 2.6 | 2.4 |
| | FeSSIF | 0.07 | 4.3 | 0.07 | 4.3 | 0.06 | 4.3 | 0.05 | 4.4 |
| Crystal form A of maleate | H₂O | 2.9 | 2.4 | 2.8 | 2.6 | 2.7 | 2.5 | 1.7 | 2.1 |
| | SGF | 6.8 | 2.1 | 6.4 | 2.1 | 6.6 | 2.1 | 3.9 | 1.9 |
| | FaSSIF | 0.98 | 3.0 | 0.96 | 2.9 | 0.98 | 2.9 | 0.77 | 2.8 |
| | FeSSIF | 0.06 | 4.6 | 0.05 | 4.5 | 0.05 | 4.5 | 0.03 | 4.6 |
| Crystal form C of hydrochloride | H₂O | 2.7 | 2.0 | 2.4 | 2.1 | 2.3 | 2.0 | 2.0 | 1.9 |
| | SGF | 5.6 | 1.8 | 5.3 | 1.8 | 5.1 | 1.8 | 4.3 | 1.7 |
| | FaSSIF | 0.67 | 2.7 | 0.64 | 2.6 | 0.59 | 2.6 | 0.49 | 2.6 |
| | FeSSIF | 0.02 | 4.6 | 0.02 | 4.6 | 0.02 | 4.6 | 0.01 | 4.7 |

| | | | | | | | | | |
|---|---|---|---|---|---|---|---|---|---|
| S: solubility (mg/mL). | | | | | | | | | |

### Embodiment 16 Hygroscopicity evaluation

The hygroscopicity of the crystal form B of the sulfate, the crystal form A of the maleate and the crystal form C of the hydrochloride was evaluated by DVS. The water adsorption (second adsorption curve) of the three salt forms at 25°C/80% RH is 7.62%, 1.35% and 4.77% respectively.

### Embodiment 17 Solid state stability

The chemical stability of the samples was determined by HPLC after placing the crystal form B of the sulfate, the crystal form A of the maleate and the crystal form C of the hydrochloride at 25°C/60% RH and 40°C/75% RH for 1 week, respectively. For the maleate, additional stability data for one month and two months storage at 25°C/60%RH and 40°C/75%RH (open) were collected, and using the crystal form A of maleate as the starting sample, stability data for 24 hours storage at 60°C (closed) were collected. Purity data are listed in Table 18. The results showed that none of the three salt forms were significantly degraded under the corresponding conditions, and none of the crystal forms were changed.

**Table-18 Summary of solid state stability evaluation**

| **Starting material** | **Condition** | **Time point** | **Purity (area%)** | **Purity/Initial Purity (%)** |
|---|---|---|---|---|
| Crystal form B of sulfate | Initial | - | 98.40 | -- |
| | 25°C/60%RH | 1 week | 97.98 | 99.57 |
| | 40°C/75%RH | 1 week | 98.06 | 99.65 |
| Crystal form A of maleate | Initial | - | 98.39 | -- |
| | 25°C/60%RH | 1 week | 98.14 | 99.75 |
| | | 1 month | 98.31 | 99.92 |
| | | 2 months | 98.78 | 99.90 |
| | 40°C/75%RH | 1 week | 98.08 | 99.68 |
| | | 1 month | 98.33 | 99.94 |
| | | 2 months | 98.84 | 99.91 |
| Crystal form A of maleate | Initial | - | 98.72 | -- |
| | 60°C/Closed | 24 hours | 98.66 | 99.94 |
| Crystal form C of hydrochloride | Initial | - | 99.03 | -- |
| | 25°C/60%RH | 1 week | 98.99 | 99.96 |
| | 40°C/75%RH | 1 week | 98.95 | 99.92 |

### Embodiment 18 Biological activity assay

### Experimental example 18- 1: In vitro activity assay of JAK1, JAK2, JAK3, TYK2 kinases

### Experimental materials

Recombinant human JAK1, JAK2, JAK3 and TYK2 protease, and main instruments and reagents were provided by Eurofins, UK.

### Experimental method

Dilution of JAK2, JAK3 and TYK2: 20 mM 3-(*N*-morpholino)propanesulfonic acid (MOPS), 1 mM EDTA, 0.01% Brij-35.5% glycerol, 0.1% β-mercaptoethanol, 1 mg/mL BSA; dilution of JAK1: 20 mM TRIS, 0.2 mM EDTA, 0.1% β-mercaptoethanol, 0.01% Brij-35.5% glycerol. All compounds were prepared into a 100% DMSO solution and brought to 50-fold the final assay concentration. The test compounds were diluted in 3-fold concentration gradient with 9 final concentrations from 10 µM to 0.001 µM, wherein DMSO content in the assay reaction was 2%. Working stock solutions of the compounds were added to the assay wells as the first component of the reaction, and then the remaining components were added according to the protocol detailed for the assay below.

### JAK1(h) enzymatic reaction

JAK1(h) was incubated with 20 mM Tris/HCl pH 7.5, 0.2 mM EDTA, 500 µM MGEEPLYWSFPAKKK, 10 mM magnesium acetate and [γ-³³P]-ATP (activity and concentration were determined as required). The reaction was started by adding the Mg/ATP mixture and terminated by adding 0.5% phosphoric acid after 40 min of incubation at room temperature. 10 µL of the reaction mixture was then added dropwise on a P30 filter pad and washed three times with 0.425% phosphoric acid and once with methanol within 4 minutes, then dried and analyzed using a scintillation counter.

### JAK2(h) enzymatic reaction

JAK2(h) was incubated with 8 mM MOPS pH 7.0, 0.2 mM EDTA, 100 µM KTFCGTPEYLAPEVRREPRILSEEEQEMFRDFDYIADWC, 10 mM magnesium acetate and [γ-³³P]-ATP (activity and concentration were determined as required). The reaction was started by adding the Mg/ATP mixture and terminated by adding 0.5% phosphoric acid after 40 min of incubation at room temperature. 10 µL of the reaction mixture was then added dropwise on a P30 filter pad and washed three times with 0.425% phosphoric acid and once with methanol within 4 minutes, then dried and analyzed using a scintillation counter.

### JAK3(h) enzymatic reaction

JAK3(h) was incubated with 8 mM MOPS pH 7.0, 0.2 mM EDTA, 500 µM GGEEEEYFELVKKKK, 10 mM magnesium acetate and [γ-³³P]-ATP (activity and concentration were determined as required). The reaction was started by adding the Mg/ATP mixture and terminated by adding 0.5% phosphoric acid after 40 min of incubation at room temperature. 10 µL of the reaction mixture was then added dropwise on a P30 filter pad and washed three times with 0.425% phosphoric acid and once with methanol within 4 minutes, then dried and analyzed using a scintillation counter.

### TYK2(h) enzymatic reaction

TYK2(h) was incubated with 8 mM MOPS pH 7.0, 0.2 mM EDTA, 250 µM GGMEDIYFEFMGGKKK, 10 mM magnesium acetate and [γ-³³P]-ATP (activity and concentration were determined as required). The reaction was started by adding the Mg/ATP mixture and terminated by adding 0.5% phosphoric acid after 40 min of incubation at room temperature. 10 µL of the reaction mixture was then added dropwise on a P30 filter pad and washed three times with 0.425% phosphoric acid and once with methanol within 4 minutes, then dried and analyzed using a scintillation counter.

### Data analysis

The IC₅₀ results were obtained by XLFIT5 (formula 205) from IDBS corporation, as shown in Table 19.

**Table 19. Results of in vitro screening for the compounds of the present disclosure**

| Compound | **JAK1** (IC₅₀, nM) | **JAK2** (IC₅₀, nM) | **JAK3** (IC₅₀, nM) | **TYK2** (IC₅₀, nM) |
|---|---|---|---|---|
| The compound represented by formula (II) prepared in Embodiment 2 | 1 | 8 | 118 | 15 |

**Conclusion:** The compounds of the present disclosure exhibited good selective inhibition on JAK1 and/or JAK2 in the *in vitro* activity assay of the 4 kinase subtypes JAK1, JAK2, JAK3 and TYK2.

### Experimental example 18-2: Permeability test

### Experimental materials

The transport buffer solution was HBSS (Hanks' balanced salt solution) and 10 mM HEPES [*N*-(2-hydroxyethyl)piperazine-*N*'-(2-ethanesulfonic acid) solution], with a pH value of 7.40 ± 0.05; Caco-2 cells were purchased from ATCC.

### Experimental method

Caco-2 cells were seeded at 1 × 10 ⁵ cells/cm² in 96-well BD insert plates of polyethylene terephthalate (PET), and the medium was refreshed every 4-5 days until day 21-28 to form a confluent monolayer. Test compounds were tested in a two-way format at 2 µM in duplicate. Digoxin was added at 10 µM in the two-way format, and nadolol and metoprolol were added at 2 µM in the two-way format. Final concentration of DMSO was adjusted to less than 1%. The plates were incubated in a CO₂ incubator at 37 ± 1 °C for 2 h with 5% CO₂ at saturated humidity without shaking. All samples were mixed with acetonitrile containing an internal standard, and centrifuged at 4000 rpm for 10 min. 100 microliters of supernatant was diluted with 100 microliters of distilled water for LC/MS/MS analysis. By LC/MS/MS analysis, the concentrations of the test compounds and the reference compound in the initial test article solution, the test article solution and the test compound solution using the peak area ratio of the analyte to the internal standard. After the transport test, the integrity of the Caco-2 cell monolayer was determined by fluorescein yellow exclusion reaction, and the apparent permeability coefficient and efflux ratio were calculated. The experimental results are shown in Table 20:

**Table 20 Permeability of the compound represented by formula (II) prepared in Embodiment 2**

| Compound name | Mean apparent permeability coefficient | | Efflux ratio |
|---|---|---|---|
| | Mean Pₐₚₚ (10⁻⁶ cm/s) | | |
| | A to B | B to A | |
| Nadolol | 0.16 | ND | - |
| Metoprolol | 18. 1 | ND | - |
| Digoxin | 0.04 | 13.88 | 349.25 |
| The compound represented by formula (II) prepared in Embodiment 2 | 28.58 | 31.18 | 1.09 |

**Conclusion:** the compound of the present disclosure has characteristic high permeability, and is favorable for realizing good target tissue concentration and oral bioavailability.

Note: ND: no detected.

### Experimental example 18-3: Pharmacokinetic (PK) test

Clear solutions obtained by dissolving the test compounds were administered to male mice (C57BL/6) or rats (SD) by tail vein injection and oral gavage (overnight fasting, 7-8 weeks old). After the administration of the test compounds, blood was collected from the mandibular vein and centrifuged to obtain plasma at 0.117, 0.333, 1, 2, 4, 7 and 24 h for the intravenous injection group (1 mg/kg), and at 0.25, 0.5, 1, 2, 4, 8 and 24 h for the oral gavage group (3 mg/kg). The plasma concentration was measured by LC-MS/MS, and the relevant pharmacokinetic parameters were calculated by WinNonlin^{™} Version 6.3 pharmacokinetic software using non-compartmental model linear logarithmic trapezoid method. The test results are as follows:

**Table 21 Results of PK test in mice for the compound represented by formula (II) prepared in Embodiment 2**

| PK parameters | Results |
|---|---|
| T_{1/2} (hr) | 1.89 |
| Cₘₐₓ (nM) | 6000 |
| AUC_{0-inf} (nM.hr) | 12765 |
| Bioavailability (%)^{a} | 88.4 |

| | |
|---|---|
| Note: T_{1/2}: half-life; Cₘₐₓ: peak concentration; AUC_{0-inf}: area under the plasma concentration-time curve from time 0 to infinity; Bioavailability: bioavailability. | |

**Conclusion:** the compound of the present disclosure has good oral bioavailability and higher exposure in mice, and is favorable for producing good *in-vivo* efficacy.

### Experimental example 18-4: In-vivo pharmacodynamic study of adjuvant-induced arthritis (AIA) in rats

### Experimental procedures:

The compounds disclosed herein were tested for their efficacy on arthritis using a model of adjuvant-induced arthritis in rat. Female, 160 to 180 gram Lewis rats were anesthetized with isoflurane and injected subcutaneously with 0.1 mL of Mycobacterium tuberculosis suspension in the left hind paw. The animals were grouped and administered with the test compounds after 13 days of modeling, for example, the rats were given different doses as shown in Table 4-2. The compound represented by formula (II) was dissolved in a mixed menstruum of [5% DMSO, 95% (12% SBE-β-CD), 0.5% MC]. The female Lewis rats were orally administered 2 times daily (8 animals in each group). The status of the rats was observed for two weeks during which the swelling of the foot volume was recorded and scored with the criterion shown in Table 22.

**Table 22. Clinical scoring criterion for arthritis**

| Score | Clinical symptoms |
|---|---|
| 0 | No erythema and redness |
| 1 | Erythema or mild redness near the tarsal bones or at the ankle joints or metatarsal bones, and erythema and redness in one toe |
| 2 | Slight erythema and redness of the ankle joints and metatarsal bones, or redness and erythema in two or more toes |
| 3 | Moderate erythema and swelling in the ankle joints, wrist joints, and metatarsals |
| 4 | Severe redness and swelling in the ankle joints, wrist joints, metatarsals and toes |

### Experimental results:

The two treatment groups of the compound represented by formula (II) showed significant relieving effect on the weight loss trend of animals caused by disease attack, and the low and medium dose groups (3 mg/kg and 10 mg/kg) showed significant difference since day 20 as compared to the solvent control group, suggesting good weight recovery effect. The compound represented by formula (II) inhibited the increase in arthritis clinical score and paw volume, and this inhibition was dose-dependent. The effect of 10 mg/kg of the compound represented by formula (II) demonstrated the most significant efficacy (significant difference from the solvent control group starting from day 15). The mean clinical score of arthritis for this group decreased from a peak of 6 points on day 13 to 1.4 points at the experimental endpoint on day 27, and was significantly different as compared to the solvent control group.

**Table 23. Inhibition on area under the clinical score curve (AUC)**

| Compound | Dosage (mg/kg) | AUC (%) |
|---|---|---|
| Menstruum control group | 0 | 0% |
| The compound represented by formula (II) prepared in Embodiment 2 | 3 | 49.6 |
| | 10 | 60.5 |

**Conclusion:** The compound represented by formula (II) prepared in Embodiment 2 of the present disclosure showed significant therapeutic effect (for the inhibition rate versus menstruum control group, P < 0.0001) at doses 3 mg/kg and 10 mg/kg, and the compound represented by formula (II) prepared in Embodiment 2 of the present disclosure showed a good dose-effect positive correlation (3 mg/kg and 10 mg/kg).

### Comparative embodiment 1

The present disclosure also performs salt-forming screening on L-aspartic acid, fumaric acid, L-tartaric acid and citric acid. Using the crystal form A of the compound represented by formula (II) as raw material, according to the basic pKa (4.47) of the compound and the rough solubility of the starting material in different solvents at room temperature, L-aspartic acid, fumaric acid, L-tartaric acid and citric acid were selected as acidic ligands (molar ratio of free state to acidic ligand was 1:1), and the specific steps of the screening test of five solvent systems as shown in Table 24 were as follows: about 20 mg of the starting crystal form A of the compound represented by formula (II) and equimolar amounts of the above acid were weighed into an HPLC vial, and 0.5 mL of the following solvent were added thereto and mixed to obtain a suspension. After suspending and stirring at room temperature for 3 days, the solid was separated by centrifugation and dried under vacuum at room temperature for about 4 hours. The results are shown in Table 24.

**Table 24**

| **No.** | **Ligand acid** | **ACN/H₂O (19:1, v:v)** | **Acetone** | **Ethyl acetate** | **Ethanol** | **Tetrahydrofuran** |
|---|---|---|---|---|---|---|
| 1 | L-Aspartic acid | Free state | Free state | Free state | Free state | Free state |
| 2 | Fumaric acid | Free state | Free state | Free state | Free state | Free state |
| 3 | L-Tartaric acid | Free state | Free state | Free state | Free state | Free state |
| 4 | Citric acid | Free state | Free state | Citrate | Free state | Free state |

As can be seen from Table 24, when the acid is L-aspartic acid, fumaric acid and L-tartaric acid, none of the above-mentioned five solvents can form a salt with the compound represented by formula (II). When the acid is citric acid, a salt can be formed only when the solvent is ethanol, and the other four solvents cannot prepare salt.

Although the specific embodiments of the present disclosure have been described above, those skilled in the art should understand that these are only examples, various changes or modifications can be made to these embodiments without departing from the principle and essence of the present disclosure. Therefore, the protection scope of the present disclosure is defined by the appended claims.

## Claims

1. A crystal form, a salt, a crystal form of the salt, a hydrate of the salt, or a crystal form of the hydrate of the salt of a compound represented by formula (II), 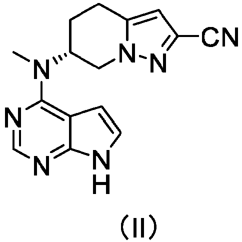 and acid in the salt is selected from maleic acid, hydrochloric acid and sulfuric acid.

2. The crystal form, a salt, a crystal form of the salt, a hydrate of the salt, or a crystal form of the hydrate of the salt of the compound represented by formula (II) as claimed in claim 1, wherein the acid in the salt and the compound represented by formula (II) have a molar ratio of (0.25-1.5):1.

3. The crystal form, a salt, a crystal form of the salt, a hydrate of the salt, or a crystal form of the hydrate of the salt of the compound represented by formula (II) as claimed in claim 2, wherein the salt of the compound represented by formula (II) is selected from any one of the following compounds:
a sulfate represented by formula (II-3) 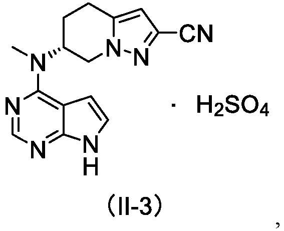
a hydrochloride represented by formula (II-2) 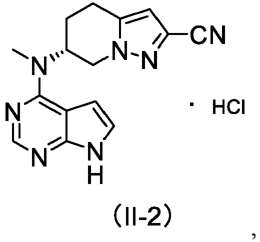
and a maleate represented by formula (II-1) 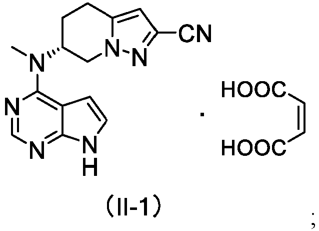 or,
the hydrate of the salt of the compound represented by formula (II) is selected from a hydrate of a sulfate represented by formula (II-3)

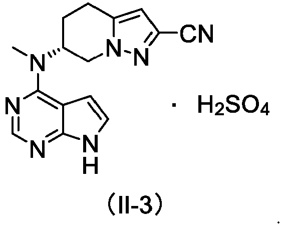

4. The crystal form, a salt, a crystal form of the salt, a hydrate of the salt, or a crystal form of the hydrate of the salt of the compound represented by formula (II) as claimed in claim 3, wherein,
the crystal form of the compound represented by formula (II) is a crystal form A, which has an X-ray powder diffraction pattern expressed by 2θ angles having diffraction peaks at 12.69°, 13.84°, 15.37°, 15.90°, 16.62°, 19.07°, 27.66° and 25.62°;
or, the crystal form of the compound represented by formula (II) is a crystal form B, which has an X-ray powder diffraction pattern expressed by 2θ angles having diffraction peaks at 12.40°, 13.31°, 15.75°, 22.16°, 23.72°, 25.49°, 26.12° and 26.87°;
or, the maleate represented by formula (II-1) is a crystal form A of the maleate, which has an X-ray powder diffraction pattern expressed by 2θ angles having diffraction peaks at 12.24°, 13.14°, 13.73°, 14.56°, 15.52°, 17.54°, 19.54°, 23.19°, 26.55° and 26.91°;
or, the hydrochloride represented by formula (II-2) is a crystal form A of the hydrochloride, which has an X-ray powder diffraction pattern expressed by 2θ angles having diffraction peaks at 7.20°, 7.85°, 8.63°, 10.82°, 21.25°, 21.78°, 24.12°, 25.56°, 26.11° and 27.03°;
or, the hydrochloride represented by formula (II-2) is a crystal form B of the hydrochloride, which has an X-ray powder diffraction pattern expressed by 2θ angles having diffraction peaks at 5.95°, 11.92°, 12.53°, 13.14°, 20.94°, 24.96°, 25.67°, 30.09° and 31.69°;
or, the hydrochloride represented by formula (II-2) is a crystal form C of the hydrochloride, which has an X-ray powder diffraction pattern expressed by 2θ angles having diffraction peaks at 5.89°, 11.77°, 13.21°, 13.52°, 15.75°, 23.51°, 25.51°, 24.65°, 26.31° and 27.15°;
or, the hydrochloride represented by formula (II-2) is a crystal form D of the hydrochloride, which has an X-ray powder diffraction pattern expressed by 2θ angles having diffraction peaks at 3.08°, 6.07°, 9.05°, 12.06°, 12.73°, 13.23°, 13.78° 15.08°, 21.28°, 24.94°, 26.06° and 31.72°;
or, the sulfate represented by formula (II-3) is a crystal form A of the sulfate, which has an X-ray powder diffraction pattern expressed by 2θ angles having diffraction peaks at 4.16°, 4.46°, 7.56°, 8.02°, 12.65°, 13.36°, 15.75°, 17.91°, 20.43°, 24.54°, 24.94°, 25.90° and 26.99°;
or, the sulfate represented by formula (II-3) is a crystal form C of the sulfate, which has an X-ray powder diffraction pattern expressed by 2θ angles having diffraction peaks at 6.09°, 12.17°, 13.25°, 15.60°, 16.09°, 17.45°, 18.66°, 22.61°, 23.62°, 24.44°, 25.04°, 25.89°, 26.30° and 26.62°;
or, the sulfate represented by formula (II-3) is a crystal form D of the sulfate, which has an X-ray powder diffraction pattern expressed by 2θ angles having diffraction peaks at 4.61°, 6.11°, 8.00°, 9.10°, 10.91°, 13.73°, 18.86°, 23.27°, 25.28°, and 25.97°;
or, the hydrate of the sulfate represented by formula (II-3) is a crystal form B of the hydrate of the sulfate, which has an X-ray powder diffraction pattern expressed by 2θ angles having diffraction peaks at 5.18°, 12.03°, 12.92°, 15.54°, 16.09°, 17.74°, 19.06°, 20.71°, 23.99°, 24.82° and 25.79°.

5. The crystal form, a salt, a crystal form of the salt, a hydrate of the salt, or a crystal form of the hydrate of the salt of the compound represented by formula (II) as claimed in claim 4, wherein when the crystal form of the compound represented by formula (II) is the crystal form A, in the X-ray powder diffraction pattern of the crystal form A expressed by 2θ angles, 2θ values are shown in the following table:
| 2θ Angle (°) | Relative intensity (%) |
|---|---|
| 6.33 | 9.91 |
| 12.69 | 97.83 |
| 13.84 | 69.04 |
| 14.91 | 27.95 |
| 15.37 | 76.70 |
| 15.90 | 45.28 |
| 16.62 | 66.33 |
| 19.07 | 40.00 |
| 22.15 | 17.81 |
| 24.96 | 24.95 |
| 25.62 | 100.00 |
| 26.95 | 30.65 |
| 27.66 | 29.86 |
| 30.19 | 5.78 |
| 37.92 | 3.13 |
| 38.88 | 7.58 |
;
or, the crystal form A of the compound represented by formula (II) has a differential scanning calorimetry (DSC) curve having an absorption peak with a peak temperature of 300.4°C; or, the crystal form A of the compound represented by formula (II) has a thermogravimetric analysis curve with a weight loss of 2.1% in a temperature range of 25.1°C to 250°C;
or, when the crystal form of the compound represented by formula (II) is the crystal form B, in the X-ray powder diffraction pattern of the crystal form B expressed by 2θ angles, 2θ values are shown in the following table:
| 2θ Angle (°) | Relative intensity (%) |
|---|---|
| 12.40 | 43.82 |
| 13.31 | 79.40 |
| 15.75 | 76.57 |
| 19.12 | 15.04 |
| 22.16 | 100.00 |
| 23.72 | 62.88 |
| 25.49 | 20.80 |
| 26.12 | 42.21 |
| 26.87 | 26.28 |
| 33.42 | 2.44 |
;
or, when the maleate represented by formula (II-1) is the crystal form A of the maleate, in the X-ray powder diffraction pattern of the crystal form A of the maleate expressed by 2θ angles, 2θ values are shown in the following table:
| 2θ Angle (°) | Relative intensity (%) |
|---|---|
| 4.40 | 14.26 |
| 8.76 | 9.45 |
| 12.24 | 21.69 |
| 13.14 | 24.97 |
| 13.73 | 100.00 |
| 14.56 | 19.33 |
| 15.52 | 16.05 |
| 16.42 | 2.53 |
| 17.54 | 35.08 |
| 18.89 | 2.59 |
| 19.54 | 32.55 |
| 20.37 | 11.04 |
| 23.19 | 18.59 |
| 23.70 | 5.19 |
| 24.59 | 5.61 |
| 25.52 | 7.75 |
| 26.55 | 15.18 |
| 26.91 | 15.77 |
| 27.11 | 9.87 |
| 27.98 | 3.54 |
| 29.30 | 6.68 |
| 30.01 | 3.87 |
| 36.24 | 1.53 |
| 37.44 | 2.47 |
;
or, the crystal form A of the maleate represented by formula (II-1) has a differential scanning calorimetry curve having an absorption peak with a peak temperature of 173.3°C; or, the crystal form A of the maleate represented by formula (II-1) has a thermogravimetric analysis curve with a weight loss of 1.86% in a temperature range of 29. 1°C to 150°C;
or, when the hydrochloride represented by formula (II-2) is the crystal form A of the hydrochloride, in the X-ray powder diffraction pattern of the crystal form A of the hydrochloride represented by formula (II-2) expressed by 2θ angles, 2θ values are shown in the following table:
| 2θ Angle (°) | Relative intensity (%) |
|---|---|
| 6.17 | 8.01 |
| 7.20 | 38.14 |
| 7.85 | 100.00 |
| 8.63 | 30.15 |
| 10.82 | 51.15 |
| 12.55 | 6.78 |
| 13.75 | 6.92 |
| 15.95 | 9.57 |
| 19.08 | 5.50 |
| 20.39 | 7.33 |
| 21.25 | 35.03 |
| 21.78 | 33.53 |
| 24.12 | 26.63 |
| 25.56 | 38.38 |
| 26.11 | 37.55 |
| 27.03 | 28.20 |
| 31.72 | 11.22 |
;
or, the crystal form A of the hydrochloride represented by formula (II-2) has a differential scanning calorimetry curve having three absorption peaks with peak temperatures of 78.1°C, 92.2°C and 274°C, respectively; or, the crystal form A of the hydrochloride represented by formula (II-2) has a thermogravimetric analysis curve with a weight loss of 6.71% in a temperature range of 27.2°C to 100°C;
or, when the hydrochloride represented by formula (II-2) is the crystal form B of the hydrochloride, in the X-ray powder diffraction pattern of the crystal form B of the hydrochloride expressed by 2θ angles, 2θ values are shown in the following table:
| 2θ Angle (°) | Relative intensity (%) |
|---|---|
| 5.95 | 100.00 |
| 11.05 | 13.61 |
| 11.92 | 61.44 |
| 12.53 | 31.64 |
| 13.14 | 48.62 |
| 16.67 | 8.26 |
| 19.31 | 6.81 |
| 20.94 | 90.42 |
| 22.04 | 5.05 |
| 23.19 | 13.88 |
| 24.96 | 43.42 |
| 25.67 | 49.06 |
| 30.09 | 18.58 |
| 31.69 | 36.23 |
| 39.46 | 14.32 |
;
or, the crystal form B of the hydrochloride represented by formula (II-2) has a differential scanning calorimetry curve having an absorption peak with a peak temperature of 274.7°C; or, the crystal form B of the hydrochloride represented by formula (II-2) has a thermogravimetric analysis curve with a weight loss of 2.34% in a temperature range of 31.4°C to 100°C;
or, when the hydrochloride represented by formula (II-2) is the crystal form C of the hydrochloride, in the X-ray powder diffraction pattern of the crystal form C of the hydrochloride represented by formula (II-2) expressed by 2θ angles, 2θ values are shown in the following table:
| 2θ Angle (°) | Relative intensity (%) |
|---|---|
| 5.89 | 60.87 |
| 11.77 | 100.00 |
| 12.58 | 22.24 |
| 13.21 | 71.07 |
| 15.75 | 59.51 |
| 17.71 | 13.70 |
| 18.41 | 17.93 |
| 23.51 | 90.26 |
| 24.65 | 18.91 |
| 25.51 | 25.11 |
| 26.31 | 55.95 |
| 27.15 | 43.81 |
;
or, the crystal form C of the hydrochloride represented by formula (II-2) has a differential scanning calorimetry curve having an absorption peak with a peak temperature of 275.1°C; or, the crystal form C of the hydrochloride represented by formula (II-2) has a thermogravimetric analysis curve with a weight loss of 1.32% in a temperature range of 33.7°C to 100°C;
or, when the hydrochloride represented by formula (II-2) is the crystal form D of the hydrochloride, in the X-ray powder diffraction pattern of the crystal form D of the hydrochloride expressed by 2θ angles, 2θ values are shown in the following table:
| 2θ Angle (°) | Relative intensity (%) |
|---|---|
| 3.08 | 79.15 |
| 6.07 | 100.00 |
| 9.05 | 30.52 |
| 12.06 | 29.69 |
| 12.73 | 34.25 |
| 13.23 | 25.66 |
| 13.78 | 25.88 |
| 14.53 | 17.45 |
| 15.08 | 26.72 |
| 16.32 | 14.81 |
| 17.45 | 11.57 |
| 18.20 | 10.95 |
| 19.31 | 6.12 |
| 21.28 | 79.37 |
| 22.02 | 15.50 |
| 23.36 | 10.90 |
| 24.94 | 28.49 |
| 26.06 | 36.10 |
| 29.23 | 5.77 |
| 30.51 | 11.55 |
| 31.72 | 40.73 |
| 36.83 | 5.80 |
;
or, the crystal form D of the hydrochloride represented by formula (II-2) has a differential scanning calorimetry curve having two absorption peaks with peak temperatures of 273.7°C and 279.3°C, respectively; or, the crystal form D of the hydrochloride represented by formula (II-2) has a thermogravimetric analysis curve with a weight loss of 2.92% in a temperature range of 28.3°C to 100°C;
or, when the sulfate represented by formula (II-3) is the crystal form A of the sulfate, in the X-ray powder diffraction pattern of the crystal form A of the sulfate expressed by 2θ angles, 2θ values are shown in the following table:
| 2θ Angle (°) | Relative intensity (%) |
|---|---|
| 4.16 | 81.60 |
| 4.46 | 71.99 |
| 7.56 | 100.00 |
| 8.02 | 63.19 |
| 9.67 | 3.76 |
| 12.65 | 30.66 |
| 13.36 | 77.29 |
| 14.36 | 14.12 |
| 15.75 | 40.01 |
| 17.91 | 38.83 |
| 19.02 | 21.35 |
| 20.43 | 34.62 |
| 22.34 | 11.00 |
| 24.54 | 61.38 |
| 24.94 | 65.92 |
| 25.90 | 49.58 |
| 26.99 | 43.14 |
| 27.87 | 11.82 |
;
or, the crystal form A of the sulfate represented by formula (II-3) has a differential scanning calorimetry curve having two absorption peaks with peak temperatures of 85.1°C and 126.7°C, respectively; or, the crystal form A of the sulfate represented by formula (II-3) has a thermogravimetric analysis curve with a weight loss of 8.14% in a temperature range of 28.4°C to 150°C;
or, when the crystal salt of the sulfate represented by formula (II-3) is the crystal form C of the sulfate, in the X-ray powder diffraction pattern of the crystal form C of the sulfate expressed by 2θ angles, 2θ values are shown in the following table:
| 2θ Angle (°) | Relative intensity (%) |
|---|---|
| 6.09 | 99.99 |
| 9.87 | 8.42 |
| 10.84 | 8.31 |
| 12.17 | 91.39 |
| 13.25 | 28.45 |
| 15.60 | 28.16 |
| 16.09 | 43.58 |
| 17.45 | 31.44 |
| 18.66 | 100.00 |
| 19.80 | 25.64 |
| 20.88 | 22.81 |
| 22.61 | 32.22 |
| 23.62 | 35.33 |
| 24.44 | 30.65 |
| 25.04 | 31.87 |
| 25.89 | 63.39 |
| 26.30 | 43.49 |
| 26.62 | 62.05 |
| 27.43 | 21.62 |
| 27.76 | 15.17 |
;
or, the crystal form C of the sulfate represented by formula (II-3) has a differential scanning calorimetry curve having two absorption peaks with peak temperatures of 69.3°C and 118.1°C, respectively; or, the crystal form C of the sulfate represented by formula (II-3) has a thermogravimetric analysis curve with a weight loss of 6.82% in a temperature range of 28.2°C to 150°C;
or, when the sulfate represented by formula (II-3) is the crystal form D of the sulfate, in the X-ray powder diffraction pattern of the crystal form D of the sulfate expressed by 2θ angles, 2θ values are shown in the following table:
| 2θ Angle (°) | Relative intensity (%) |
|---|---|
| 4.61 | 68.84 |
| 6.11 | 29.48 |
| 8.00 | 45.48 |
| 9.10 | 64.58 |
| 10.05 | 18.88 |
| 10.91 | 46.90 |
| 12.72 | 24.73 |
| 13.73 | 54.06 |
| 16.18 | 23.96 |
| 18.86 | 100.00 |
| 20.53 | 14.88 |
| 23.27 | 39.33 |
| 25.28 | 30.71 |
| 25.97 | 55.48 |
| 31.91 | 13.58 |
| 34.12 | 5.79 |
| 38.61 | 4.36 |
;
or, the crystal form D of the sulfate represented by formula (II-3) has a differential scanning calorimetry curve having two absorption peaks with peak temperatures of 78.5°C and 144.3°C, respectively; or, the crystal form D of the sulfate represented by formula (II-3) has a thermogravimetric analysis curve with a weight loss of 8.13% in a temperature range of 23.3°C to 150°C;
or, when the hydrate of the salt of the compound represented by formula (II) is the hydrate of the sulfate represented by formula (II-3), water in the crystal form B of the hydrate of the sulfate represented by formula (II-3) and the sulfate represented by formula (II-3) can have a number ratio of 1.5; or, in the X-ray powder diffraction pattern of the crystal form B of the hydrate of the sulfate represented by formula (II-3) expressed by 2θ angles, 2θ values are shown in the following table:
| 2θ Angle | Relative intensity |
|---|---|
| 5.18 | 45.44 |
| 7.95 | 11.00 |
| 8.82 | 19.56 |
| 10.23 | 16.98 |
| 12.03 | 24.82 |
| 12.92 | 73.75 |
| 15.54 | 47.91 |
| 16.09 | 90.49 |
| 17.74 | 41.11 |
| 19.06 | 38.98 |
| 20.71 | 33.34 |
| 23.99 | 100.00 |
| 24.82 | 22.22 |
| 25.79 | 56.92 |
;
or, the crystal form B of the hydrate of the sulfate represented by formula (II-3) has a differential scanning calorimetry curve having two absorption peaks with peak temperatures of 98°C and 140.2°C, respectively; or, the crystal form B of the hydrate of the sulfate represented by formula (II-3) has a thermogravimetric analysis curve with a weight loss of 6.67% in a temperature range of 28.6°C to 150°C.

6. A method for preparing the crystal form, a salt, a crystal form of the salt, a hydrate of the salt, or a crystal form of the hydrate of the salt of the compound represented by formula (II) as claimed in any one of claims 1 to 5, wherein the method for preparing the salt of the compound represented by formula (II) comprises the following steps: performing a salt-forming reaction of the compound represented by formula (II) as claimed in any one of claims 1 to 3 and the acid as claimed in claim 1 in a solvent; preferably, the solvent is selected from one or more of tetrahydrofuran, 2-methylfuran, ethyl acetate, cyclohexane, dimethyl sulfoxide, water, *n*-butanol, isopropanol, ethanol, *N*-methylpyrrolidone, acetonitrile, acetone, butanone, methyl isobutyl ketone, toluene, dichloromethane, 1,4-dioxane and anisole; preferably, the solvent is selected from one or more of tetrahydrofuran, ethyl acetate, cyclohexane, dimethyl sulfoxide, ethanol, water, *n*-butanol, *N*-methylpyrrolidone, acetonitrile and butanone; more preferably, the solvent is selected from tetrahydrofuran, acetone, ethanol, ethyl acetate and acetonitrile aqueous solution.

7. The method for preparing the crystal form, a salt, a crystal form of the salt, a hydrate of the salt, or a crystal form of the hydrate of the salt of the compound represented by formula (II) as claimed in claim 6, wherein,
when the compound represented by formula (II) is the crystal form A, the method for preparing the crystal form A of the compound represented by formula (II) comprises the following steps: adding an antisolvent into a solution of the compound represented by formula (II) in tetrahydrofuran at room temperature, and obtaining the crystal form A after crystallization; the antisolvent is an ester solvent or an alkane solvent;
or, when the compound represented by formula (II) is the crystal form B, the method for preparing the crystal form B of the compound represented by formula (II) comprises the following steps: dissolving the crystal form A of the compound represented by formula (II-1) in NMP at room temperature, adding water, and obtaining the crystal form B after crystallization;
or, when the salt of the compound represented by formula (II) is the maleate represented by formula (II-1) and the maleate represented by formula (II-1) is the crystal form A of the maleate, the method for preparing the crystal form A of the maleate comprises the following steps: dispersing the compound represented by formula (II) and maleic acid in an ester solvent to carry out a reaction shown in the following formula, and collecting a solid to obtain the crystal form A; 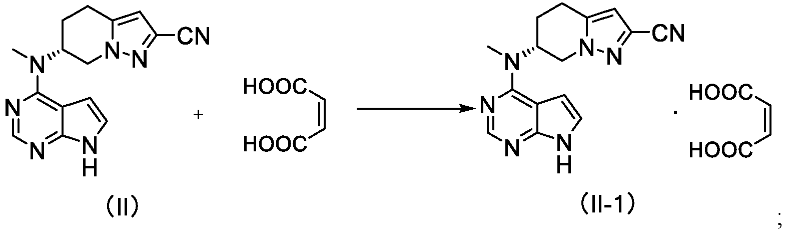
or, when the salt of the compound represented by formula (II) is the hydrochloride represented by formula (II-2) and the hydrochloride represented by formula (II-2) is the crystal form A of the hydrochloride, the method for preparing the crystal form A of the hydrochloride comprises the following steps: adding hydrochloric acid to a solution of the compound represented by formula (II) to carry out a reaction shown in the following formula, and collecting a solid to obtain the crystal form A; a solvent of the solution is an acetonitrile aqueous solution; 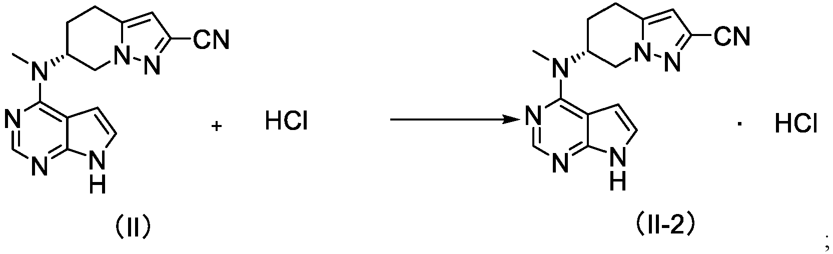
or, when the salt of the compound represented by formula (II) is the hydrochloride represented by formula (II-2) and the hydrochloride represented by formula (II-2) is the crystal form B of the hydrochloride, the method for preparing the crystal form B of the hydrochloride comprises the following steps: adding hydrochloric acid to a solution of the compound represented by formula (II) in ethyl acetate to carry out a reaction shown in the following formula, and collecting a solid to obtain the crystal form B; 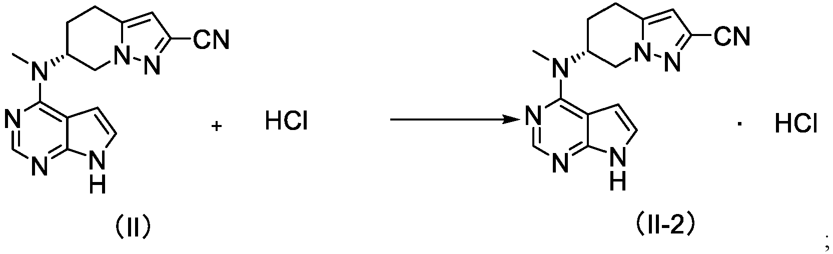
or, when the salt of the compound represented by formula (II) is the hydrochloride represented by formula (II-2) and the hydrochloride represented by formula (II-2) is the crystal form C of the hydrochloride, the method for preparing the crystal form C of the hydrochloride comprises the following steps: adding hydrochloric acid to a solution of the compound represented by formula (II) in ethanol to carry out a reaction shown in the following formula, and collecting a solid to obtain the crystal form C; 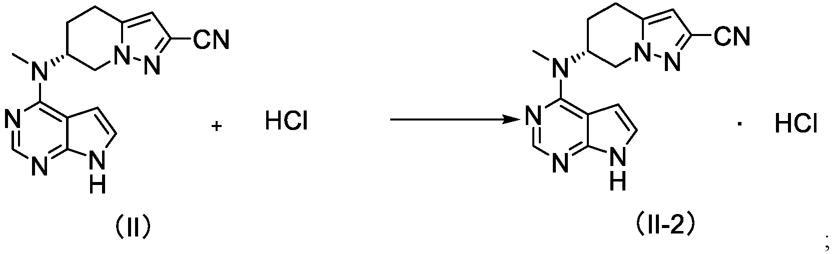
or, when the salt of the compound represented by formula (II) is the hydrochloride represented by formula (II-2) and the hydrochloride represented by formula (II-2) is the crystal form D of the hydrochloride, the method for preparing the crystal form D of the hydrochloride comprises the following steps: adding hydrochloric acid to a solution of the compound represented by formula (II) in tetrahydrofuran to carry out a reaction shown in the following formula, and collecting a solid to obtain the crystal form D; 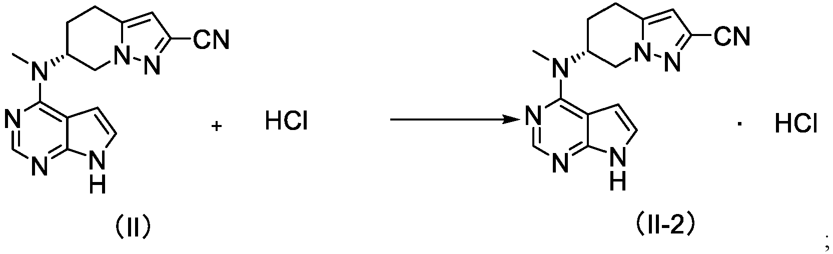
or, when the salt of the compound represented by formula (II) is the sulfate represented by formula (II-3) and the sulfate represented by formula (II-3) is the crystal form A of the sulfate, the method for preparing the crystal form A of the sulfate comprises the following steps: adding sulfuric acid to a solution of the compound represented by formula (II) to carry out a reaction shown in the following formula, and collecting a solid to obtain the crystal form A; a solvent of the solution is an acetonitrile aqueous solution; 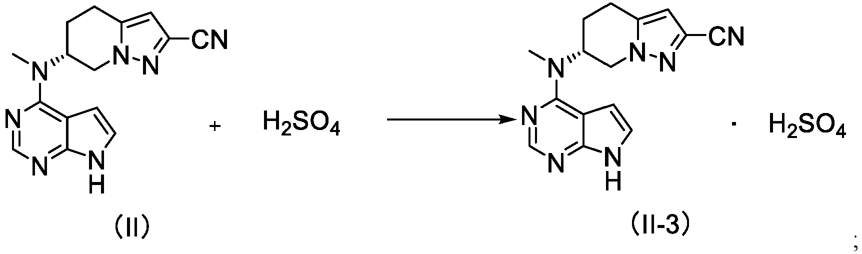
or, when the salt of the compound represented by formula (II) is the sulfate represented by formula (II-3) and the sulfate represented by formula (II-3) is the crystal form C of the sulfate, the method for preparing the crystal form C of the sulfate comprises the following steps: adding sulfuric acid to a solution of the compound represented by formula (II) in ethyl acetate to carry out a reaction shown in the following formula, and collecting a solid to obtain the crystal form C; 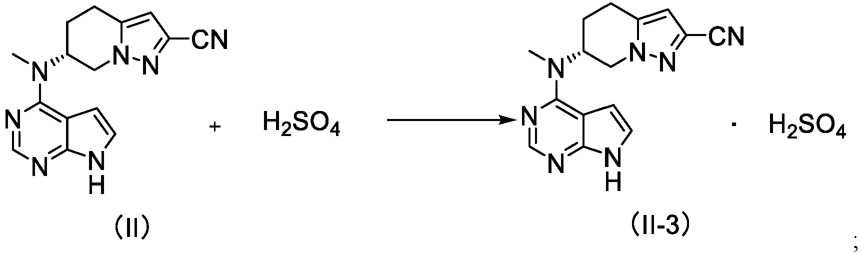
or, when the salt of the compound represented by formula (II) is the sulfate represented by formula (II-3) and the sulfate represented by formula (II-3) is the crystal form D of the sulfate, the method for preparing the crystal form D of the sulfate comprises the following steps: adding sulfuric acid to a solution of the compound represented by formula (II) in ethanol to carry out a reaction shown in the following formula, and collecting a solid to obtain the crystal form D; 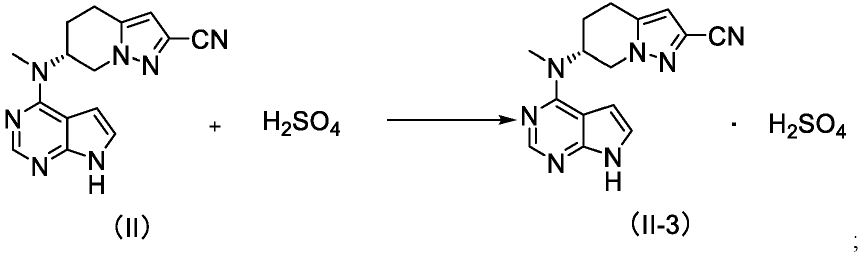
or, when the hydrate of the salt of the compound represented by formula (II) is the hydrate of the sulfate represented by formula (II-3), and the hydrate of the sulfate represented by formula (II-3) is the crystal form B of the hydrate of the sulfate, the method for preparing the crystal form B of the hydrate of the sulfate comprises the following steps: adding sulfuric acid into a solution of the compound represented by formula (II) in acetone to carry out a reaction shown in the following formula, and collecting a solid to obtain the crystal form B;

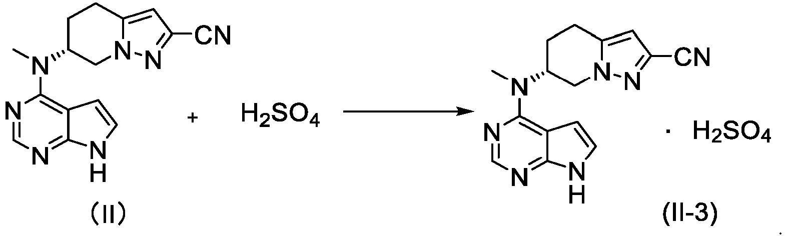

8. The method for preparing the crystal form, a salt, a crystal form of the salt, a hydrate of the salt, or a crystal form of the hydrate of the salt of the compound represented by formula (II) as claimed in claim 7, wherein,
when the compound represented by formula (II) is the crystal form A, the ester solvent is ethyl acetate or *n*-butyl acetate, such as ethyl acetate; or the alkane solvent is n-hexane, cyclohexane or *n*-heptane, such as cyclohexane;
or, when the compound represented by formula (II) is the crystal form A, the tetrahydrofuran and the antisolvent have a volume ratio of 1:(0.1-10); preferably 1:(0.5-3), more preferably 1:1 or 1.25:1;
or, when the compound represented by formula (II) is the crystal form A, the compound represented by formula (II) and the tetrahydrofuran have a mass-volume ratio of 2-6 mg/mL; preferably 5:1 mg/mL;
or, when the compound represented by formula (II) is the crystal form A, the compound represented by formula (II) and the antisolvent have a mass-volume ratio of 2-6 mg/mL; preferably 5:1 mg/mL or 4:1 mg/mL;
or, when the compound represented by formula (II) is the crystal form B, the crystal form A of the compound represented by formula (II-1) and the NMP have a mass-volume ratio of 1-5 mg/mL; preferably 2:1 mg/mL;
or, when the compound represented by formula (II) is the crystal form B, the NMP and the water have a volume ratio of 1:(0.1-10); preferably 1:(0.2-2), more preferably 1:0.4;
or, when the salt of the compound represented by formula (II) is the maleate represented by formula (II-1) and the maleate represented by formula (II-1) is the crystal form A of the maleate, the maleic acid and the compound represented by formula (II) have a molar ratio of 1.04:1;
or, when the salt of the compound represented by formula (II) is the maleate represented by formula (II-1) and the maleate represented by formula (II-1) is the crystal form A of the maleate, the compound represented by formula (II) and the ester solvent have a mass-volume ratio of 10 mg/mL;
or, when the salt of the compound represented by formula (II) is the maleate represented by formula (II-1) and the maleate represented by formula (II-1) is the crystal form A of the maleate, the ester solvent is ethyl acetate;
or, when the salt of the compound represented by formula (II) is the hydrochloride represented by formula (II-2) and the hydrochloride represented by formula (II-2) is the crystal form A of the hydrochloride, the hydrochloric acid and the compound represented by formula (II) have a molar ratio of 1.05:1;
or, when the salt of the compound represented by formula (II) is the hydrochloride represented by formula (II-2) and the hydrochloride represented by formula (II-2) is the crystal form A of the hydrochloride, the compound represented by formula (II) and the acetonitrile aqueous solution have a mass-volume ratio of 40 mg/mL;
or, when the salt of the compound represented by formula (II) is the hydrochloride represented by formula (II-2) and the hydrochloride represented by formula (II-2) is the crystal form A of the hydrochloride, the acetonitrile aqueous solution has a volume ratio of the acetonitrile and the H₂O in the acetonitrile aqueous solution have a volume ratio of 19:1;
or, when the salt of the compound represented by formula (II) is the hydrochloride represented by formula (II-2) and the hydrochloride represented by formula (II-2) is the crystal form B of the hydrochloride, the hydrochloric acid and the compound represented by formula (II) have a molar ratio of 1.05:1;
or, when the salt of the compound represented by formula (II) is the hydrochloride represented by formula (II-2) and the hydrochloride represented by formula (II-2) is the crystal form B of the hydrochloride, the compound represented by formula (II) and the ethyl acetate have a mass-volume ratio of 40 mg/mL;
or, when the salt of the compound represented by formula (II) is the hydrochloride represented by formula (II-2) and the hydrochloride represented by formula (II-2) is the crystal form C of the hydrochloride, the hydrochloric acid and the compound represented by formula (II) have a molar ratio of 1.05:1;
or, when the salt of the compound represented by formula (II) is the hydrochloride represented by formula (II-2) and the hydrochloride represented by formula (II-2) is the crystal form C of the hydrochloride, the compound represented by formula (II) and the ethanol have a mass-volume ratio of 40 mg/mL;
or, when the salt of the compound represented by formula (II) is the hydrochloride represented by formula (II-2) and the hydrochloride represented by formula (II-2) is the crystal form D of the hydrochloride, the hydrochloric acid and the compound represented by formula (II) have a molar ratio of 1.05:1;
or, when the salt of the compound represented by formula (II) is the hydrochloride represented by formula (II-2) and the hydrochloride represented by formula (II-2) is the crystal form D of the hydrochloride, the compound represented by formula (II) and the tetrahydrofuran have a mass-volume ratio of 40 mg/mL;
or, when the salt of the compound represented by formula (II) is the sulfate represented by formula (II-3) and the sulfate represented by formula (II-3) is the crystal form A of the sulfate, the sulfuric acid and the compound represented by formula (II) have a molar ratio of 1.03:1;
or, when the salt of the compound represented by formula (II) is the sulfate represented by formula (II-3) and the sulfate represented by formula (II-3) is the crystal form A of the sulfate, the compound represented by formula (II) and the acetonitrile aqueous solution have a mass-volume ratio of 30 mg/mL;
or, when the salt of the compound represented by formula (II) is the sulfate represented by formula (II-3) and the sulfate represented by formula (II-3) is the crystal form A of the sulfate, the acetonitrile and the H₂O in the acetonitrile aqueous solution have a volume ratio of 19:1;
or, when the salt of the compound represented by formula (II) is the sulfate represented by formula (II-3) and the sulfate represented by formula (II-3) is the crystal form A of the sulfate, the sulfuric acid has a molar concentration of 4 mol/L;
or, when the salt of the compound represented by formula (II) is the sulfate represented by formula (II-3) and the crystal salt of the sulfate represented by formula (II-3) is the crystal form C of the sulfate, the sulfuric acid and the compound represented by formula (II) have a molar ratio of 1.03:1;
or, when the salt of the compound represented by formula (II) is the sulfate represented by formula (II-3) and the crystal salt of the sulfate represented by formula (II-3) is the crystal form C of the sulfate, the compound represented by formula (II) and the ethyl acetate have a mass-volume ratio of 30 mg/mL;
or, when the salt of the compound represented by formula (II) is the sulfate represented by formula (II-3) and the crystal salt of the sulfate represented by formula (II-3) is the crystal form C of the sulfate, the sulfuric acid has a molar concentration of 4 mol/L;
or, when the salt of the compound represented by formula (II) is the sulfate represented by formula (II-3) and the sulfate represented by formula (II-3) is the crystal form D of the sulfate, the sulfuric acid and the compound represented by formula (II) have a molar ratio of 1.03:1;
or, when the salt of the compound represented by formula (II) is the sulfate represented by formula (II-3) and the sulfate represented by formula (II-3) is the crystal form D of the sulfate, the compound represented by formula (II) and the ethanol have a mass-volume ratio of 30 mg/mL;
or, when the salt of the compound represented by formula (II) is the sulfate represented by formula (II-3) and the sulfate represented by formula (II-3) is the crystal form D of the sulfate, the sulfuric acid has a molar concentration of 4 mol/L;
or, when the hydrate of the salt of the compound represented by formula (II) is the hydrate of the sulfate represented by formula (II-3), and the hydrate of the sulfate represented by formula (II-3) is the crystal form B of the hydrate of the sulfate, the sulfuric acid and the compound represented by formula (II) have a molar ratio of 1.03:1;
or, when the hydrate of the salt of the compound represented by formula (II) is the hydrate of the sulfate represented by formula (II-3), and the hydrate of the sulfate represented by formula (II-3) is the crystal form B of the hydrate of the sulfate, the compound represented by formula (II) and the acetone have a mass-volume ratio of 30 mg/mL;
or, when the hydrate of the salt of the compound represented by formula (II) is the hydrate of the sulfate represented by formula (II-3), and the hydrate of the sulfate represented by formula (II-3) is the crystal form B of the hydrate of the sulfate, the sulfuric acid has a molar concentration of 4 mol/L.

9. The crystal form, the salt, the crystal form of the salt, the hydrate of the salt, or the crystal form of the hydrate of the salt of the compound represented by formula (II) as claimed in any one of claims 1-5 for use in the treatment of rheumatoid arthritis.

## Patentansprüche

1. Kristallform, Salz, Kristallform des Salzes, Hydrat des Salzes oder Kristallform des Hydrats des Salzes einer Verbindung dargestellt durch Formel (II), 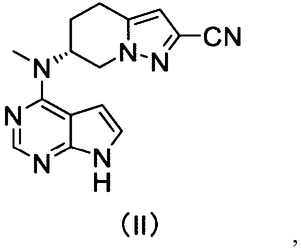 und wobei Säure in dem Salz aus Maleinsäure, Salzsäure und Schwefelsäure ausgewählt ist.

2. Kristallform, Salz, Kristallform des Salzes, Hydrat des Salzes oder Kristallform des Hydrats des Salzes der Verbindung dargestellt durch Formel (II) nach Anspruch 1, wobei die Säure in dem Salz und die Verbindung dargestellt durch Formel (II) ein Molverhältnis von (0,25-1,5):1 aufweisen.

3. Kristallform, Salz, Kristallform des Salzes, Hydrat des Salzes oder Kristallform des Hydrats des Salzes der Verbindung dargestellt durch Formel (II) nach Anspruch 2, wobei das Salz der Verbindung dargestellt durch Formel (II) ausgewählt ist aus einer beliebigen der folgenden Verbindungen:
einem Sulfat dargestellt durch Formel (II-3) 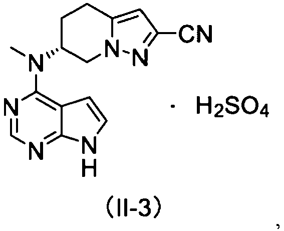
einem Hydrochlorid dargestellt durch Formel (II-2) 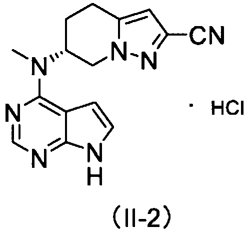
und einem Maleat dargestellt durch Formel (II-1) 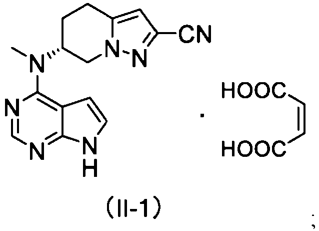 oder
das Hydrat des Salzes der Verbindung dargestellt durch Formel (II) ausgewählt ist aus einem Hydrat eines Sulfats dargestellt durch Formel (II-3)

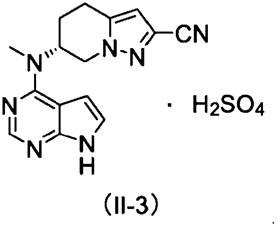

4. Kristallform, Salz, Kristallform des Salzes, Hydrat des Salzes oder Kristallform des Hydrats des Salzes der Verbindung dargestellt durch Formel (II) nach Anspruch 3, wobei
die Kristallform der Verbindung dargestellt durch Formel (II) eine Kristallform A ist, die ein Röntgenpulverbeugungsmuster ausgedrückt durch 2θ-Winkel mit Beugungsspitzen bei 12,69°, 13,84°, 15,37°, 15,90°, 16,62°, 19,07°, 27,66° und 25,62° aufweist;
oder die Kristallform der Verbindung dargestellt durch Formel (II) eine Kristallform B ist, die ein Röntgenpulverbeugungsmuster ausgedrückt durch 20-Winkel mit Beugungsspitzen bei 12,40°, 13,31°, 15,75°, 22,16°, 23,72°, 25,49°, 26,12° und 26,87° aufweist;
oder das Maleat dargestellt durch Formel (II-1) eine Kristallform A des Maleats ist, die ein Röntgenpulverbeugungsmuster ausgedrückt durch 2θ-Winkel mit Beugungsspitzen bei 12,24°, 13,14°, 13,73°, 14,56°, 15,52°, 17,54°, 19,54°, 23,19°, 26,55° und 26,91° aufweist;
oder das Hydrochlorid dargestellt durch Formel (II-2) eine Kristallform A des Hydrochlorids ist, die ein Röntgenpulverbeugungsmuster ausgedrückt durch 2θ-Winkel mit Beugungsspitzen bei 7,20°, 7,85°, 8,63°, 10,82°, 21,25°, 21,78°, 24,12°, 25,56°, 26,11° und 27,03° aufweist;
oder das Hydrochlorid dargestellt durch Formel (II-2) eine Kristallform B des Hydrochlorids ist, die ein Röntgenpulverbeugungsmuster ausgedrückt durch 20-Winkel mit Beugungsspitzen bei 5,95°, 11,92°, 12,53°, 13,14°, 20,94°, 24,96°, 25,67°, 30,09° und 31,69° aufweist;
oder das Hydrochlorid dargestellt durch Formel (II-2) eine Kristallform C des Hydrochlorids ist, die ein Röntgenpulverbeugungsmuster ausgedrückt durch 20-Winkel mit Beugungsspitzen bei 5,89°, 11,77°, 13,21°, 13,52°, 15,75°, 23,51°, 25,51°, 24,65°, 26,31° und 27,15° aufweist;
oder das Hydrochlorid dargestellt durch Formel (II-2) eine Kristallform D des Hydrochlorids ist, die ein Röntgenpulverbeugungsmuster ausgedrückt durch 2θ-Winkel mit Beugungsspitzen bei 3,08°, 6,07°, 9,05°, 12,06°, 12,73°, 13,23°, 13,78° 15,08°, 21,28°, 24,94°, 26,06° und 31,72° aufweist;
oder das Sulfat dargestellt durch Formel (II-3) eine Kristallform A des Sulfats ist, die ein Röntgenpulverbeugungsmuster ausgedrückt durch 2θ-Winkel mit Beugungsspitzen bei 4,16°, 4,46°, 7,56°, 8,02°, 12,65°, 13,36°, 15,75°, 17,91°, 20,43°, 24,54°, 24,94°, 25,90° und 26,99° aufweist;
oder das Sulfat dargestellt durch Formel (II-3) eine Kristallform C des Sulfats ist, die ein Röntgenpulverbeugungsmuster ausgedrückt durch 2θ-Winkel mit Beugungsspitzen bei 6,09°, 12,17°, 13,25°, 15,60°, 16,09°, 17,45°, 18,66°, 22,61°, 23,62°, 24,44°, 25,04°, 25,89°, 26,30° und 26,62° aufweist;
oder das Sulfat dargestellt durch Formel (II-3) eine Kristallform D des Sulfats ist, die ein Röntgenpulverbeugungsmuster ausgedrückt durch 2θ-Winkel mit Beugungsspitzen bei 4,61°, 6,11°, 8,00°, 9,10°, 10,91°, 13,73°, 18,86°, 23,27°, 25,28° und 25,97° aufweist;
oder das Hydrat des Sulfats dargestellt durch Formel (II-3) eine Kristallform B des Hydrats des Sulfats ist, die ein Röntgenpulverbeugungsmuster ausgedrückt durch 2θ-Winkel mit Beugungsspitzen bei 5,18°, 12,03°, 12,92°, 15,54°, 16,09°, 17,74°, 19,06°, 20,71°, 23,99°, 24,82° und 25,79° aufweist.

5. Kristallform, Salz, Kristallform des Salzes, Hydrat des Salzes oder Kristallform des Hydrats des Salzes der Verbindung dargestellt durch Formel (II) nach Anspruch 4, wobei, wenn die Kristallform der Verbindung dargestellt durch Formel (II) die Kristallform A ist, in dem Röntgenpulverbeugungsmuster der Kristallform A ausgedrückt durch 2θ Winkel 20-Werte in der folgenden Tabelle gezeigt sind:
| 20-Winkel (°) | Relative Intensität (%) |
|---|---|
| 6,33 | 9,91 |
| 12,69 | 97,83 |
| 13,84 | 69,04 |
| 14,91 | 27,95 |
| 15,37 | 76,70 |
| 15,90 | 45,28 |
| 16,62 | 66,33 |
| 19,07 | 40,00 |
| 22,15 | 17,81 |
| 24,96 | 24,95 |
| 25,62 | 100,00 |
| 26,95 | 30,65 |
| 27,66 | 29,86 |
| 30,19 | 5,78 |
| 37,92 | 3,13 |
| 38,88 | 7,58 |
oder die Kristallform A der Verbindung dargestellt durch Formel (II) eine Differential-Scanning-Kalorimetrie(DSC-)Kurve mit einer Absorptionsspitze mit einer Spitzentemperatur von 300,4 °C aufweist; oder die Kristallform A der Verbindung dargestellt durch Formel (II) eine thermogravimetrische Analysekurve mit einem Gewichtsverlust von 2,1 % in einem Temperaturbereich von 25,1 °C bis 250 °C aufweist;
oder, wenn die Kristallform der Verbindung dargestellt durch Formel (II) die Kristallform B ist, in dem Röntgenpulverbeugungsmuster der Kristallform B ausgedrückt durch 20-Winkel 20-Werte in der folgenden Tabelle gezeigt sind:
| 20-Winkel (°) | Relative Intensität (%) |
|---|---|
| 12,40 | 43,82 |
| 13,31 | 79,40 |
| 15,75 | 76,57 |
| 19,12 | 15,04 |
| 22,16 | 100,00 |
| 23,72 | 62,88 |
| 25,49 | 20,80 |
| 26,12 | 42,21 |
| 26,87 | 26,28 |
| 33,42 | 2,44 |
oder, wenn das Maleat dargestellt durch Formel (II-1) die Kristallform A des Maleats ist, in dem Röntgenpulverbeugungsmuster der Kristallform A des Maleats ausgedrückt durch 2θ-Winkel 20-Werte in der folgenden Tabelle gezeigt sind:
| 20-Winkel (°) | Relative Intensität (%) |
|---|---|
| 4,40 | 14,26 |
| 8,76 | 9,45 |
| 12,24 | 21,69 |
| 13,14 | 24,97 |
| 13,73 | 100,00 |
| 14,56 | 19,33 |
| 15,52 | 16,05 |
| 16,42 | 2,53 |
| 17,54 | 35,08 |
| 18,89 | 2,59 |
| 19,54 | 32,55 |
| 20,37 | 11,04 |
| 23,19 | 18,59 |
| 23,70 | 5,19 |
| 24,59 | 5,61 |
| 25,52 | 7,75 |
| 26,55 | 15,18 |
| 26,91 | 15,77 |
| 27,11 | 9,87 |
| 27,98 | 3,54 |
| 29,30 | 6,68 |
| 30,01 | 3,87 |
| 36,24 | 1,53 |
| 37,44 | 2,47 |
oder die Kristallform A des Maleats dargestellt durch Formel (II-1) eine Differential-Scanning-Kalorimetrie-Kurve mit einer Absorptionsspitze mit einer Spitzentemperatur von 173,3 °C aufweist; oder die Kristallform A des Maleats dargestellt durch Formel (II-1) eine thermogravimetrische Analysekurve mit einem Gewichtsverlust von 1,86 % in einem Temperaturbereich von 29,1 °C bis 150 °C aufweist;
oder, wenn das Hydrochlorid dargestellt durch Formel (II-2) die Kristallform A des Hydrochlorids ist, in dem Röntgenpulverbeugungsmuster der Kristallform A des Hydrochlorids dargestellt durch Formel (II-2) ausgedrückt durch 20-Winkel 20-Werte in der folgenden Tabelle gezeigt sind:
| 2θ-Winkel (°) | Relative Intensität (%) |
|---|---|
| 6,17 | 8,01 |
| 7,20 | 38,14 |
| 7,85 | 100,00 |
| 8,63 | 30,15 |
| 10,82 | 51,15 |
| 12,55 | 6,78 |
| 13,75 | 6,92 |
| 15,95 | 9,57 |
| 19,08 | 5,50 |
| 20,39 | 7,33 |
| 21,25 | 35,03 |
| 21,78 | 33,53 |
| 24,12 | 26,63 |
| 25,56 | 38,38 |
| 26,11 | 37,55 |
| 27,03 | 28,20 |
| 31,72 | 11,22 |
oder die Kristallform A des Hydrochlorids dargestellt durch Formel (II-2) eine Differential-Scanning-Kalorimetrie-Kurve mit drei Absorptionsspitzen mit Spitzentemperaturen von jeweils 78,1 °C, 92,2 °C und 274 °C aufweist; oder die Kristallform A des Hydrochlorids dargestellt durch Formel (II-2) eine thermogravimetrische Analysekurve mit einem Gewichtsverlust von 6,71 % in einem Temperaturbereich von 27,2 °C bis 100 °C aufweist;
oder, wenn das Hydrochlorid dargestellt durch Formel (II-2) die Kristallform B des Hydrochlorids ist, in dem Röntgenpulverbeugungsmuster der Kristallform B des Hydrochlorids ausgedrückt durch 2Θ-Winkel 20-Werte in der folgenden Tabelle gezeigt sind:
| 2θ-Winkel (°) | Relative Intensität (%) |
|---|---|
| 5,95 | 100,00 |
| 11,05 | 13,61 |
| 11,92 | 61,44 |
| 12,53 | 31,64 |
| 13,14 | 48,62 |
| 16,67 | 8,26 |
| 19,31 | 6,81 |
| 20,94 | 90,42 |
| 22,04 | 5,05 |
| 23,19 | 13,88 |
| 24,96 | 43,42 |
| 25,67 | 49,06 |
| 30,09 | 18,58 |
| 31,69 | 36,23 |
| 39,46 | 14,32 |
oder die Kristallform B des Hydrochlorids dargestellt durch Formel (II-2) eine Differential-Scanning-Kalorimetrie-Kurve mit einer Absorptionsspitze mit einer Spitzentemperatur von 274,7 °C aufweist; oder die Kristallform B des Hydrochlorids dargestellt durch Formel (II-2) eine thermogravimetrische Analysekurve mit einem Gewichtsverlust von 2,34 % in einem Temperaturbereich von 31,4 °C bis 100 °C aufweist;
oder, wenn das Hydrochlorid dargestellt durch Formel (II-2) die Kristallform C des Hydrochlorids ist, in dem Röntgenpulverbeugungsmuster der Kristallform C des Hydrochlorids dargestellt durch Formel (II-2) ausgedrückt durch 2θ-Winkel 20-Werte in der folgenden Tabelle gezeigt sind:
| 2θ-Winkel (°) | Relative Intensität (%) |
|---|---|
| 5,89 | 60,87 |
| 11,77 | 100,00 |
| 12,58 | 22,24 |
| 13,21 | 71,07 |
| 15,75 | 59,51 |
| 17,71 | 13,70 |
| 18,41 | 17,93 |
| 23,51 | 90,26 |
| 24,65 | 18,91 |
| 25,51 | 25,11 |
| 26,31 | 55,95 |
| 27,15 | 43,81 |
oder die Kristallform C des Hydrochlorids dargestellt durch Formel (II-2) eine Differential-Scanning-Kalorimetrie-Kurve mit einer Absorptionsspitze mit einer Spitzentemperatur von 275,1 °C aufweist; oder die Kristallform C des Hydrochlorids dargestellt durch Formel (II-2) eine thermogravimetrische Analysekurve mit einem Gewichtsverlust von 1,32 % in einem Temperaturbereich von 33,7 °C bis 100 °C aufweist;
oder, wenn das Hydrochlorid dargestellt durch Formel (II-2) die Kristallform D des Hydrochlorids ist, in dem Röntgenpulverbeugungsmuster der Kristallform D des Hydrochlorids ausgedrückt durch 2Θ-Winkel 20-Werte in der folgenden Tabelle gezeigt sind:
| 2θ-Winkel (°) | Relative Intensität (%) |
|---|---|
| 3,08 | 79,15 |
| 6,07 | 100,00 |
| 9,05 | 30,52 |
| 12,06 | 29,69 |
| 12,73 | 34,25 |
| 13,23 | 25,66 |
| 13,78 | 25,88 |
| 14,53 | 17,45 |
| 15,08 | 26,72 |
| 16,32 | 14,81 |
| 17,45 | 11,57 |
| 18,20 | 10,95 |
| 19,31 | 6,12 |
| 21,28 | 79,37 |
| 22,02 | 15,50 |
| 23,36 | 10,90 |
| 24,94 | 28,49 |
| 26,06 | 36,10 |
| 29,23 | 5,77 |
| 30,51 | 11,55 |
| 31,72 | 40,73 |
| 36,83 | 5,80 |
oder die Kristallform D des Hydrochlorids dargestellt durch Formel (II-2) eine Differential-Scanning-Kalorimetrie-Kurve mit zwei Absorptionsspitzen mit Spitzentemperaturen von jeweils 273,7 °C und 279,3 °C aufweist; oder die Kristallform D des Hydrochlorids dargestellt durch Formel (II-2) eine thermogravimetrische Analysekurve mit einem Gewichtsverlust von 2,92 % in einem Temperaturbereich von 28,3 °C bis 100 °C aufweist;
oder, wenn das Sulfat dargestellt durch Formel (II-3) die Kristallform A des Sulfats ist, in dem Röntgenpulverbeugungsmuster der Kristallform A des Sulfats ausgedrückt durch 2θ-Winkel 20-Werte in der folgenden Tabelle gezeigt sind:
| 2θ-Winkel (°) | Relative Intensität (%) |
|---|---|
| 4,16 | 81,60 |
| 4,46 | 71,99 |
| 7,56 | 100,00 |
| 8,02 | 63,19 |
| 9,67 | 3,76 |
| 12,65 | 30,66 |
| 13,36 | 77,29 |
| 14,36 | 14,12 |
| 15,75 | 40,01 |
| 17,91 | 38,83 |
| 19,02 | 21,35 |
| 20,43 | 34,62 |
| 22,34 | 11,00 |
| 24,54 | 61,38 |
| 24,94 | 65,92 |
| 25,90 | 49,58 |
| 26,99 | 43,14 |
| 27,87 | 11,82 |
oder die Kristallform A des Sulfats dargestellt durch Formel (II-3) eine Differential-Scanning-Kalorimetrie-Kurve mit zwei Absorptionsspitzen mit Spitzentemperaturen von jeweils 85,1 °C und 126,7 °C aufweist; oder die Kristallform A des Sulfats dargestellt durch Formel (II-3) eine thermogravimetrische Analysekurve mit einem Gewichtsverlust von 8,14 % in einem Temperaturbereich von 28,4 °C bis 150 °C aufweist;
oder, wenn das Kristallsalz des Sulfats dargestellt durch Formel (II-3) die Kristallform C des Sulfats ist, in dem Röntgenpulverbeugungsmuster der Kristallform C des Sulfats ausgedrückt durch 2Θ-Winkel 20-Werte in der folgenden Tabelle gezeigt sind:
| 2θ-Winkel (°) | Relative Intensität (%) |
|---|---|
| 6,09 | 99,99 |
| 9,87 | 8,42 |
| 10,84 | 8,31 |
| 12,17 | 91,39 |
| 13,25 | 28,45 |
| 15,60 | 28,16 |
| 16,09 | 43,58 |
| 17,45 | 31,44 |
| 18,66 | 100,00 |
| 19,80 | 25,64 |
| 20,88 | 22,81 |
| 22,61 | 32,22 |
| 23,62 | 35,33 |
| 24,44 | 30,65 |
| 25,04 | 31,87 |
| 25,89 | 63,39 |
| 26,30 | 43,49 |
| 26,62 | 62,05 |
| 27,43 | 21,62 |
| 27,76 | 15,17 |
oder die Kristallform C des Sulfats dargestellt durch Formel (II-3) eine Differential-Scanning-Kalorimetrie-Kurve mit zwei Absorptionsspitzen mit Spitzentemperaturen von jeweils 69,3 °C und 118,1 °C aufweist; oder die Kristallform C des Sulfats dargestellt durch Formel (II-3) eine thermogravimetrische Analyse-Kurve mit einem Gewichtsverlust von 6,82 % in einem Temperaturbereich von 28,2 °C bis 150 °C aufweist;
oder, wenn das Sulfat dargestellt durch Formel (II-3) die Kristallform D des Sulfats ist, in dem Röntgenpulverbeugungsmuster der Kristallform D des Sulfats ausgedrückt durch 2θ-Winkel 20-Werte in der folgenden Tabelle gezeigt sind:
| 2θ-Winkel (°) | Relative Intensität (%) |
|---|---|
| 4,61 | 68,84 |
| 6,11 | 29,48 |
| 8,00 | 45,48 |
| 9,10 | 64,58 |
| 10,05 | 18,88 |
| 10,91 | 46,90 |
| 12,72 | 24,73 |
| 13,73 | 54,06 |
| 16,18 | 23,96 |
| 18,86 | 100,00 |
| 20,53 | 14,88 |
| 23,27 | 39,33 |
| 25,28 | 30,71 |
| 25,97 | 55,48 |
| 31,91 | 13,58 |
| 34,12 | 5,79 |
| 38,61 | 4,36 |
oder die Kristallform D des Sulfats dargestellt durch Formel (II-3) eine Differential-Scanning-Kalorimetrie-Kurve mit zwei Absorptionsspitzen mit Spitzentemperaturen von jeweils 78,5 °C und 144,3 °C aufweist; oder die Kristallform D des Sulfats dargestellt durch Formel (II-3) eine thermogravimetrische Analysekurve mit einem Gewichtsverlust von 8,13 % in einem Temperaturbereich von 23,3 °C bis 150 °C aufweist;
oder, wenn das Hydrat des Salzes der Verbindung dargestellt durch Formel (II) das Hydrat des Sulfats dargestellt durch Formel (II-3) ist, Wasser in der Kristallform B des Hydrats des Sulfats dargestellt durch Formel (II-3) und des Sulfats dargestellt durch Formel (II-3) ein Zahlenverhältnis von 1,5 aufweisen kann; oder in dem Röntgenpulverbeugungsmuster der Kristallform B des Hydrats des Sulfats dargestellt durch Formel (II-3) ausgedrückt durch 2Θ-Winkel 20-Werte in der folgenden Tabelle gezeigt sind:
| 20-Winkel | Relative Intensität |
|---|---|
| 5,18 | 45,44 |
| 7,95 | 11,00 |
| 8,82 | 19,56 |
| 10,23 | 16,98 |
| 12,03 | 24,82 |
| 12,92 | 73,75 |
| 15,54 | 47,91 |
| 16,09 | 90,49 |
| 17,74 | 41,11 |
| 19,06 | 38,98 |
| 20,71 | 33,34 |
| 23,99 | 100,00 |
| 24,82 | 22,22 |
| 25,79 | 56,92 |
oder die Kristallform B des Hydrats des Sulfats dargestellt durch Formel (II-3) eine Differential-Scanning-Kalorimetrie-Kurve mit zwei Absorptionsspitzen mit Spitzentemperaturen von jeweils 98 °C und 140,2 °C aufweist; oder die Kristallform B des Hydrats des Sulfats dargestellt durch Formel (II-3) eine thermogravimetrische Analysekurve mit einem Gewichtsverlust von 6,67 % in einem Temperaturbereich von 28,6 °C bis 150 °C aufweist.

6. Verfahren zur Herstellung der Kristallform, eines Salzes, einer Kristallform des Salzes, eines Hydrats des Salzes oder einer Kristallform des Hydrats des Salzes der Verbindung dargestellt durch Formel (II) nach einem der Ansprüche 1 bis 5, wobei das Verfahren zur Herstellung des Salzes der Verbindung dargestellt durch Formel (II) die folgenden Schritte umfasst: Durchführen einer Salzbildungsreaktion der Verbindung dargestellt durch Formel (II) nach einem der Ansprüche 1 bis 3 und der Säure nach Anspruch 1 in einem Lösungsmittel; das Lösungsmittel bevorzugt ausgewählt ist aus einem oder mehreren von Tetrahydrofuran, 2-Methylfuran, Ethylacetat, Cyclohexan, Dimethylsulfoxid, Wasser, n-Butanol, Isopropanol, Ethanol, N-Methylpyrrolidon, Acetonitril, Aceton, Butanon, Methylisobutylketon, Toluol, Dichlormethan, 1,4-Dioxan und Anisol; das Lösungsmittel bevorzugt ausgewählt ist aus einem oder mehreren von Tetrahydrofuran, Ethylacetat, Cyclohexan, Dimethylsulfoxid, Ethanol, Wasser, n-Butanol, N-Methylpyrrolidon, Acetonitril und Butanon; das Lösungsmittel bevorzugter ausgewählt ist aus Tetrahydrofuran, Aceton, Ethanol, Ethylacetat und wässriger Acetonitrillösung.

7. Verfahren zur Herstellung der Kristallform, eines Salzes, einer Kristallform des Salzes, eines Hydrats des Salzes oder einer Kristallform des Hydrats des Salzes der Verbindung dargestellt durch Formel (II) nach Anspruch 6, wobei,
wenn die Verbindung dargestellt durch Formel (II) die Kristallform A ist, das Verfahren zur Herstellung der Kristallform A der Verbindung dargestellt durch Formel (II) die folgenden Schritte umfasst: Hinzufügen eines Antilösungsmittels in eine Lösung der Verbindung dargestellt durch Formel (II) in Tetrahydrofuran bei Raumtemperatur und Erhalten der Kristallform A nach Kristallisation; wobei das Antilösungsmittel ein Esterlösungsmittel oder ein Alkanlösungsmittel ist;
oder, wenn die Verbindung dargestellt durch Formel (II) die Kristallform B ist, das Verfahren zur Herstellung der Kristallform B der Verbindung dargestellt durch Formel (II) die folgenden Schritte umfasst: Auflösen der Kristallform A der Verbindung dargestellt durch Formel (II-1) in NMP bei Raumtemperatur, Hinzufügen von Wasser und Erhalten der Kristallform B nach Kristallisation;
oder, wenn das Salz der Verbindung dargestellt durch Formel (II) das Maleat dargestellt durch Formel (II-1) ist und das Maleat dargestellt durch Formel (II-1) die Kristallform A des Maleats ist, das Verfahren zur Herstellung der Kristallform A des Maleats die folgenden Schritte umfasst: Dispergieren der Verbindung dargestellt durch Formel (II) und Maleinsäure in einem Esterlösungsmittel, um eine Reaktion durchzuführen, die in der folgenden Formel gezeigt ist, und Sammeln eines Feststoffes, um die Kristallform A zu erhalten; 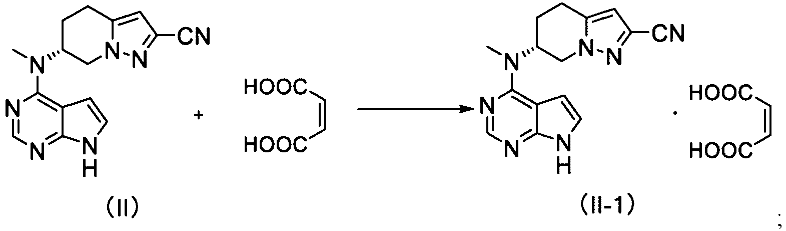
oder, wenn das Salz der Verbindung dargestellt durch Formel (II) das Hydrochlorid dargestellt durch Formel (II-2) ist und das Hydrochlorid dargestellt durch Formel (II-2) die Kristallform A des Hydrochlorids ist, das Verfahren zur Herstellung der Kristallform A des Hydrochlorids die folgenden Schritte umfasst: Hinzufügen von Salzsäure zu einer Lösung der Verbindung dargestellt durch Formel (II), um eine Reaktion durchzuführen, die in der folgenden Formel gezeigt ist, und Sammeln eines Feststoffes, um die Kristallform A zu erhalten; wobei ein Lösungsmittel der Lösung eine wässrige Acetonitrillösung ist; 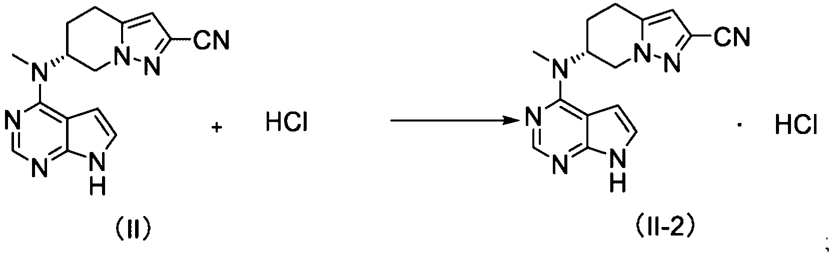
oder, wenn das Salz der Verbindung dargestellt durch Formel (II) das Hydrochlorid dargestellt durch Formel (II-2) ist und das Hydrochlorid dargestellt durch Formel (II-2) die Kristallform B des Hydrochlorids ist, das Verfahren zur Herstellung der Kristallform B des Hydrochlorids die folgenden Schritte umfasst: Hinzufügen von Salzsäure zu einer Lösung der Verbindung dargestellt durch Formel (II) in Ethylacetat, um eine Reaktion durchzuführen, die in der folgenden Formel gezeigt ist, und Sammeln eines Feststoffes, um die Kristallform B zu erhalten; 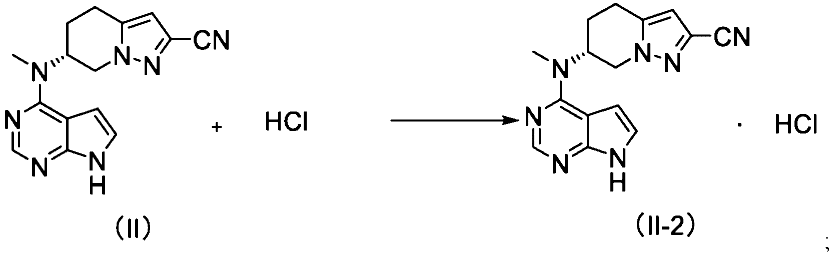
oder, wenn das Salz der Verbindung dargestellt durch Formel (II) das Hydrochlorid dargestellt durch Formel (II-2) ist und das Hydrochlorid dargestellt durch Formel (II-2) die Kristallform C des Hydrochlorids ist, das Verfahren zur Herstellung der Kristallform C des Hydrochlorids die folgenden Schritte umfasst: Hinzufügen von Salzsäure zu einer Lösung der Verbindung dargestellt durch Formel (II) in Ethanol, um eine Reaktion durchzuführen, die in der folgenden Formel gezeigt ist, und Sammeln eines Feststoffes, um die Kristallform C zu erhalten; 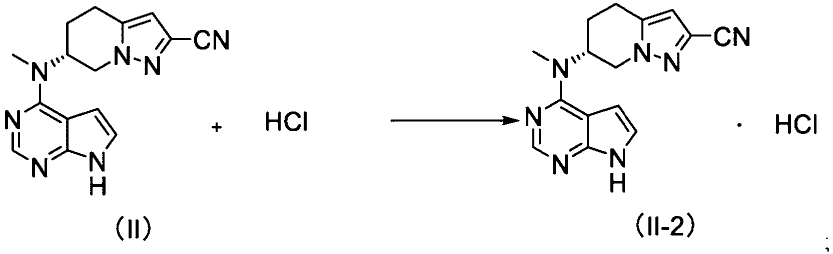
oder, wenn das Salz der Verbindung dargestellt durch Formel (II) das Hydrochlorid dargestellt durch Formel (II-2) ist und das Hydrochlorid dargestellt durch Formel (II-2) die Kristallform D des Hydrochlorids ist, das Verfahren zur Herstellung der Kristallform D des Hydrochlorids die folgenden Schritte umfasst: Hinzufügen von Salzsäure zu einer Lösung der Verbindung dargestellt durch Formel (II) in Tetrahydrofuran, um eine Reaktion durchzuführen, die in der folgenden Formel gezeigt ist, und Sammeln eines Feststoffes, um die Kristallform D zu erhalten; 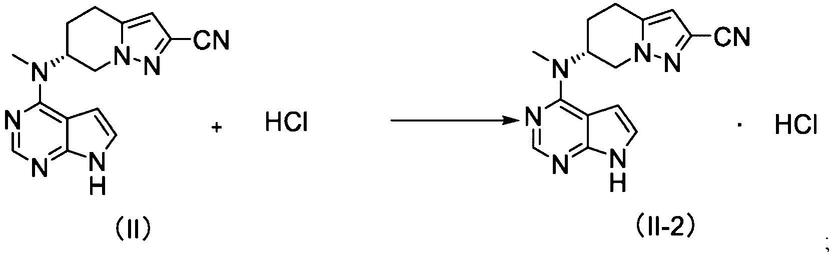
oder, wenn das Salz der Verbindung dargestellt durch Formel (II) das Sulfat dargestellt durch Formel (II-3) ist und das Sulfat dargestellt durch Formel (II-3) die Kristallform A des Sulfats ist, das Verfahren zur Herstellung der Kristallform A des Sulfats die folgenden Schritte umfasst: Hinzufügen von Schwefelsäure zu einer Lösung der Verbindung dargestellt durch Formel (II), um eine Reaktion durchzuführen, die in der folgenden Formel gezeigt ist, und Sammeln eines Feststoffes, um die Kristallform A zu erhalten; wobei ein Lösungsmittel der Lösung eine wässrige Acetonitrillösung ist; 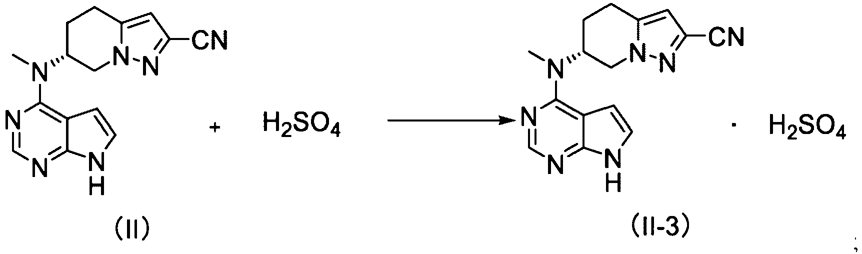
oder, wenn das Salz der Verbindung dargestellt durch Formel (II) das Sulfat dargestellt durch Formel (II-3) ist und das Sulfat dargestellt durch Formel (II-3) die Kristallform C des Sulfats ist, das Verfahren zur Herstellung der Kristallform C des Sulfats die folgenden Schritte umfasst: Hinzufügen von Schwefelsäure zu einer Lösung der Verbindung dargestellt durch Formel (II) in Ethylacetat, um eine Reaktion durchzuführen, die in der folgenden Formel gezeigt ist, und Sammeln eines Feststoffes, um die Kristallform C zu erhalten; 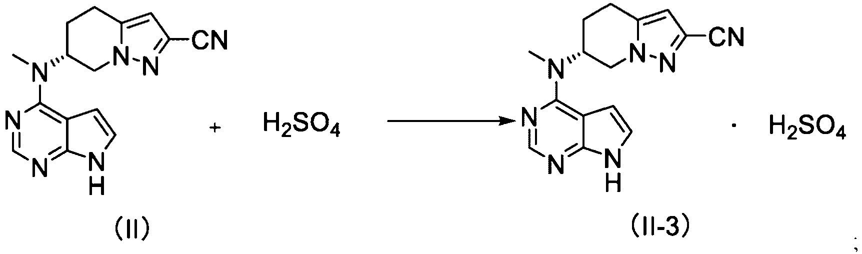
oder, wenn das Salz der Verbindung dargestellt durch Formel (II) das Sulfat dargestellt durch Formel (II-3) ist und das Sulfat dargestellt durch Formel (II-3) die Kristallform D des Sulfats ist, das Verfahren zur Herstellung der Kristallform D des Sulfats die folgenden Schritte umfasst: Hinzufügen von Schwefelsäure zu einer Lösung der Verbindung dargestellt durch Formel (II) in Ethanol, um eine Reaktion durchzuführen, die in der folgenden Formel gezeigt ist, und Sammeln eines Feststoffes, um die Kristallform D zu erhalten; 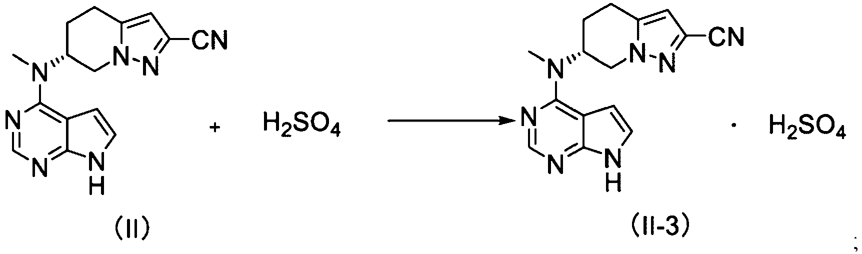
oder, wenn das Hydrat des Salzes der Verbindung dargestellt durch Formel (II) das Hydrat des Sulfats dargestellt durch Formel (II-3) ist und das Hydrat des Sulfats dargestellt durch Formel (II-3) die Kristallform B des Hydrats des Sulfats ist, das Verfahren zur Herstellung der Kristallform B des Hydrats des Sulfats die folgenden Schritte umfasst: Hinzufügen von Schwefelsäure in eine Lösung der Verbindung dargestellt durch Formel (II) in Aceton, um eine Reaktion durchzuführen, die in der folgenden Formel gezeigt ist, und Sammeln eines Feststoffes, um die Kristallform B zu erhalten;

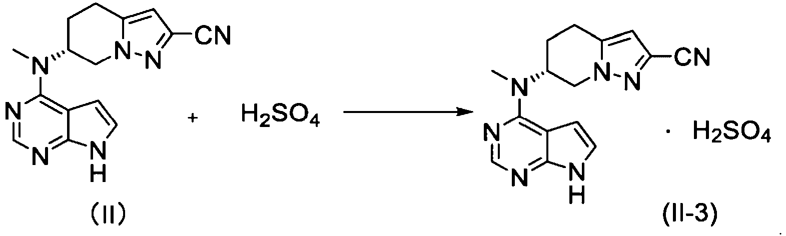

8. Verfahren zur Herstellung der Kristallform, eines Salzes, einer Kristallform des Salzes, eines Hydrats des Salzes oder einer Kristallform des Hydrats des Salzes der Verbindung dargestellt durch Formel (II) nach Anspruch 7, wobei,
wenn die Verbindung dargestellt durch Formel (II) die Kristallform A ist, das Esterlösungsmittel Ethylacetat oder n-Butylacetat ist, wie Ethylacetat; oder das Alkanlösungsmittel n-Hexan, Cyclohexan oder n-Heptan ist, wie Cyclohexan;
oder, wenn die Verbindung dargestellt durch Formel (II) die Kristallform A ist, das Tetrahydrofuran und das Antilösungsmittel ein Volumenverhältnis von 1:(0,1-10) aufweisen; bevorzugt 1:(0,5-3), bevorzugter 1:1 oder 1,25:1;
oder, wenn die Verbindung dargestellt durch Formel (II) die Kristallform A ist, die Verbindung dargestellt durch Formel (II) und das Tetrahydrofuran ein Massen-Volumen-Verhältnis von 2-6 mg/ml aufweisen; bevorzugt 5:1 mg/ml;
oder, wenn die Verbindung dargestellt durch Formel (II) die Kristallform A ist, die Verbindung dargestellt durch Formel (II) und das Antilösungsmittel ein Massen-Volumen-Verhältnis von 2-6 mg/ml aufweisen; bevorzugt 5:1 mg/ml oder 4:1 mg/ml;
oder, wenn die Verbindung dargestellt durch Formel (II) die Kristallform B ist, die Kristallform A der Verbindung dargestellt durch Formel (II-1) und das NMP ein Massen-Volumen-Verhältnis von 1-5 mg/ml aufweisen; bevorzugt 2:1 mg/ml;
oder, wenn die Verbindung dargestellt durch Formel (II) die Kristallform B ist, das NMP und das Wasser ein Volumenverhältnis von 1:(0,1-10) aufweisen; bevorzugt 1:(0,2-2), bevorzugter 1:0,4;
oder, wenn das Salz der Verbindung dargestellt durch Formel (II) das Maleat dargestellt durch Formel (II-1) ist und das Maleat dargestellt durch Formel (II-1) die Kristallform A des Maleats ist, die Maleinsäure und die Verbindung dargestellt durch Formel (II) ein Molverhältnis von 1,04:1 aufweisen;
oder, wenn das Salz der Verbindung dargestellt durch Formel (II) das Maleat dargestellt durch Formel (II-1) ist und das Maleat dargestellt durch Formel (II-1) die Kristallform A des Maleats ist, die Verbindung dargestellt durch Formel (II) und das Esterlösungsmittel ein Massen-Volumen-Verhältnis von 10 mg/ml aufweisen;
oder, wenn das Salz der Verbindung dargestellt durch Formel (II) das Maleat dargestellt durch Formel (II-1) ist und das Maleat dargestellt durch Formel (II-1) die Kristallform A des Maleats ist, das Esterlösungsmittel Ethylacetat ist;
oder, wenn das Salz der Verbindung dargestellt durch Formel (II) das Hydrochlorid dargestellt durch Formel (II-2) ist und das Hydrochlorid dargestellt durch Formel (II-2) die Kristallform A des Hydrochlorids ist, die Salzsäure und die Verbindung dargestellt durch Formel (II) ein Molverhältnis von 1,05:1 aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform A des Hydrochlorids ist, die Verbindung dargestellt durch Formel (II) und die wässrige Acetonitrillösung ein Massen-Volumen-Verhältnis von 40 mg/ml aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform A des Hydrochlorids ist, die wässrige Acetonitrillösung ein Volumenverhältnis des Acetonitrils und des H₂O aufweist in der wässrigen Acetonitrillösung ein Volumenverhältnis von 19:1 aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform B des Hydrochlorids ist, die Salzsäure und die Verbindung dargestellt durch Formel (II) ein Molverhältnis von 1,05:1 aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform B des Hydrochlorids ist, die Verbindung dargestellt durch Formel (II) und das Ethylacetat ein Massen-Volumen-Verhältnis von 40 mg/ml aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform C des Hydrochlorids ist, die Salzsäure und die Verbindung dargestellt durch Formel (II) ein Molverhältnis von 1,05:1 aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform C des Hydrochlorids ist, die Verbindung dargestellt durch Formel (II) und das Ethanol ein Massen-Volumen-Verhältnis von 40 mg/ml aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform D des Hydrochlorids ist, die Salzsäure und die Verbindung dargestellt durch Formel (II) ein Molverhältnis von 1,05:1 aufweisen;
oder, wenn das Salz der Verbindung dargestellt durch Formel (II) das Hydrochlorid dargestellt durch Formel (II-2) ist und das Hydrochlorid dargestellt durch Formel (II-2) die Kristallform D des Hydrochlorids ist, die Verbindung dargestellt durch Formel (II) und das Tetrahydrofuran ein Massen-Volumen-Verhältnis von 40 mg/ml aufweisen;
oder, wenn das Salz der Verbindung dargestellt durch Formel (II) das Sulfat dargestellt durch Formel (II-3) ist und das Sulfat dargestellt durch Formel (II-3) die Kristallform A des Sulfats ist, die Schwefelsäure und die Verbindung dargestellt durch Formel (II) ein Molverhältnis von 1,03:1 aufweisen;
oder, wenn das Salz der Verbindung dargestellt durch Formel (II) das Sulfat dargestellt durch Formel (II-3) ist und das Sulfat dargestellt durch Formel (II-3) die Kristallform A des Sulfats ist, die Verbindung dargestellt durch Formel (II) und die wässrige Acetonitrillösung ein Massen-Volumen-Verhältnis von 30 mg/ml aufweisen;
oder, wenn das Salz der Verbindung dargestellt durch Formel (II) das Sulfat dargestellt durch Formel (II-3) ist und das Sulfat dargestellt durch Formel (II-3) die Kristallform A des Sulfats ist, das Acetonitril und das H₂O in der wässrigen Acetonitrillösung ein Volumenverhältnis von 19:1 aufweisen;
oder, wenn das Salz der Verbindung dargestellt durch Formel (II) das Sulfat dargestellt durch Formel (II-3) ist und das Sulfat dargestellt durch Formel (II-3) die Kristallform A des Sulfats ist, die Schwefelsäure eine Molkonzentration von 4 mol/l aufweist;
oder, wenn das Salz der Verbindung dargestellt durch Formel (II) das Sulfat dargestellt durch Formel (II-3) ist und das Kristallsalz des Sulfats dargestellt durch Formel (II-3) die Kristallform C des Sulfats ist, die Schwefelsäure und die Verbindung dargestellt durch Formel (II) ein Molverhältnis von 1,03:1 aufweisen;
oder, wenn das Salz der Verbindung dargestellt durch Formel (II) das Sulfat dargestellt durch Formel (II-3) ist und das Kristallsalz des Sulfats dargestellt durch Formel (II-3) die Kristallform C des Sulfats ist, die Verbindung dargestellt durch Formel (II) und das Ethylacetat ein Massen-Volumen-Verhältnis von 30 mg/ml aufweisen;
oder, wenn das Salz der Verbindung dargestellt durch Formel (II) das Sulfat dargestellt durch Formel (II-3) ist und das Kristallsalz des Sulfats dargestellt durch Formel (II-3) die Kristallform C des Sulfats ist, die Schwefelsäure eine Molkonzentration von 4 mol/l aufweist;
oder, wenn das Salz der Verbindung dargestellt durch Formel (II) das Sulfat dargestellt durch Formel (II-3) ist und das Sulfat dargestellt durch Formel (11-3) die Kristallform D des Sulfats ist, die Schwefelsäure und die Verbindung dargestellt durch Formel (II) ein Molverhältnis von 1,03:1 aufweisen;
oder, wenn das Salz der Verbindung dargestellt durch Formel (II) das Sulfat dargestellt durch Formel (II-3) ist und das Sulfat dargestellt durch Formel (II-3) die Kristallform D des Sulfats ist, die Verbindung dargestellt durch Formel (II) und das Ethanol ein Massen-Volumen-Verhältnis von 30 mg/ml aufweisen;
oder, wenn das Salz der Verbindung dargestellt durch Formel (II) das Sulfat dargestellt durch Formel (II-3) ist und das Sulfat dargestellt durch Formel (II-3) die Kristallform D des Sulfats ist, die Schwefelsäure eine Molkonzentration von 4 mol/l aufweist;
oder, wenn das Hydrat des Salzes der Verbindung dargestellt durch Formel (II) das Hydrat des Sulfats dargestellt durch Formel (II-3) ist und das Hydrat des Sulfats dargestellt durch Formel (II-3) die Kristallform B des Hydrats des Sulfats ist, die Schwefelsäure und die Verbindung dargestellt durch Formel (II) ein Molverhältnis von 1,03:1 aufweisen;
oder, wenn das Hydrat des Salzes der Verbindung dargestellt durch Formel (II) das Hydrat des Sulfats dargestellt durch Formel (II-3) ist und das Hydrat des Sulfats dargestellt durch Formel (II-3) die Kristallform B des Hydrats des Sulfats ist, die Verbindung dargestellt durch Formel (II) und das Aceton ein Massen-Volumen-Verhältnis von 30 mg/ml aufweisen;
oder, wenn das Hydrat des Salzes der Verbindung dargestellt durch Formel (II) das Hydrat des Sulfats dargestellt durch Formel (II-3) ist und das Hydrat des Sulfats dargestellt durch Formel (II-3) die Kristallform B des Hydrats des Sulfats ist, die Schwefelsäure eine Molkonzentration von 4 mol/l aufweist.

9. Kristallform, Salz, Kristallform des Salzes, Hydrat des Salzes oder Kristallform des Hydrats des Salzes der Verbindung dargestellt durch Formel (II) nach einem der Ansprüche 1-5 zur Verwendung bei der Behandlung von rheumatoider Arthritis.

## Revendications

1. Forme cristalline, sel, forme cristalline du sel, hydrate du sel, ou forme cristalline de l'hydrate du sel d'un composé représenté par la formule (II), 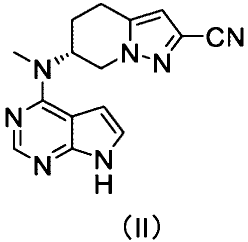 et un acide dans le sel est choisi parmi l'acide maléique, l'acide chlorhydrique et l'acide sulfurique.

2. Forme cristalline, sel, forme cristalline du sel, hydrate du sel ou forme cristalline de l'hydrate du sel du composé représenté par la formule (II) selon la revendication 1, ledit acide dans le sel et ledit composé représenté par la formule (II) présentant un rapport molaire de (0,25-1,5): 1.

3. Forme cristalline, sel, forme cristalline du sel, hydrate du sel ou forme cristalline de l'hydrate du sel du composé représenté par la formule (II) selon la revendication 2, ledit sel du composé représenté par la formule (II) est choisi parmi l'un quelconque des composés suivants :
un sulfate représenté par la formule (II-3) 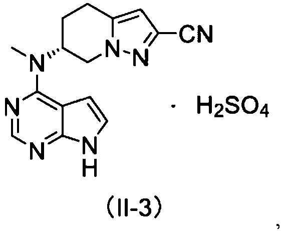
un chlorhydrate représenté par la formule (II-2) 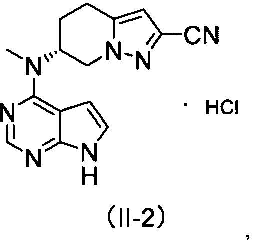
et un maléate représenté par la formule (II-1) 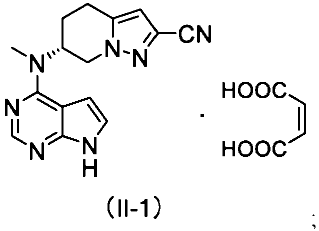 ou,
l'hydrate du sel du composé représenté par la formule (II) est choisi parmi un hydrate d'un sulfate représenté par la formule (II-3)

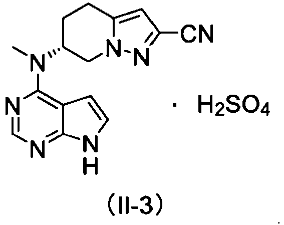

4. Forme cristalline, sel, forme cristalline du sel, hydrate du sel, ou forme cristalline de l'hydrate du sel du composé représenté par la formule (II) selon la revendication 3,
ladite forme cristalline du composé représenté par la formule (II) étant une forme cristalline A, qui présente un diagramme de diffraction des rayons X sur poudre exprimé par des angles 2θ présentant des pics de diffraction à 12,69°, 13,84°, 15,37°, 15,90°, 16,62°, 19,07°, 27,66° et 25,62° ;
ou, ladite forme cristalline du composé représenté par la formule (II) étant une forme cristalline B, qui présente un diagramme de diffraction des rayons X sur poudre exprimé par des angles 2θ présentant des pics de diffraction à 12,40°, 13,31°, 15,75°, 22,16°, 23,72°, 25,49°, 26,12° et 26,87° ;
ou, ledit maléate représenté par la formule (II-1) étant une forme cristalline A du maléate, qui présente un diagramme de diffraction des rayons X sur poudre exprimé par des angles 2θ présentant des pics de diffraction à 12,24°, 13,14°, 13,73°, 14,56°, 15,52 °, 17,54°, 19,54°, 23,19°, 26,55° et 26,91° ;
ou, ledit chlorhydrate représenté par la formule (II-2) étant une forme cristalline A du chlorhydrate, qui présente un diagramme de diffraction des rayons X sur poudre exprimé par des angles 2θ présentant des pics de diffraction à 7,20°, 7,85°, 8,63°, 10,82°, 21,25°, 21,78°, 24,12°, 25,56°, 26,11° et 27,03° ;
ou, ledit chlorhydrate représenté par la formule (II-2) étant une forme cristalline B du chlorhydrate, qui présente un diagramme de diffraction des rayons X sur poudre exprimé par des angles 2θ présentant des pics de diffraction à 5,95°, 11,92°, 12,53°, 13,14°, 20,94°, 24,96°, 25,67°, 30,09° et 31,69° ;
ou, ledit chlorhydrate représenté par la formule (II-2) étant une forme cristalline C du chlorhydrate, qui présente un diagramme de diffraction des rayons X sur poudre exprimé par des angles 20 présentant des pics de diffraction à 5,89°, 11,77°, 13,21°, 13,52°, 15,75°, 23,51°, 25,51°, 24,65°, 26,31° et 27,15° ;
ou, ledit chlorhydrate représenté par la formule (II-2) étant une forme cristalline D du chlorhydrate, qui présente un diagramme de diffraction des rayons X sur poudre exprimé par des angles 20 présentant des pics de diffraction à 3,08°, 6,07°, 9,05°, 12,06°, 12,73°, 13,23°, 13,78°, 15,08°, 21,28°, 24,94°, 26,06° et 31,72° ;
ou, ledit sulfate représenté par la formule (II-3) étant une forme cristalline A du sulfate, qui présente un diagramme de diffraction des rayons X sur poudre exprimé par des angles 20 présentant des pics de diffraction à 4,16°, 4,46°, 7,56°, 8,02°, 12,65°, 13,36°, 15,75°, 17,91°, 20,43°, 24,54°, 24,94°, 25,90° et 26,99° ;
ou, ledit sulfate représenté par la formule (II-3) étant une forme cristalline C du sulfate, qui présente un diagramme de diffraction des rayons X sur poudre exprimé par des angles 20 présentant des pics de diffraction à 6,09°, 12,17°, 13,25°, 15,60°, 16,09°, 17,45°, 18,66°, 22,61°, 23,62°, 24,44°, 25,04°, 25,89°, 26,30° et 26,62° ;
ou, ledit sulfate représenté par la formule (II-3) étant une forme cristalline D du sulfate, qui présente un diagramme de diffraction des rayons X sur poudre exprimé par des angles 20 présentant des pics de diffraction à 4,61°, 6,11°, 8,00°, 9,10°, 10,91°, 13,73°, 18,86°, 23,27°, 25,28° et 25,97° ;
ou, ledit hydrate du sulfate représenté par la formule (II-3) étant une forme cristalline B de l'hydrate du sulfate, qui présente un diagramme de diffraction des rayons X sur poudre exprimé par des angles 2θ présentant des pics de diffraction à 5,18°, 12,03°, 12,92°, 15,54°, 16,09°, 17,74°, 19,06°, 20,71°, 23,99°, 24,82° et 25,79°.

5. Forme cristalline, sel, forme cristalline du sel, hydrate du sel, ou forme cristalline de l'hydrate du sel du composé représenté par la formule (II) selon la revendication 4, où lorsque la forme cristalline du composé représenté par la formule (II) est la forme cristalline A, dans le diagramme de diffraction des rayons X sur poudre de la forme cristalline A exprimé par des angles 2θ, des valeurs 2θ sont indiquées dans le tableau suivant :
| Angle 2θ (°) | Intensité relative (%) |
|---|---|
| 6,33 | 9,91 |
| 12,69 | 97,83 |
| 13,84 | 69,04 |
| 14,91 | 27,95 |
| 15,37 | 76,70 |
| 15,90 | 45,28 |
| 16,62 | 66,33 |
| 19,07 | 40,00 |
| 22,15 | 17,81 |
| 24,96 | 24,95 |
| 25,62 | 100,00 |
| 26,95 | 30,65 |
| 27,66 | 29,86 |
| 30,19 | 5,78 |
| 37,92 | 3,13 |
| 38,88 | 7,58 |
;
ou, la forme cristalline A du composé représenté par la formule (II) présente une courbe de calorimétrie à balayage différentiel (DSC) présentant un pic d'absorption avec une température de pic de 300,4 °C ; ou, la forme cristalline A du composé représenté par la formule (II) présente une courbe d'analyse thermogravimétrique avec une perte de poids de 2,1 % dans une plage de température de 25,1 °C à 250 °C ;
ou, lorsque la forme cristalline du composé représenté par la formule (II) est la forme cristalline B, dans le diagramme de diffraction des rayons X sur poudre de la forme cristalline B exprimé par des angles 2θ, des valeurs 2θ sont indiquées dans le tableau suivant :
| Angle 2θ (°) | Intensité relative (%) |
|---|---|
| 12,40 | 43,82 |
| 13,31 | 79,40 |
| 15,75 | 76,57 |
| 19,12 | 15,04 |
| Angle 2θ (°) | Intensité relative (%) |
|---|---|
| 22,16 | 100,00 |
| 23,72 | 62,88 |
| 25,49 | 20,80 |
| 26,12 | 42,21 |
| 26,87 | 26,28 |
| 33,42 | 2,44 |
;
ou, lorsque le maléate représenté par la formule (II-1) est la forme cristalline A du maléate, dans le diagramme de diffraction des rayons X sur poudre de la forme cristalline A du maléate exprimé par des angles 2θ, des valeurs 2θ sont indiquées dans le tableau suivant :
| Angle 2θ (°) | Intensité relative (%) |
|---|---|
| 4,40 | 14,26 |
| 8,76 | 9,45 |
| 12,24 | 21,69 |
| 13,14 | 24,97 |
| 13,73 | 100,00 |
| 14,56 | 19,33 |
| 15,52 | 16,05 |
| 16,42 | 2,53 |
| 17,54 | 35,08 |
| 18,89 | 2,59 |
| 19,54 | 32,55 |
| 20,37 | 11,04 |
| 23,19 | 18,59 |
| 23,70 | 5,19 |
| 24,59 | 5,61 |
| Angle 2θ (°) | Intensité relative (%) |
|---|---|
| 25,52 | 7,75 |
| 26,55 | 15,18 |
| 26,91 | 15,77 |
| 27,11 | 9,87 |
| 27,98 | 3,54 |
| 29,30 | 6,68 |
| 30,01 | 3,87 |
| 36,24 | 1,53 |
| 37,44 | 2,47 |
;
ou, la forme cristalline A du maléate représenté par la formule (II-1) présente une courbe de calorimétrie à balayage différentiel présentant un pic d'absorption avec une température de pic de 173,3 °C ; ou, la forme cristalline A du maléate représenté par la formule (II-1) présente une courbe d'analyse thermogravimétrique avec une perte de poids de 1,86 % dans une plage de température de 29,1 °C à 150 °C ;
ou, lorsque le chlorhydrate représenté par la formule (II-2) est la forme cristalline A du chlorhydrate, dans le diagramme de diffraction des rayons X sur poudre de la forme cristalline A du chlorhydrate représenté par la formule (II-2) exprimé par des angles 2θ, des valeurs 2θ sont indiquées dans le tableau suivant :
| Angle 2θ (°) | Intensité relative (%) |
|---|---|
| 6,17 | 8,01 |
| 7,20 | 38,14 |
| 7,85 | 100,00 |
| 8,63 | 30,15 |
| 10,82 | 51,15 |
| 12,55 | 6.78 |
| 13,75 | 6,92 |
| 15,95 | 9,57 |
| Angle 2θ (°) | Intensité relative (%) |
|---|---|
| 19,08 | 5,50 |
| 20,39 | 7,33 |
| 21,25 | 35,03 |
| 21,78 | 33,53 |
| 24,12 | 26,63 |
| 25,56 | 38,38 |
| 26,11 | 37,55 |
| 27,03 | 28,20 |
| 31,72 | 11,22 |
;
ou, la forme cristalline A du chlorhydrate représenté par la formule (II-2) présente une courbe de calorimétrie à balayage différentiel présentant trois pics d'absorption avec des températures de pic de 78,1 °C, 92,2 °C et 274 °C, respectivement ; ou, la forme cristalline A du chlorhydrate représenté par la formule (II-2) présente une courbe d'analyse thermogravimétrique avec une perte de poids de 6,71 % dans une plage de température de 27,2 °C à 100 °C ;
ou, lorsque le chlorhydrate représenté par la formule (II-2) est la forme cristalline B du chlorhydrate, dans le diagramme de diffraction des rayons X sur poudre de la forme cristalline B du chlorhydrate exprimé par des angles 2θ, des valeurs 2θ sont indiquées dans le tableau suivant :
| Angle 2θ (°) | Intensité relative (%) |
|---|---|
| 5,95 | 100,00 |
| 11,05 | 13,61 |
| 11,92 | 61,44 |
| 12,53 | 31,64 |
| 13,14 | 48,62 |
| 16,67 | 8,26 |
| 19,31 | 6,81 |
| 20,94 | 90,42 |
| Angle 2θ (°) | Intensité relative (%) |
|---|---|
| 22,04 | 5,05 |
| 23,19 | 13,88 |
| 24,96 | 43,42 |
| 25,67 | 49,06 |
| 30,09 | 18,58 |
| 31,69 | 36,23 |
| 39,46 | 14,32 |
;
ou, la forme cristalline B du chlorhydrate représenté par la formule (II-2) présente une courbe de calorimétrie à balayage différentiel présentant un pic d'absorption avec une température de pic de 274,7 °C ; ou, la forme cristalline B du chlorhydrate représenté par la formule (II-2) présente une courbe d'analyse thermogravimétrique avec une perte de poids de 2,34 % dans une plage de température de 31,4 °C à 100 °C ;
ou, lorsque le chlorhydrate représenté par la formule (II-2) est la forme cristalline C du chlorhydrate, dans le diagramme de diffraction des rayons X sur poudre de la forme cristalline C du chlorhydrate représenté par la formule (II-2) exprimé par des angles 2θ, des valeurs 2θ sont indiquées dans le tableau suivant :
| Angle 2θ (°) | Intensité relative (%) |
|---|---|
| 5,89 | 60,87 |
| 11,77 | 100,00 |
| 12,58 | 22,24 |
| 13,21 | 71,07 |
| 15,75 | 59,51 |
| 17,71 | 13,70 |
| 18,41 | 17,93 |
| 23,51 | 90,26 |
| 24,65 | 18,91 |
| 25,51 | 25,11 |
| Angle 2θ (°) | Intensité relative (%) |
|---|---|
| 26,31 | 55,95 |
| 27,15 | 43,81 |
;
ou, la forme cristalline C du chlorhydrate représenté par la formule (II-2) présente une courbe de calorimétrie à balayage différentiel présentant un pic d'absorption avec une température de pic de 275,1 °C ; ou, la forme cristalline C du chlorhydrate représenté par la formule (II-2) présente une courbe d'analyse thermogravimétrique avec une perte de poids de 1,32 % dans une plage de température de 33,7 °C à 100 °C ;
ou, lorsque le chlorhydrate représenté par la formule (II-2) est la forme cristalline D du chlorhydrate, dans le diagramme de diffraction des rayons X sur poudre de la forme cristalline D du chlorhydrate exprimé par des angles 2θ, des valeurs 2θ sont indiquées dans le tableau suivant :
| Angle 2θ (°) | Intensité relative (%) |
|---|---|
| 3,08 | 79,15 |
| 6,07 | 100,00 |
| 9,05 | 30,52 |
| 12,06 | 29,69 |
| 12,73 | 34,25 |
| 13,23 | 25,66 |
| 13,78 | 25,88 |
| 14,53 | 17,45 |
| 15,08 | 26,72 |
| | |
|---|---|
| 16,32 | 14,81 |
| 17,45 | 11,57 |
| 18,20 | 10,95 |
| 19,31 | 6,12 |
| 21,28 | 79,37 |
| 22,02 | 15,50 |
| Angle 2θ (°) | Intensité relative (%) |
|---|---|
| 23,36 | 10,90 |
| 24,94 | 28,49 |
| 26,06 | 36,10 |
| 29,23 | 5,77 |
| 30,51 | 11,55 |
| 31,72 | 40,73 |
| 36,83 | 5,80 |
;
ou, la forme cristalline D du chlorhydrate représenté par la formule (II-2) présente une courbe de calorimétrie à balayage différentiel présentant deux pics d'absorption avec des températures de pic de 273,7 °C et 279,3 °C, respectivement ; ou, la forme cristalline D du chlorhydrate représenté par la formule (II-2) présente une courbe d'analyse thermogravimétrique avec une perte de poids de 2,92 % dans une plage de température de 28,3 °C à 100 °C ;
ou, lorsque le sulfate représenté par la formule (II-3) est la forme cristalline A du sulfate, dans le diagramme de diffraction des rayons X sur poudre de la forme cristalline A du sulfate exprimé par des angles 2θ, des valeurs 2θ sont indiquées dans le tableau suivant :
| Angle 2θ (°) | Intensité relative (%) |
|---|---|
| 4,16 | 81,60 |
| 4,46 | 71,99 |
| 7,56 | 100,00 |
| 8,02 | 63,19 |
| 9,67 | 3,76 |
| 12,65 | 30,66 |
| 13,36 | 77,29 |
| 14,36 | 14,12 |
| 15,75 | 40,01 |
| 17,91 | 38,83 |
| Angle 2θ (°) | Intensité relative (%) |
|---|---|
| 19,02 | 21,35 |
| 20,43 | 34,62 |
| 22,34 | 11,00 |
| 24,54 | 61,38 |
| 24,94 | 65,92 |
| 25,90 | 49,58 |
| 26,99 | 43,14 |
| 27,87 | 11,82 |
;
ou, la forme cristalline A du sulfate représenté par la formule (II-3) présente une courbe de calorimétrie à balayage différentiel présentant deux pics d'absorption avec des températures de pic de 85,1 °C et 126,7 °C, respectivement ; ou, la forme cristalline A du sulfate représenté par la formule (II-3) présente une courbe d'analyse thermogravimétrique avec une perte de poids de 8,14 % dans une plage de température de 28,4 °C à 150 °C ;
ou, lorsque le sel cristallin du sulfate représenté par la formule (II-3) est la forme cristalline C du sulfate, dans le diagramme de diffraction des rayons X sur poudre de la forme cristalline C du sulfate exprimé par des angles 2θ, des valeurs 2θ sont indiquées dans le tableau suivant :
| Angle 2θ (°) | Intensité relative (%) |
|---|---|
| 6,09 | 99,99 |
| 9,87 | 8,42 |
| 10,84 | 8,31 |
| 12,17 | 91,39 |
| 13,25 | 28,45 |
| 15,60 | 28,16 |
| 16,09 | 43,58 |
| 17,45 | 31,44 |
| 18,66 | 100,00 |
| Angle 2θ (°) | Intensité relative (%) |
|---|---|
| 19,80 | 25,64 |
| 20,88 | 22,81 |
| 22,61 | 32,22 |
| 23,62 | 35,33 |
| 24,44 | 30,65 |
| 25,04 | 31,87 |
| 25,89 | 63,39 |
| 26,30 | 43,49 |
| 26,62 | 62,05 |
| 27,43 | 21,62 |
| 27,76 | 15,17 |
;
ou, la forme cristalline C du sulfate représenté par la formule (II-3) présente une courbe de calorimétrie à balayage différentiel présentant deux pics d'absorption avec des températures de pic de 69,3 °C et 118,1 °C, respectivement ; ou, la forme cristalline C du sulfate représenté par la formule (II-3) présente une courbe d'analyse thermogravimétrique avec une perte de poids de 6,82 % dans une plage de température de 28,2 °C à 150 °C ;
ou, lorsque le sulfate représenté par la formule (II-3) est la forme cristalline D du sulfate, dans le diagramme de diffraction des rayons X sur poudre de la forme cristalline D du sulfate exprimé par des angles 2θ, des valeurs 2θ sont indiquées dans le tableau suivant :
| Angle 2θ (°) | Intensité relative (%) |
|---|---|
| 4,61 | 68,84 |
| 6,11 | 29,48 |
| 8,00 | 45,48 |
| 9,10 | 64,58 |
| 10,05 | 18,88 |
| 10,91 | 46,90 |
| 12,72 | 24,73 |
| Angle 2θ (°) | Intensité relative (%) |
|---|---|
| 13,73 | 54,06 |
| 16,18 | 23,96 |
| 18,86 | 100,00 |
| 20,53 | 14,88 |
| 23,27 | 39,33 |
| 25,28 | 30,71 |
| 25,97 | 55,48 |
| 31,91 | 13,58 |
| 34,12 | 5,79 |
| 38,61 | 4,36 |
;
ou, la forme cristalline D du sulfate représenté par la formule (II-3) présente une courbe de calorimétrie à balayage différentiel présentant deux pics d'absorption avec des températures de pic de 78,5 °C et 144,3 °C, respectivement ; ou, la forme cristalline D du sulfate représenté par la formule (II-3) présente une courbe d'analyse thermogravimétrique avec une perte de poids de 8,13 % dans une plage de température de 23,3 °C à 150 °C ;
ou, lorsque l'hydrate du sel du composé représenté par la formule (II) est l'hydrate du sulfate représenté par la formule (II-3), l'eau sous forme cristalline B de l'hydrate du sulfate représenté par la formule (II-3) et le sulfate représenté par la formule (II-3) peuvent avoir un rapport numérique de 1,5 ; ou, dans le diagramme de diffraction des rayons X sur poudre de la forme cristalline B de l'hydrate du sulfate représenté par la formule (II-3) exprimé par des angles 2θ, des valeurs 2θ sont indiquées dans le tableau suivant :
| Angle 2θ | Intensité relative |
|---|---|
| 5,18 | 45,44 |
| 7,95 | 11,00 |
| 8,82 | 19,56 |
| 10,23 | 16,98 |
| 12,03 | 24,82 |
| 12,92 | 73,75 |
| 15,54 | 47,91 |
| 16,09 | 90,49 |
| 17,74 | 41,11 |
| 19,06 | 38,98 |
| 20,71 | 33,34 |
| 23,99 | 100,00 |
| 24,82 | 22,22 |
| 25,79 | 56,92 |
;
ou, la forme cristalline B de l'hydrate du sulfate représenté par la formule (II-3) présente une courbe de calorimétrie à balayage différentiel présentant deux pics d'absorption avec des températures de pic de 98 °C et 140,2 °C, respectivement ; ou, la forme cristalline B de l'hydrate du sulfate représenté par la formule (II-3) présente une courbe d'analyse thermogravimétrique avec une perte de poids de 6,67 % dans une plage de température de 28,6 °C à 150 °C.

6. Procédé permettant la préparation de la forme cristalline, d'un sel, d'une forme cristalline du sel, d'un hydrate du sel ou d'une forme cristalline de l'hydrate du sel du composé représenté par la formule (II) selon l'une quelconque des revendications 1 à 5, ledit procédé permettant la préparation du sel du composé représenté par la formule (II) comprenant les étapes suivantes : la réalisation d'une réaction de formation de sel du composé représenté par la formule (II) selon l'une quelconque des revendications 1 à 3 et de l'acide selon la revendication 1 dans un solvant ; de préférence, le solvant est choisi parmi un ou plusieurs parmi le tétrahydrofurane, le 2-méthylfurane, l'acétate d'éthyle, le cyclohexane, le diméthylsulfoxyde, l'eau, le *n*-butanol, l'isopropanol, l'éthanol, la *N*-méthylpyrrolidone, l'acétonitrile, l'acétone, la butanone, la méthylisobutylcétone, le toluène, le dichlorométhane, le 1,4-dioxane et l'anisole ; de préférence, le solvant est choisi parmi un ou plusieurs parmi le tétrahydrofurane, l'acétate d'éthyle, le cyclohexane, le diméthylsulfoxyde, l'éthanol, l'eau, le *n*-butanol, la *N*-méthylpyrrolidone, l'acétonitrile et la butanone ; idéalement, le solvant est choisi parmi le tétrahydrofurane, l'acétone, l'éthanol, l'acétate d'éthyle et une solution aqueuse d'acétonitrile.

7. Procédé permettant la préparation de la forme cristalline, d'un sel, d'une forme cristalline du sel, d'un hydrate du sel ou d'une forme cristalline de l'hydrate du sel du composé représenté par la formule (II) selon la revendication 6, dans lequel,
lorsque le composé représenté par la formule (II) est la forme cristalline A, le procédé permettant la préparation de la forme cristalline A du composé représenté par la formule (II) comprend les étapes suivantes : l'ajout d'un anti-solvant dans une solution du composé représenté par la formule (II) dans du tétrahydrofurane à température ambiante, et l'obtention de la forme cristalline A après cristallisation ; l'anti-solvant est un solvant ester ou un solvant alcane ;
ou, lorsque le composé représenté par la formule (II) est la forme cristalline B, le procédé permettant la préparation de la forme cristalline B du composé représenté par la formule (II) comprend les étapes suivantes : la dissolution de la forme cristalline A du composé représenté par la formule (II-1) dans la NMP à température ambiante, l'ajout d'eau et l'obtention de la forme cristalline B après cristallisation ;
ou, lorsque le sel du composé représenté par la formule (II) est le maléate représenté par la formule (II-1) et que le maléate représenté par la formule (II-1) est la forme cristalline A du maléate, le procédé permettant la préparation de la forme cristalline A du maléate comprend les étapes suivantes : la dispersion du composé représenté par la formule (II) et de l'acide maléique dans un solvant ester pour effectuer une réaction représentée par la formule suivante, et la collecte d'un solide pour obtenir la forme cristalline A ; 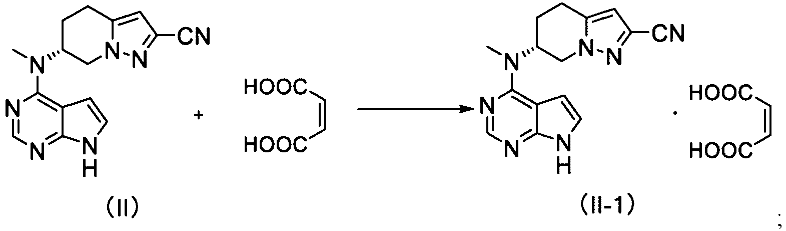
ou, lorsque le sel du composé représenté par la formule (II) est le chlorhydrate représenté par la formule (II-2) et que le chlorhydrate représenté par la formule (II-2) est la forme cristalline A du chlorhydrate, le procédé permettant la préparation de la forme cristalline A du chlorhydrate comprend les étapes suivantes : l'ajout d'acide chlorhydrique à une solution du composé représenté par la formule (II) pour effectuer une réaction représentée par la formule suivante, et la collecte d'un solide pour obtenir la forme cristalline A ; un solvant de la solution est une solution aqueuse d'acétonitrile ; 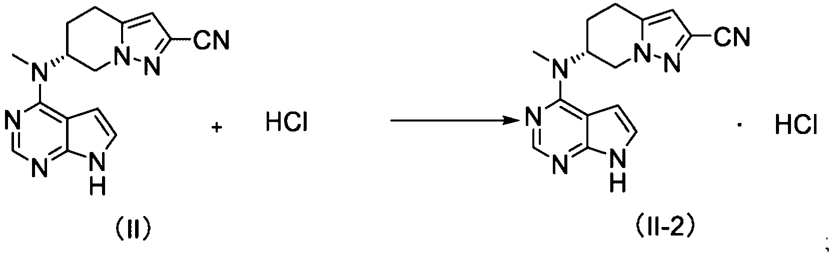
ou, lorsque le sel du composé représenté par la formule (II) est le chlorhydrate représenté par la formule (II-2) et que le chlorhydrate représenté par la formule (II-2) est la forme cristalline B du chlorhydrate, le procédé permettant la préparation de la forme cristalline B du chlorhydrate comprend les étapes suivantes : l'ajout de l'acide chlorhydrique à une solution du composé représenté par la formule (II) dans de l'acétate d'éthyle pour effectuer une réaction représentée par la formule suivante, et la collecte d'un solide pour obtenir la forme cristalline B ; 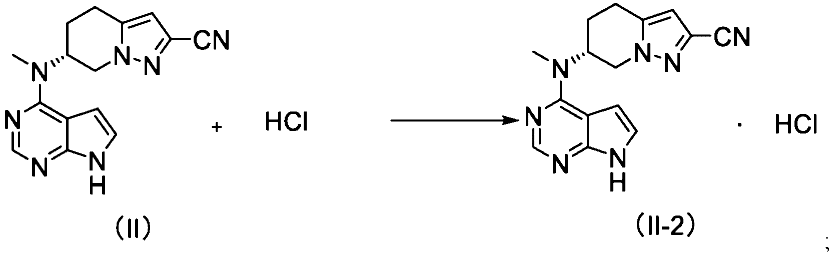
ou, lorsque le sel du composé représenté par la formule (II) est le chlorhydrate représenté par la formule (II-2) et que le chlorhydrate représenté par la formule (II-2) est la forme cristalline C du chlorhydrate, le procédé permettant la préparation de la forme cristalline C du chlorhydrate comprend les étapes suivantes : l'ajout d'acide chlorhydrique à une solution du composé représenté par la formule (II) dans de l'éthanol pour effectuer une réaction représentée par la formule suivante, et la collecte d'un solide pour obtenir la forme cristalline C ; 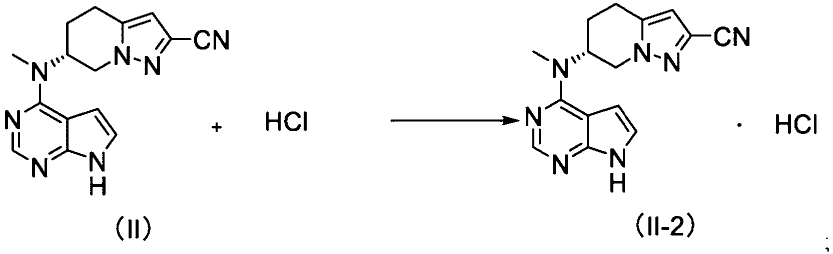
ou, lorsque le sel du composé représenté par la formule (II) est le chlorhydrate représenté par la formule (II-2) et que le chlorhydrate représenté par la formule (II-2) est la forme cristalline D du chlorhydrate, le procédé permettant la préparation de la forme cristalline D du chlorhydrate comprend les étapes suivantes : l'ajout d'acide chlorhydrique à une solution du composé représenté par la formule (II) dans du tétrahydrofurane pour effectuer une réaction représentée par la formule suivante, et la collecte d'un solide pour obtenir la forme cristalline D ; 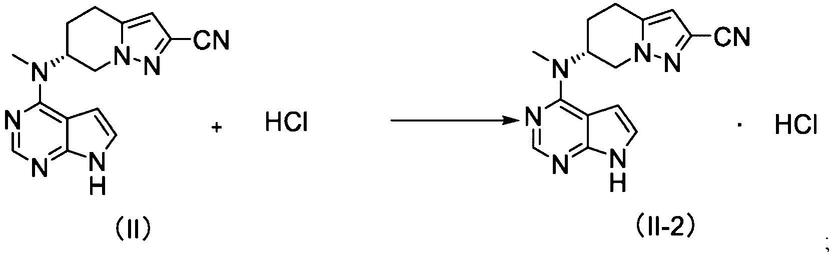
ou, lorsque le sel du composé représenté par la formule (II) est le sulfate représenté par la formule (II-3) et que le sulfate représenté par la formule (II-3) est la forme cristalline A du sulfate, le procédé permettant la préparation de la forme cristalline A du sulfate comprend les étapes suivantes : l'ajout d'acide sulfurique à une solution du composé représenté par la formule (II) pour effectuer une réaction représentée par la formule suivante, et la collecte d'un solide pour obtenir la forme cristalline A ; un solvant de la solution est une solution aqueuse d'acétonitrile ; 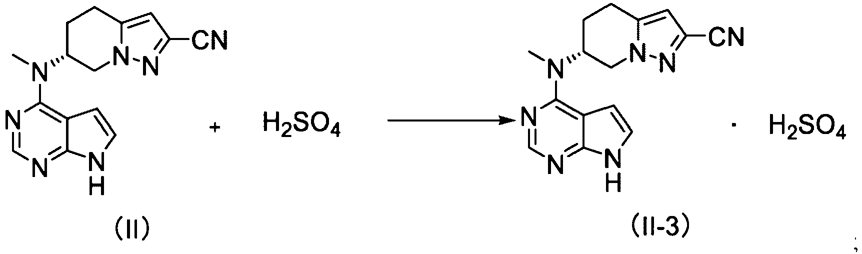
ou, lorsque le sel du composé représenté par la formule (II) est le sulfate représenté par la formule (II-3) et que le sulfate représenté par la formule (II-3) est la forme cristalline C du sulfate, le procédé permettant la préparation de la forme cristalline C du sulfate comprend les étapes suivantes : l'ajout d'acide sulfurique à une solution du composé représenté par la formule (II) dans de l'acétate d'éthyle pour effectuer une réaction représentée par la formule suivante, et la collecte d'un solide pour obtenir la forme cristalline C ; 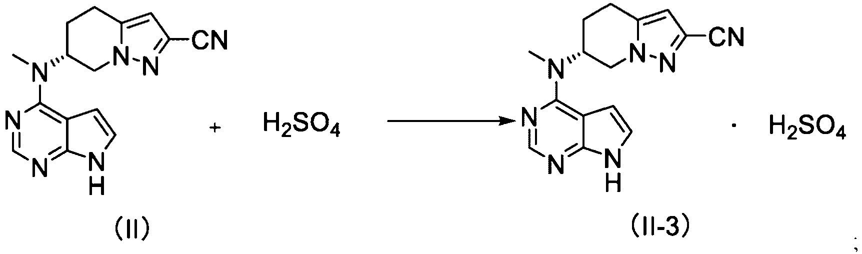
ou, lorsque le sel du composé représenté par la formule (II) est le sulfate représenté par la formule (II-3) et que le sulfate représenté par la formule (II-3) est la forme cristalline D du sulfate, le procédé permettant la préparation de la forme cristalline D du sulfate comprend les étapes suivantes : l'ajout d'acide sulfurique à une solution du composé représenté par la formule (II) dans de l'éthanol pour effectuer une réaction représentée par la formule suivante, et la collecte d'un solide pour obtenir la forme cristalline D ; 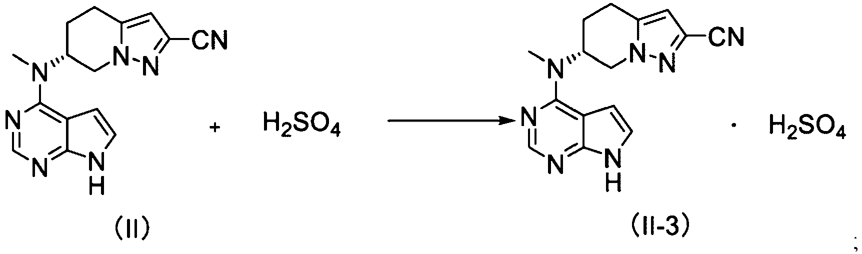
ou, lorsque l'hydrate du sel du composé représenté par la formule (II) est l'hydrate du sulfate représenté par la formule (II-3), et que l'hydrate du sulfate représenté par la formule (II-3) est la forme cristalline B de l'hydrate du sulfate, le procédé permettant la préparation de la forme cristalline B de l'hydrate du sulfate comprend les étapes suivantes : l'ajout d'acide sulfurique dans une solution du composé représenté par la formule (II) dans de l'acétone pour effectuer une réaction représentée par la formule suivante, et la collecte d'un solide pour obtenir la forme cristalline B ;

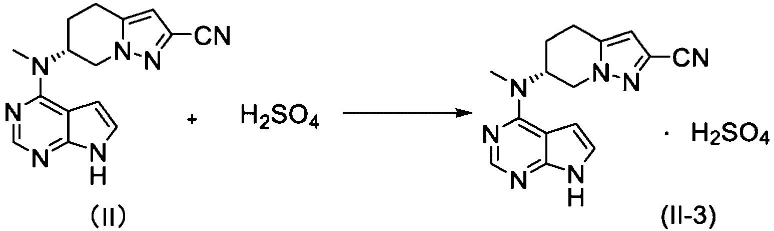

8. Procédé permettant la préparation de la forme cristalline, d'un sel, d'une forme cristalline du sel, d'un hydrate du sel ou d'une forme cristalline de l'hydrate du sel du composé représenté par la formule (II) selon la revendication 7, dans lequel,
lorsque le composé représenté par la formule (II) est la forme cristalline A, le solvant ester est l'acétate d'éthyle ou l'acétate de n-butyle, tel que l'acétate d'éthyle ; ou le solvant alcane est le n-hexane, le cyclohexane ou le n-heptane, tel que le cyclohexane ;
ou, lorsque le composé représenté par la formule (II) est la forme cristalline A, le tétrahydrofurane et l'anti-solvant présentent un rapport volumique de 1:(0,1-10) ; de préférence de 1:(0,5-3), idéalement de 1:1 ou 1,25:1 ;
ou, lorsque le composé représenté par la formule (II) est la forme cristalline A, le composé représenté par la formule (II) et le tétrahydrofurane présentent un rapport masse-volume de 2-6 mg/ml ; de préférence de 5:1 mg/ml ;
ou, lorsque le composé représenté par la formule (II) est la forme cristalline A, le composé représenté par la formule (II) et l'anti-solvant présentent un rapport masse-volume de 2-6 mg/ml ; de préférence 5:1 mg/ml ou 4:1 mg/ml ;
ou, lorsque le composé représenté par la formule (II) est la forme cristalline B, la forme cristalline A du composé représenté par la formule (II-1) et la NMP présentent un rapport masse-volume de 1-5 mg/ml ; de préférence 2:1 mg/ml ;
ou, lorsque le composé représenté par la formule (II) est la forme cristalline B, la NMP et l'eau présentent un rapport volumique de 1:(0,1-10) ; de préférence de 1:(0,2-2), idéalement de 1:0,4 ;
ou, lorsque le sel du composé représenté par la formule (II) est le maléate représenté par la formule (II-1) et que le maléate représenté par la formule (II-1) est la forme cristalline A du maléate, l'acide maléique et le composé représenté par la formule (II) présentent un rapport molaire de 1,04:1 ;
ou, lorsque le sel du composé représenté par la formule (II) est le maléate représenté par la formule (II-1) et que le maléate représenté par la formule (II-1) est la forme cristalline A du maléate, le composé représenté par la formule (II) et le solvant ester présentent un rapport masse-volume de 10 mg/ml ;
ou, lorsque le sel du composé représenté par la formule (II) est le maléate représenté par la formule (II-1) et que le maléate représenté par la formule (II-1) est la forme cristalline A du maléate, le solvant ester est l'acétate d'éthyle ;
ou, lorsque le sel du composé représenté par la formule (II) est le chlorhydrate représenté par la formule (II-2) et que le chlorhydrate représenté par la formule (II-2) est la forme cristalline A du chlorhydrate, l'acide chlorhydrique et le composé représenté par la formule (II) présentent un rapport molaire de 1,05:1 ;
ou, lorsque le sel du composé représenté par la formule (II) est le chlorhydrate représenté par la formule (II-2) et que le chlorhydrate représenté par la formule (II-2) est la forme cristalline A du chlorhydrate, le composé représenté par la formule (II) et la solution aqueuse d'acétonitrile présentent un rapport masse-volume de 40 mg/ml ;
ou, lorsque le sel du composé représenté par la formule (II) est le chlorhydrate représenté par la formule (II-2) et que le chlorhydrate représenté par la formule (II-2) est la forme cristalline A du chlorhydrate, la solution aqueuse d'acétonitrile présente un rapport volumique entre l'acétonitrile et H₂O dans la solution aqueuse d'acétonitrile présentant un rapport volumique de 19:1 ;
ou, lorsque le sel du composé représenté par la formule (II) est le chlorhydrate représenté par la formule (II-2) et que le chlorhydrate représenté par la formule (II-2) est la forme cristalline B du chlorhydrate, l'acide chlorhydrique et le composé représenté par la formule (II) présentent un rapport molaire de 1,05:1 ;
ou, lorsque le sel du composé représenté par la formule (II) est le chlorhydrate représenté par la formule (II-2) et que le chlorhydrate représenté par la formule (II-2) est la forme cristalline B du chlorhydrate, le composé représenté par la formule (II) et l'acétate d'éthyle présentent un rapport masse-volume de 40 mg/ml ;
ou, lorsque le sel du composé représenté par la formule (II) est le chlorhydrate représenté par la formule (II-2) et que le chlorhydrate représenté par la formule (II-2) est la forme cristalline C du chlorhydrate, l'acide chlorhydrique et le composé représenté par la formule (II) présentent un rapport molaire de 1,05:1 ;
ou, lorsque le sel du composé représenté par la formule (II) est le chlorhydrate représenté par la formule (II-2) et que le chlorhydrate représenté par la formule (II-2) est la forme cristalline C du chlorhydrate, le composé représenté par la formule (II) et l'éthanol présentent un rapport masse-volume de 40 mg/ml ;
ou, lorsque le sel du composé représenté par la formule (II) est le chlorhydrate représenté par la formule (II-2) et que le chlorhydrate représenté par la formule (II-2) est la forme cristalline D du chlorhydrate, l'acide chlorhydrique et le composé représenté par la formule (II) présentent un rapport molaire de 1,05:1 ;
ou, lorsque le sel du composé représenté par la formule (II) est le chlorhydrate représenté par la formule (II-2) et que le chlorhydrate représenté par la formule (II-2) est la forme cristalline D du chlorhydrate, le composé représenté par la formule (II) et le tétrahydrofurane présentent un rapport masse-volume de 40 mg/ml ;
ou, lorsque le sel du composé représenté par la formule (II) est le sulfate représenté par la formule (II-3) et que le sulfate représenté par la formule (II-3) est la forme cristalline A du sulfate, l'acide sulfurique et le composé représenté par la formule (II) présentent un rapport molaire de 1,03:1 ;
ou, lorsque le sel du composé représenté par la formule (II) est le sulfate représenté par la formule (II-3) et que le sulfate représenté par la formule (II-3) est la forme cristalline A du sulfate, le composé représenté par la formule (II) et la solution aqueuse d'acétonitrile présentent un rapport masse-volume de 30 mg/ml ;
ou, lorsque le sel du composé représenté par la formule (II) est le sulfate représenté par la formule (II-3) et que le sulfate représenté par la formule (II-3) est la forme cristalline A du sulfate, l'acétonitrile et H₂O dans la solution aqueuse d'acétonitrile présentent un rapport volumique de 19:1 ;
ou, lorsque le sel du composé représenté par la formule (II) est le sulfate représenté par la formule (II-3) et que le sulfate représenté par la formule (II-3) est la forme cristalline A du sulfate, l'acide sulfurique présente une concentration molaire de 4 mol/l ;
ou, lorsque le sel du composé représenté par la formule (II) est le sulfate représenté par la formule (II-3) et que le sel cristallin du sulfate représenté par la formule (II-3) est la forme cristalline C du sulfate, l'acide sulfurique et le composé représenté par la formule (II) présentent un rapport molaire de 1,03:1 ;
ou, lorsque le sel du composé représenté par la formule (II) est le sulfate représenté par la formule (II-3) et que le sel cristallin du sulfate représenté par la formule (II-3) est la forme cristalline C du sulfate, le composé représenté par la formule (II) et l'acétate d'éthyle présentent un rapport masse-volume de 30 mg/ml ;
ou, lorsque le sel du composé représenté par la formule (II) est le sulfate représenté par la formule (II-3) et que le sel cristallin du sulfate représenté par la formule (II-3) est la forme cristalline C du sulfate, l'acide sulfurique présente une concentration molaire de 4 mol/l ;
ou, lorsque le sel du composé représenté par la formule (II) est le sulfate représenté par la formule (II-3) et que le sulfate représenté par la formule (II-3) est la forme cristalline D du sulfate, l'acide sulfurique et le composé représenté par la formule (II) présentent un rapport molaire de 1,03:1 ;
ou, lorsque le sel du composé représenté par la formule (II) est le sulfate représenté par la formule (II-3) et que le sulfate représenté par la formule (II-3) est la forme cristalline D du sulfate, le composé représenté par la formule (II) et l'éthanol présentent un rapport masse-volume de 30 mg/ml ;
ou, lorsque le sel du composé représenté par la formule (II) est le sulfate représenté par la formule (II-3) et que le sulfate représenté par la formule (II-3) est la forme cristalline D du sulfate, l'acide sulfurique présente une concentration molaire de 4 mol/l ;
ou, lorsque l'hydrate du sel du composé représenté par la formule (II) est l'hydrate du sulfate représenté par la formule (II-3), et que l'hydrate du sulfate représenté par la formule (II-3) est la forme cristalline B de l'hydrate du sulfate, l'acide sulfurique et le composé représenté par la formule (II) présentent un rapport molaire de 1,03:1 ;
ou, lorsque l'hydrate du sel du composé représenté par la formule (II) est l'hydrate du sulfate représenté par la formule (II-3), et que l'hydrate du sulfate représenté par la formule (II-3) est la forme cristalline B de l'hydrate du sulfate, le composé représenté par la formule (II) et l'acétone présentent un rapport masse-volume de 30 mg/ml ;
ou, lorsque l'hydrate du sel du composé représenté par la formule (II) est l'hydrate du sulfate représenté par la formule (II-3), et que l'hydrate du sulfate représenté par la formule (II-3) est la forme cristalline B de l'hydrate du sulfate, l'acide sulfurique présente une concentration molaire de 4 mol/l.

9. Forme cristalline, sel, forme cristalline du sel, hydrate du sel ou forme cristalline de l'hydrate du sel du composé représenté par la formule (II) selon l'une quelconque des revendications 1 à 5, destiné(e) à être utilisé(e) dans le traitement de la polyarthrite rhumatoïde.
